# EUROPEAN PATENT APPLICATION

(11) **EP 4 368 657 A1**
(43) Date of publication of application: **15.05.2024**
(21) Application number: 22837710.7
(22) Date of filing: 06.07.2022
(51) Int. Cl.: C08G 69/42, A61K 9/127, A61K 9/16, A61K 31/7088, A61K 38/02, A61K 45/00, A61K 47/24, A61K 47/34, A61K 47/44, A61P 35/00, A61P 43/00, C07K 16/28, C12N 15/113, C12N 15/115

(54) **PH-RESPONSIVE LIPID DERIVATIVE**

(30) Priority: 07.07.2021 JP 2021113189
(71) Applicant: NOF Corporation, Shibuya-ku Tokyo 150-6019 (JP); Tokyo Institute of Technology, Tokyo 152-8550 (JP)
(72) Inventor: OGI, Koichi, Kawasaki-shi, Kanagawa 210-0821 (JP); MICHINISHI, Junya, Kawasaki-shi, Kanagawa 210-0821 (JP); OHTAKE, Tomoyuki, Kawasaki-shi, Kanagawa 210-0821 (JP); NISHIYAMA, Nobuhiro, Tokyo 152-8550 (JP); TAKEMOTO, Hiroyasu, Tokyo 152-8550 (JP); MIURA, Yutaka, Tokyo 152-8550 (JP); NOMOTO, Takahiro, Tokyo 152-8550 (JP); MATSUI, Makoto, Tokyo 152-8550 (JP); SUNG, Yi-Jung, Tokyo 152-8550 (JP); TOYODA, Masahiro, Tokyo 152-8550 (JP); GHASEMIZADEH, Aria, Tokyo 152-8550 (JP)
(74) Representative: J A Kemp LLP
(86) International application number: PCT/JP2022/026863
(87) International publication number: WO 2023/282296

(57) **Abstract**

The present invention provides a pH-responsive lipid derivative represented by a structure represented by the following formula (i): 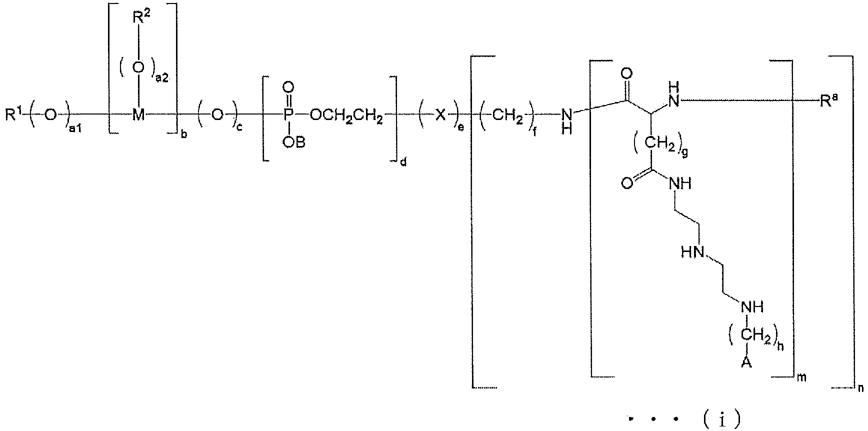 (in the above-mentioned formula, each symbol is as described in the specification), and a pH-responsive transport carrier which is useful as a transport carrier for safely and efficiently performing delivery of low-molecular-weight drugs and the like to tumor tissues.

## Description

### [Technical Field]

The present invention relates to a lipid derivative composed of a pH-responsive polymer and a lipid. The present invention also relates to a transport carrier for drug delivery that has high tumor tissue accumulation property by changing properties in response to minute pH changes around the tumor tissues.

### [Background Art]

Nucleic acid pharmaceutical products can target, with high specificity, causes of diseases that could not be targeted by conventional low molecular weight or antibody pharmaceutical products, and are expected to be used for drug discovery in many disease areas. Drug delivery system (DDS) has been studied for the purpose of more efficiently delivering nucleic acid pharmaceutical products to target cells. In addition, conventional low-molecular-weight drugs have been studied to more efficiently deliver drugs only to target cells and to reduce side effects and the like.

As such transport carriers, lipid nanoparticles (LNP) with a phospholipid or a derivative thereof, sterol, a lipid other than phospholipid, PEG lipid, or the like as a basic constituent component are generally used. In particular, PEG lipids play an important role in suppressing aggregation between carriers and improving blood retention property in the body.

This improvement in blood retention property by PEG lipids is afforded because a hydration layer of PEG covers the carrier surface and suppresses opsonization such as serum protein adsorption, which in turn avoids phagocytosis by macrophages and uptake by reticuloendothelial tissues (hereinafter referred to as stealth property). PEG lipids exhibit stealth property that suppresses random interactions with normal tissues and blood components, while also suppressing interactions with tumor tissues as well as blood components and normal tissues, and decrease accumulation in tumor tissues and uptake efficiency into cells in some cases.

The development of a transport carrier added with antibodies or antibody fragments for the general purpose of improving accumulation in tumor tissues and uptake efficiency into cells has been reported (e.g., Patent Literature 1). Such carrier is imparted with targeting properties by antibody and the like, and can be transported to specific cytoma, etc. However, since an appropriate antibody, etc. need to be selected, the transport carrier is poor in versatility for target cell type, and since the production costs for antibody, etc. are high, the cost of the transport carrier also becomes correspondingly high.

Patent Literature 2 discloses the use of a liposome for intracellular nucleic acid delivery using a temperature-responsive polymer. It has been reported that liposomes change from hydrophilic to hydrophobic due to temperature changes, thereby increasing affinity with cell membranes and improving accumulation property and uptake efficiency. However, since temperature changes are required, ancillary facility may be required in some cases, and since the temperature-responsive polymer is a vinyl polymer, decomposition in vivo cannot be expected, leaving a concern in terms of safety as a pharmaceutical product. Furthermore, in the disclosed technique, the carrier is limited to liposomes only, and the drug is also limited to nucleic acid drugs.

Non Patent Literature 1 discloses a liposome containing a phospholipid derivative in which a succinic acid skeleton that decomposes under acidic conditions is disposed between a PEG residue and a phospholipid residue. It has been reported that the liposome changes from hydrophilic to hydrophobic under acidic condition of pH 5 - 6, thereby increasing affinity with cell membranes and improving accumulation property and uptake efficiency. However, it is known that the pH around the tumor tissue is weakly acidic (about pH 6.5), and there is a possibility that the desired effect cannot be achieved around the tumor tissue. Furthermore, in the case of pH-responsive liposomes, there may be problems in terms of storage stability, such as decomposition during preparing drugs and decomposition during storage.

As described above, a technique has been desired in order to provide a transport carrier for drug delivery to tumor tissues, that changes properties by detecting minute pH changes around the tumor tissues, can encapsulate low-molecular-weight drugs and nucleic acid pharmaceutical products, and can be decomposed in vivo.

### [Citation List]

### [Patent Literature]

[PTL 1]
   WO2010/090222
[PTL 2]
   WO2016/199895

### [Non Patent Literature]

[NPL 1]
Langmuir, 2011, 27, p.10556-10561

### [Summary of Invention]

### [Technical Problem]

The present invention aims to provide a pH-responsive transport carrier which is effective as a transport carrier capable of achieving an introduction system that can safely and efficiently perform delivery of low-molecular-weight drugs and the like to tumor tissues and transfection of nucleic acid pharmaceutical products and the like to tumor cells, and further, a pH-responsive lipid derivative useful for forming such transport carriers.

### [Solution to Problem]

The present inventors have conducted intensive studies in an attempt to solve the above-mentioned problems and completed the present invention. Specifically, the present invention provides the following.

### 1. pH-responsive lipid derivative

[1] A pH-responsive lipid derivative represented by a structure represented by the following formula (i): 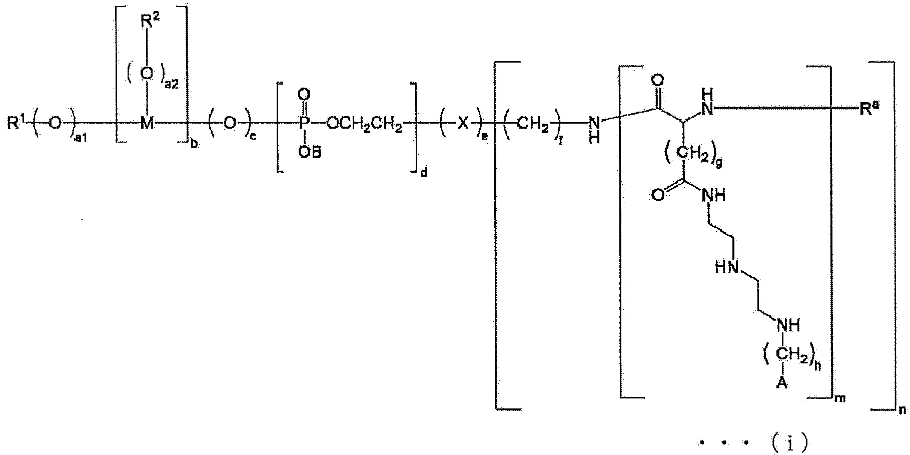 wherein
   R¹ and R² are each independently an alkyl group having 8 to 24 carbon atoms, or an acyl group having 8 to 24 carbon atoms, or a sterol residue,
   M is a trivalent group represented by>N-CH₂-CH₂- or an optionally branched hydrocarbon trivalent group having 3 to 7 carbon atoms,
   B is a hydrogen atom, an alkali metal, or an ammonium group,
   X is a linking group containing a bonding group formed by a nucleophilic substitution reaction, a Michael addition reaction, a thiol-ene reaction, or a cyclization addition reaction,
   A is a carboxy group or a sulfo group,
   a1, a2, b, c, d, and e are each independently 0 or 1,
   f is an integer of 0 to 5,
   h is an integer of 1 to 3,
   g and n are each independently 1 or 2,
   m is 5 to 300, and
   R^{a} is H, -COCH₃, or -CO-(CH₂)_{b1}-COOH (wherein b1 is 2 or 3)
   (to be also indicated as pH-responsive lipid derivative (i)).
[2] The pH-responsive lipid derivative of the above-mentioned [1], which is represented by a structure represented by the following formula (1): 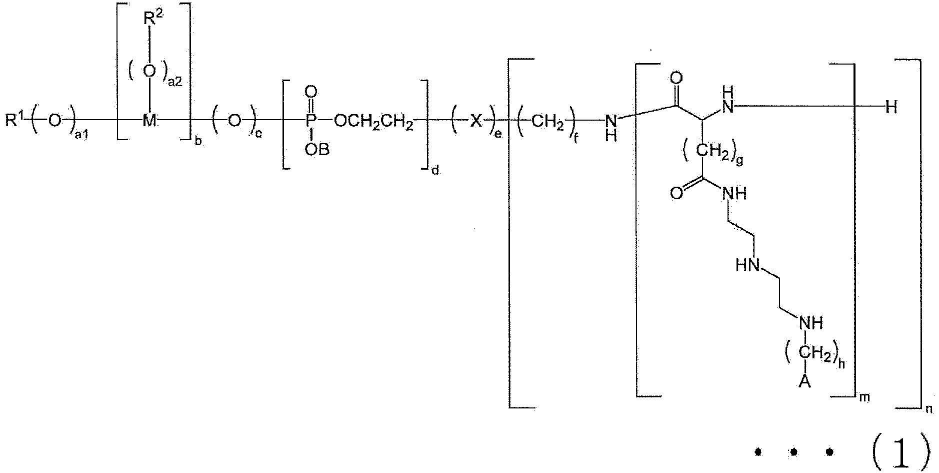 wherein
   R¹ and R² are each independently an alkyl group having 8 to 24 carbon atoms, or an acyl group having 8 to 24 carbon atoms, or a sterol residue,
   M is a trivalent group represented by>N-CH₂-CH₂- or an optionally branched hydrocarbon trivalent group having 3 to 7 carbon atoms,
   B is a hydrogen atom, an alkali metal, or an ammonium group,
   X is a linking group containing a bonding group formed by a nucleophilic substitution reaction, a Michael addition reaction, a thiol-ene reaction, or a cyclization addition reaction,
   A is a carboxy group or a sulfo group,
   a1, a2, b, c, d, and e are each independently 0 or 1,
   f is an integer of 0 to 5,
   h is an integer of 1 to 3,
   g and n are each independently 1 or 2, and
   m is 5 to 300
   (to be also indicated as pH-responsive lipid derivative (1)).
[3] The pH-responsive lipid derivative of the above-mentioned [1], which is represented by a structure represented by the following formula (1-1): 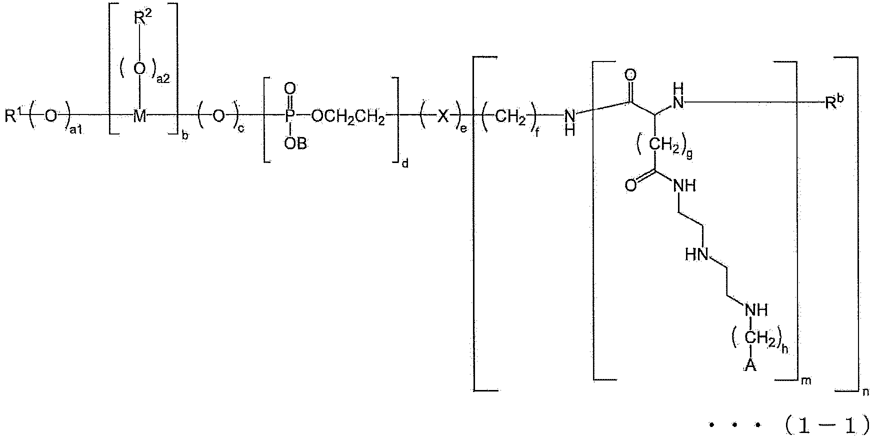 wherein
   R¹ and R² are each independently an alkyl group having 8 to 24 carbon atoms, or an acyl group having 8 to 24 carbon atoms, or a sterol residue,
   M is a trivalent group represented by>N-CH₂-CH₂- or an optionally branched hydrocarbon trivalent group having 3 to 7 carbon atoms,
   B is a hydrogen atom, an alkali metal, or an ammonium group,
   X is a linking group containing a bonding group formed by a nucleophilic substitution reaction, a Michael addition reaction, a thiol-ene reaction, or a cyclization addition reaction,
   A is a carboxy group or a sulfo group,
   a1, a2, b, c, d, and e are each independently 0 or 1,
   f is an integer of 0 to 5,
   h is an integer of 1 to 3,
   g and n are each independently 1 or 2,
   m is 5 to 300, and
   R^{b} is -COCH₃ or -CO- (CH₂)_{b1}-COOH (wherein b1 is 2 or 3),
   (to be also indicated as pH-responsive lipid derivative (1-1)).
[4] The pH-responsive lipid derivative of any of the above-mentioned [1] to [3], wherein, in the aforementioned formula (i), the aforementioned formula (1), or the aforementioned formula (1-1),
   X is a linking group represented by the following formula (2): 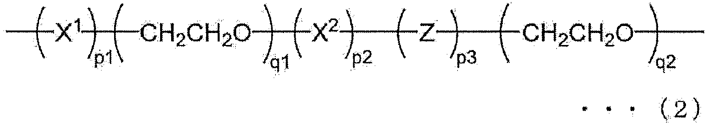 (in the formula (2), X¹ and X² are each independently a group having 1 to 5 carbon atoms and containing a bonding group resulting from a nucleophilic substitution reaction, Z is a group formed by a Michael addition reaction, a thiol-ene reaction, or a cyclization addition reaction, p1, p2, p3 are each independently 0 or 1, and q1 and q2 are each independently 0 to 50).
[5] The pH-responsive lipid derivative of any of the above-mentioned [1] to [4], wherein, in the aforementioned formula (i), the aforementioned formula (1), or the aforementioned formula (1-1),
   m is 5 to 150.
[6] The pH-responsive lipid derivative of any of the above-mentioned [1] to [5], wherein, in the aforementioned formula (i), the aforementioned formula (1), or the aforementioned formula (1-1),
   g and h are each 2, and A is a carboxy group.
[7] The pH-responsive lipid derivative of any of the above-mentioned [1] to [6], wherein, in the aforementioned formula (i), the aforementioned formula (1), or the aforementioned formula (1-1),
   R¹ and R² are each independently an alkyl group having 8 to 24 carbon atoms,
   b and n are each 1, and
   d is 0.
[8] The pH-responsive lipid derivative of any of the above-mentioned [1] to [3], [5] to [7], wherein, in the aforementioned formula (i), the aforementioned formula (1), or the aforementioned formula (1-1),
   R¹ and R² are each independently an alkyl group having 8 to 24 carbon atoms,
   M is an optionally branched hydrocarbon trivalent group having 3 to 7 carbon atoms,
   A is a carboxy group,
   a1, a2, b, and n are each 1,
   c, d, e, and f are each 0, and
   g and h are each 2.
[9] The pH-responsive lipid derivative of any of the above-mentioned [1] to [8], wherein, in the aforementioned formula (i), the aforementioned formula (1), or the aforementioned formula (1-1),
   R¹ is a sterol residue, and a1, b, c, and d are each 0.
[10] The pH-responsive lipid derivative of any of the above-mentioned [1] to [7], [9], wherein, in the aforementioned formula (i), the aforementioned formula (1), or the aforementioned formula (1-1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction, and p1, p2, and p3 are each 1.
[11] The pH-responsive lipid derivative of any of the above-mentioned [1] to [7], [9], and [10], wherein, in the aforementioned formula (i), the aforementioned formula (1), or the aforementioned formula (1-1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q2 is 0 to 5.
[12] The pH-responsive lipid derivative of any of the above-mentioned [1] to [7], [9] to [11], wherein, in the aforementioned formula (i), the aforementioned formula (1), or the aforementioned formula (1-1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 is 0 to 5.
[13] The pH-responsive lipid derivative of any of the above-mentioned [1] to [7], [9] to [12], wherein, in the aforementioned formula (i), the aforementioned formula (1), or the aforementioned formula (1-1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group,
   A is a carboxy group,
   a1, a2, b, c, d, e, and n are each 1,
   f is an integer of 2 to 5,
   g and h are each 2, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 and q2 are each independently 0 to 5.
[14] The pH-responsive lipid derivative of any of the above-mentioned [1] to [7], [9] to [13], wherein, in the aforementioned formula (i), the aforementioned formula (1), or the aforementioned formula (1-1),
   R¹ and Rare each independently an alkyl group having 8 to 24 carbon atoms or an acyl group having 8 to 24 carbon atoms,
   b, e, and n are each 1,
   d is 0,
   f is an integer of 2 to 5, and
   for X in the aforementioned formula (2),
   X¹ and X² are each a group having 1 to 5 carbon atoms and containing a bonding group resulting from a nucleophilic substitution reaction, and
   p1, p2, and p3 are each 1.
[15] The pH-responsive lipid derivative of any of the above-mentioned [1] to [7], [9] to [14], wherein, in the aforementioned formula (i), the aforementioned formula (1), or the aforementioned formula (1-1),
   R¹ and R² are each independently an alkyl group having 8 to 24 carbon atoms or an acyl group having 8 to 24 carbon atoms,
   A is a carboxy group,
   b, e, and n are each 1,
   d is 0,
   f is an integer of 2 to 5,
   g and h are each 2, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 and q2 are each independently 0 to 5.

### 2. Production method of pH-responsive lipid derivative

A method for producing a pH-responsive lipid derivative represented by a structure represented by the following formula (47-i) : 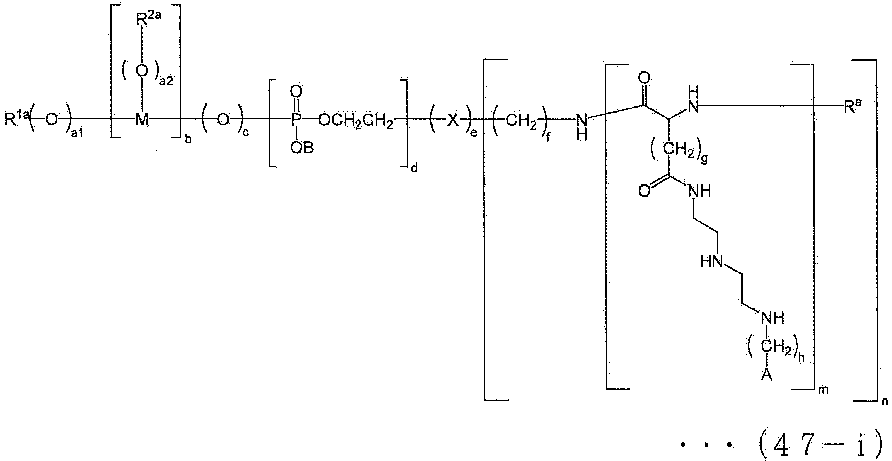 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{a} is H, -COCH₃, or -CO-(CH₂)_{b1}-COOH (wherein b1 is 2 or 3), the method comprising the following step (A), step (B), and step (C) performed in this order when R^{a} in the formula (47-i) is H, and the following step (A1), step (B), and step (C) performed in this order when R^{a} in the formula (47-i) is -COCH₃ or -CO-(CH₂)_{b1}-COOH (wherein b1 is 2 or 3):
   [step (A)]
      a step of obtaining a polymer represented by the following formula (5) by a ring opening polymerization using a polymerization initiator represented by the following formula (3) and an α-amino acid-N-carboxylic acid anhydride represented by the following formula (4) 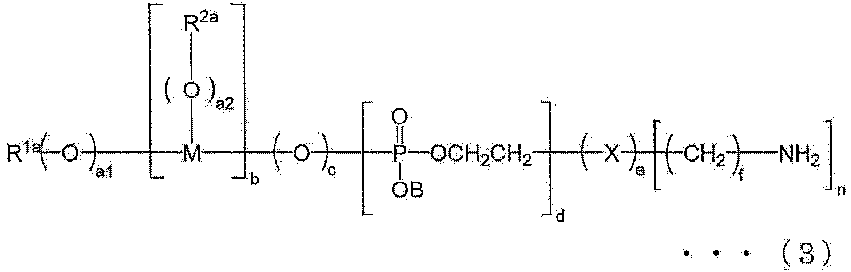 (in the formula (3), each symbol is as defined above) 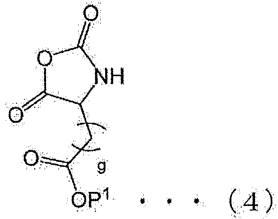 (in the formula (4), P¹ is a carboxy-protecting group, and g is 1 or 2) 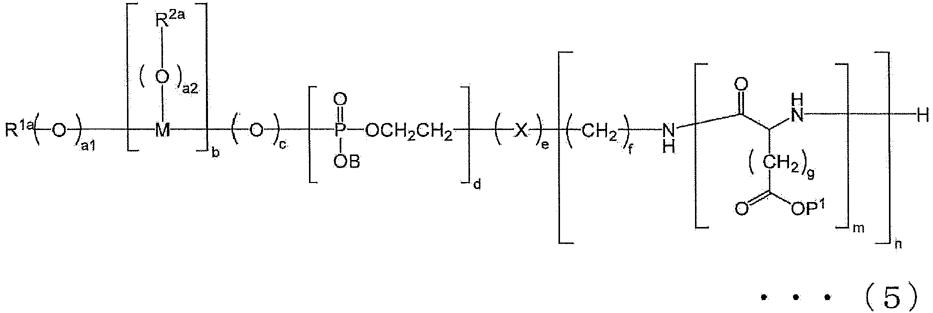 (in the formula (5), each symbol is as defined above)
[step (A1)]
   a step of obtaining a polymer represented by the following formula (5) by a ring opening polymerization using a polymerization initiator represented by the following formula (3) and an α-amino acid-N-carboxylic acid anhydride represented by the following formula (4), and further introducing -COCH₃ or -CO-(CH₂)_{b1}-COOH (wherein b1 is 2 or 3) into a terminal amino group of the obtained polymer to obtain a polymer represented by the following formula (5-1) 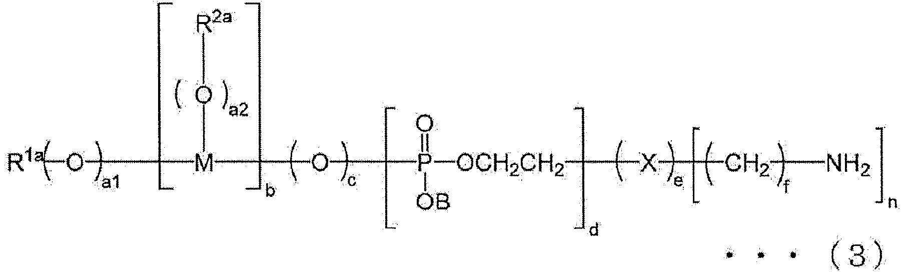 (in the formula (3), each symbol is as defined above) 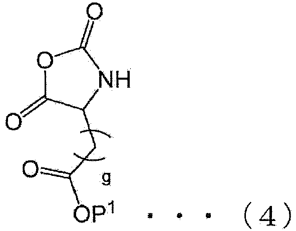 (in the formula (4), P¹ is a carboxy-protecting group, and g is 1 or 2) 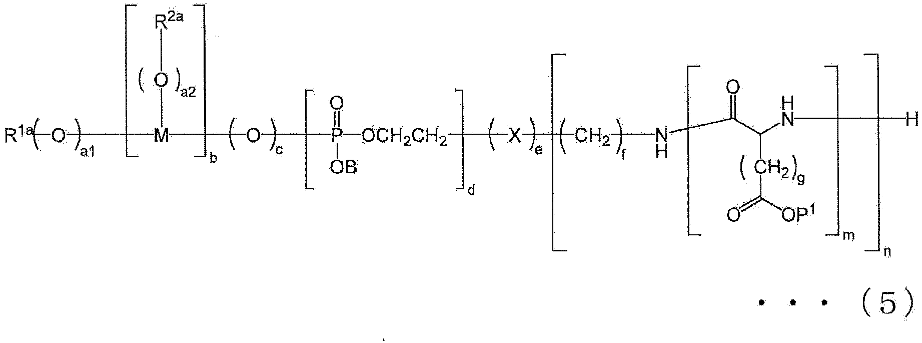 (in the formula (5), each symbol is as defined above) 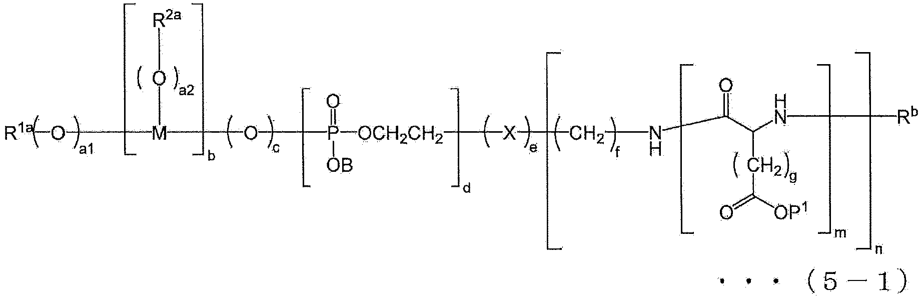 (in the formula (5-1), R^{b} is -COCH₃, or -CO-(CH₂)_{b1}-COOH (wherein b1 is 2 or 3), and other symbols are as defined above)
[step (B)]
   a step of obtaining a polymer represented by the following formula (7-i) by reacting a polymer represented by the following formula (5-i) obtained in the aforementioned step (A) or the aforementioned step (A1) (the polymer represented by the aforementioned formula (5) and the polymer represented by the aforementioned formula (5-1) are comprehensively denoted) with a diethylenetriamine derivative represented by the following formula (6) 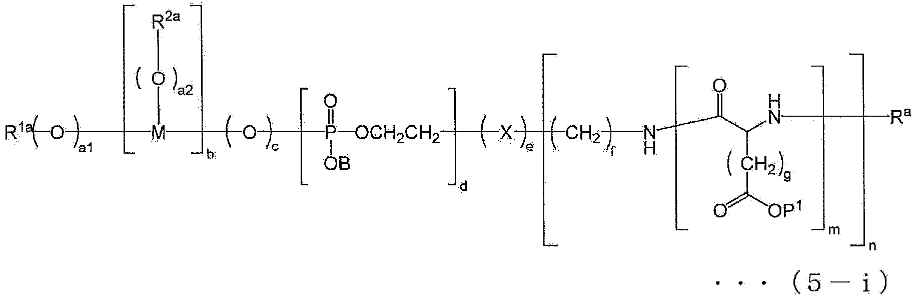 (in the formula (5-i), each symbol is as defined above) 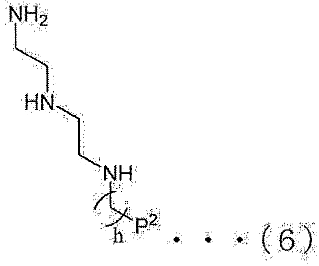 (in the formula (6), P² is a protected carboxy group or a protected sulfo group, and h is as defined above) 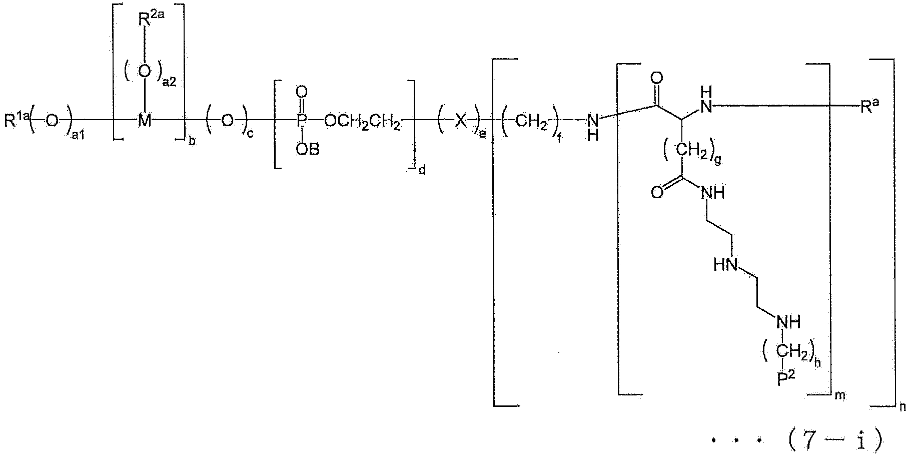 (in the formula (7-i), each symbol is as defined above)
[step (C)]
   a step of obtaining a pH-responsive lipid derivative represented by the aforementioned formula (47-i) by deprotecting a polymer represented by the aforementioned formula (7-i) obtained in the aforementioned step (B).

A method for producing a pH-responsive lipid derivative represented by a structure represented by the following formula (36-i), comprising performing the following step (D): 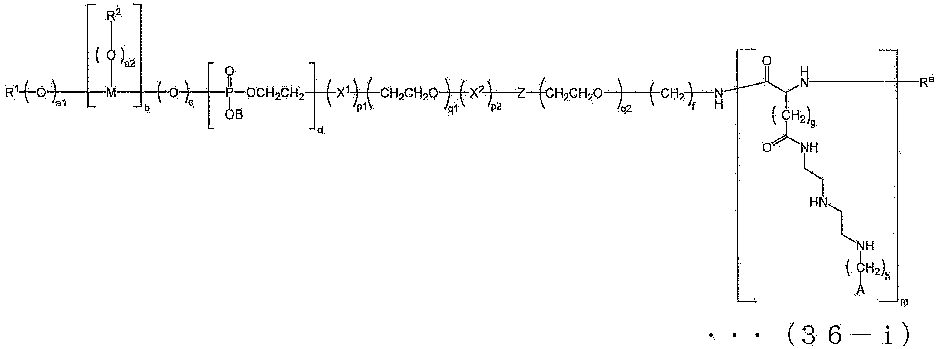 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1,
q1 and q2 are each independently 0 to 50, and
R^{a} is H, -COCH₃, or -CO-(CH₂)_{b1}-COOH (wherein b1 is 2 or 3)
[step (D)]
   a step of obtaining a pH-responsive lipid derivative represented by the aforementioned formula (36-i) by reacting a functional group-containing lipid represented by the following formula (8) with a functional group-containing pH-responsive polymer represented by the following formula (9-i) 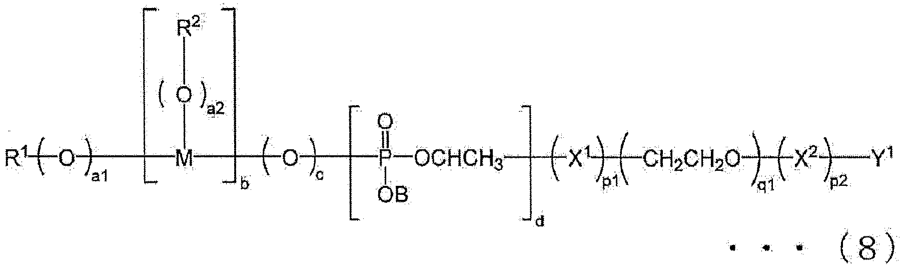 (in the formula (8), Y¹ is an atomic group containing a functional group capable of forming a covalent bond with a functional group contained in the following Y², and other symbols are as defined above) 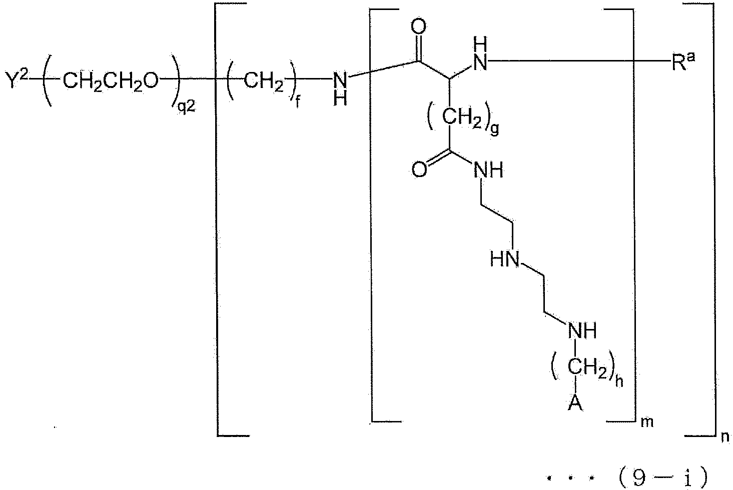 (in the formula (9-i), Y^{Z} is an atomic group containing a functional group capable of forming a covalent bond with a functional group contained in the aforementioned Y¹, and other symbols are as defined above).

A method for producing a pH-responsive lipid derivative represented by a structure represented by the following formula (46-i), comprising performing the following step (E), step (F), and step (G) in this order: 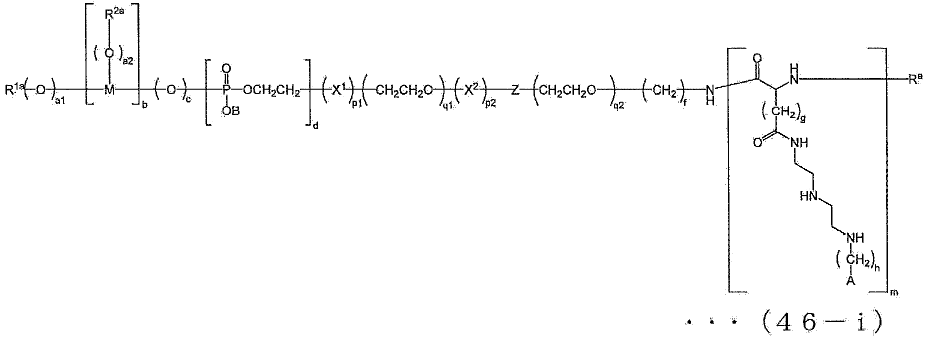 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1,
q1 and q2 are each independently 0 to 50, and
R^{a} is H, -COCH₃, or -CO-(CH₂)_{b1}-COOH (wherein b1 is 2 or 3)
[step (E)]
   a step of obtaining a polymer represented by the following formula (12-i) by reacting a functional group-containing lipid represented by the following formula (10) with a polymer represented by the following formula (11-i) 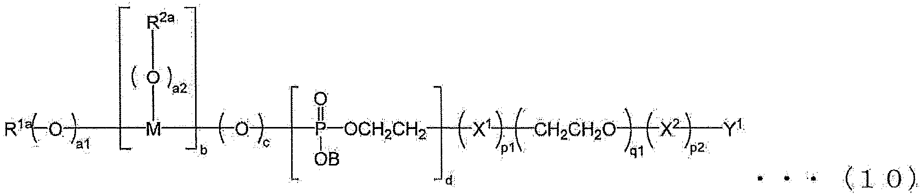 (in the formula (10), Y¹ is an atomic group containing a functional group capable of forming a covalent bond with a functional group contained in the following Y², and other symbols are as defined above) 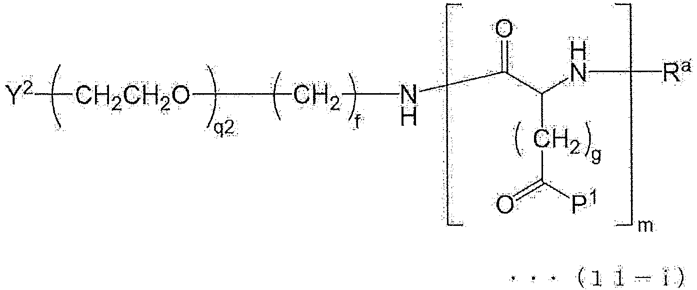 (in the formula (11-i), P¹ is a carboxy-protecting group, Y² is an atomic group containing a functional group capable of forming a covalent bond with a functional group contained in the aforementioned Y¹, and other symbols are as defined above) 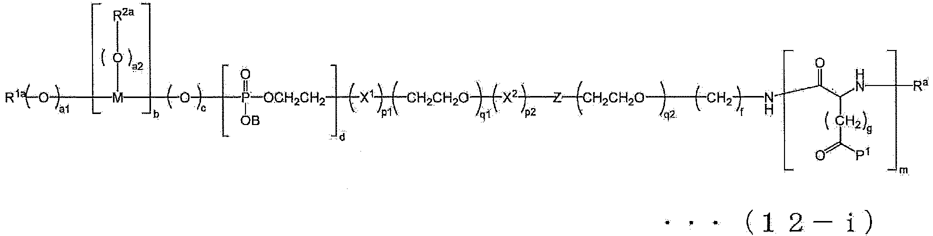 (in the formula (12-i), each symbol is as defined above)
[step (F)]
   a step of obtaining a polymer represented by the following formula (13-i) by reacting a polymer represented by the aforementioned formula (12-i) obtained in the aforementioned step (E) with a diethylenetriamine derivative represented by the aforementioned formula (6) 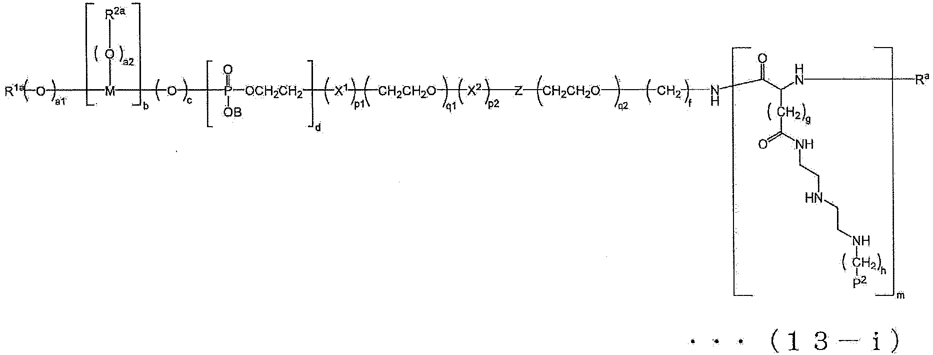 (in the formula (13-i), P² is a protected carboxy group or a protected sulfo group, and other symbols are as defined above)
[step (G)] a step of obtaining a pH-responsive lipid derivative represented by the aforementioned formula (46-i) by deprotecting a polymer represented by the aforementioned formula (13-i) obtained in the aforementioned step (F).

[19] A method for producing a pH-responsive lipid derivative represented by a structure represented by the following formula (47), comprising performing the following step (A2), step (B1), and step (C1) in this order: 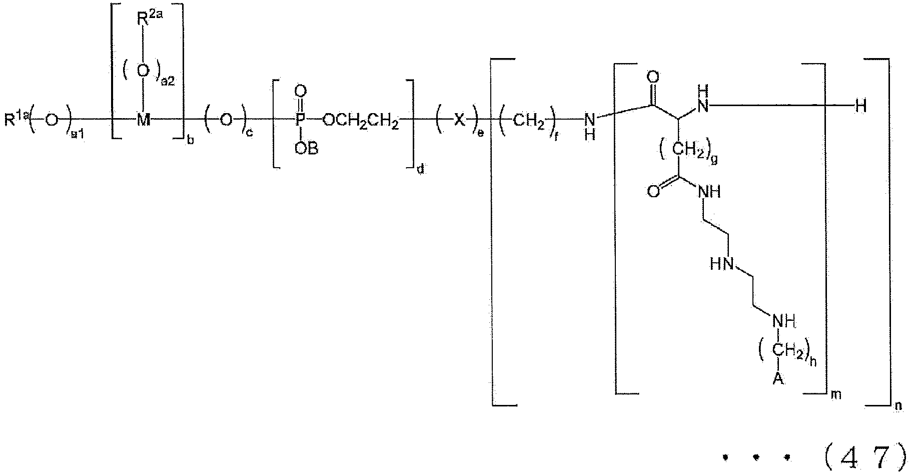 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2, and
m is 5 to 300
[step (A2)]
   a step of obtaining a polymer represented by the following formula (5) by a ring opening polymerization using a polymerization initiator represented by the following formula (3) and an α-amino acid-N-carboxylic acid anhydride represented by the following formula (4) 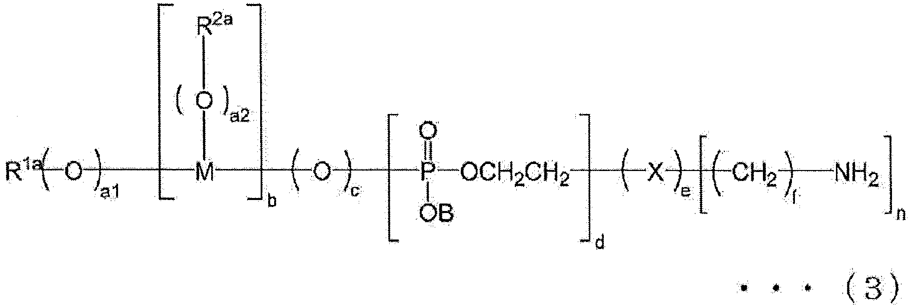 (in the formula (3), each symbol is as defined above) 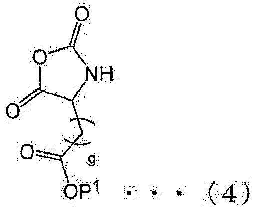 (in the formula (4), P¹ is a carboxy-protecting group, and g is 1 or 2) 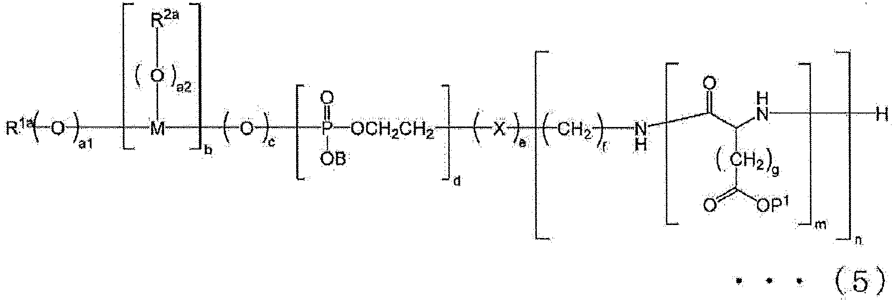 (in the formula (5), each symbol is as defined above)
[step (B1)]
   a step of obtaining a polymer represented by the following formula (7) by reacting a polymer represented by the aforementioned formula (5) obtained in the aforementioned step (A2) with a diethylenetriamine derivative represented by the following formula (6) 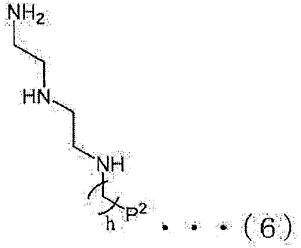 (in the formula (6), P² is a protected carboxy group or a protected sulfo group, and h is as defined above) 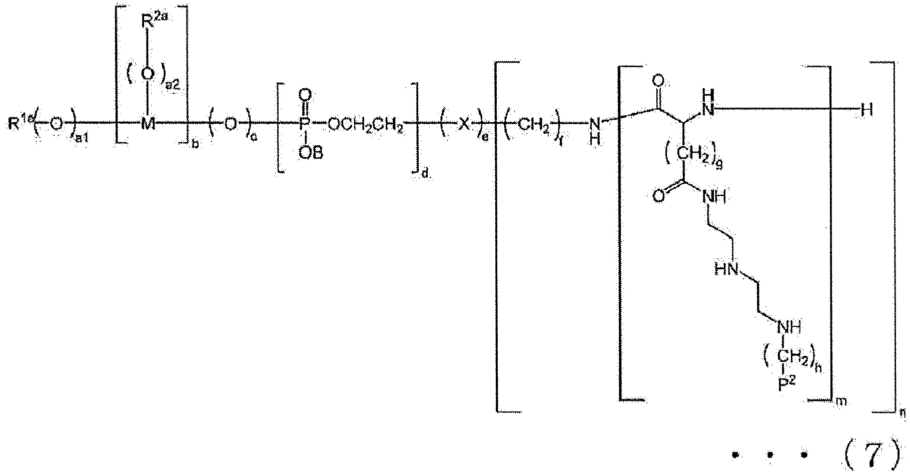
(in the formula (7), each symbol is as defined above)
[step (C1)]
   a step of obtaining a pH-responsive lipid derivative represented by the aforementioned formula (47) by deprotecting a polymer represented by the aforementioned formula (7) obtained in the aforementioned step (B1).

A method for producing a pH-responsive lipid derivative represented by a structure represented by the following formula (36), comprising performing the following step (D1): 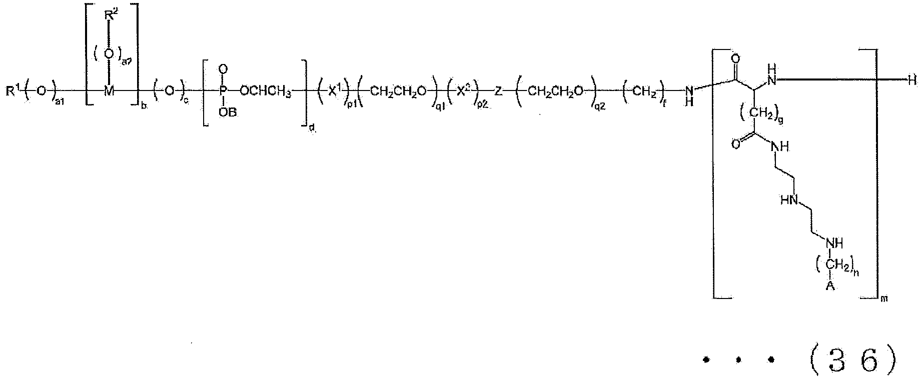 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1, and
q1 and q2 are each independently 0 to 50
[step (D1)]
   a step of obtaining a pH-responsive lipid derivative represented by the aforementioned formula (36) by reacting a functional group-containing lipid represented by the following formula (8) with a functional group-containing pH-responsive polymer represented by the following formula (9) 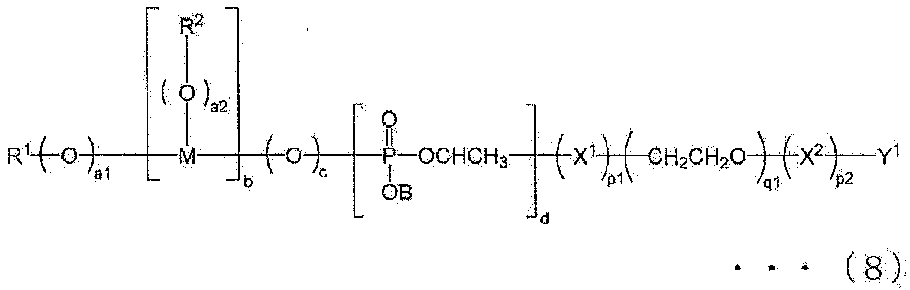 (in the formula (8), Y¹ is an atomic group containing a functional group capable of forming a covalent bond with a functional group contained in the following Y², and other symbols are as defined above) 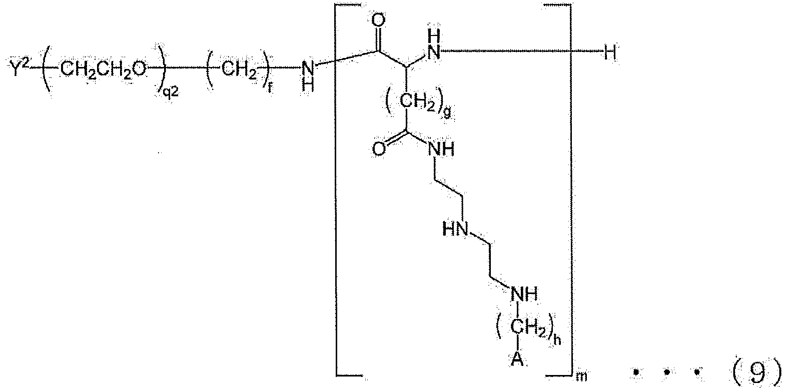 (in the formula (9), Y² is an atomic group containing a functional group capable of forming a covalent bond with a functional group contained in the aforementioned Y¹, and other symbols are as defined above).

A method for producing a pH-responsive lipid derivative represented by a structure represented by the following formula (46), comprising performing the following step (E1), step (F1), and step (G1) in this order: 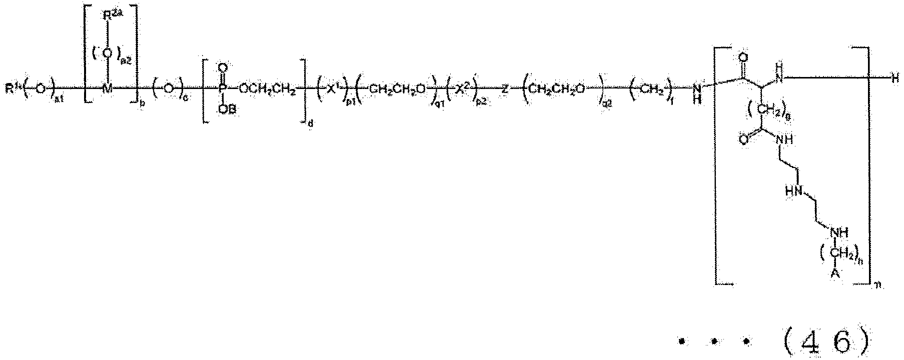 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1, and
q1 and q2 are each independently 0 to 50
[step (E1)]
   a step of obtaining a polymer represented by the following formula (12) by reacting a functional group-containing lipid represented by the following formula (10) with a polymer represented by the following formula (11) 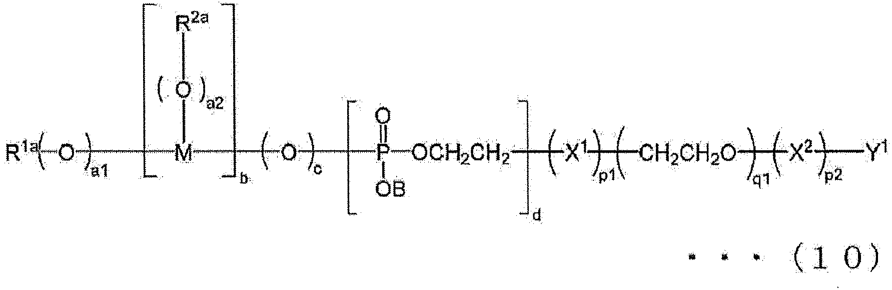 (in the formula (10), Y¹ is an atomic group containing a functional group capable of forming a covalent bond with a functional group contained in the following Y², and other symbols are as defined above) 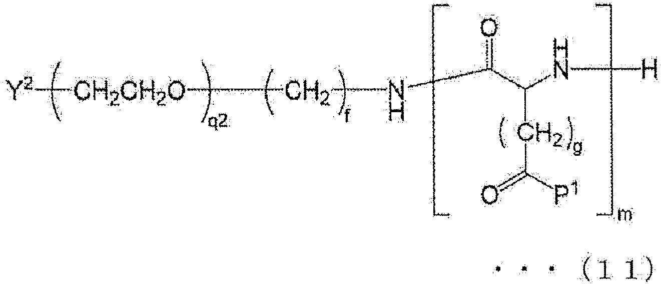 (in the formula (11), P¹ is a carboxy-protecting group, Y² is an atomic group containing a functional group capable of forming a covalent bond with a functional group contained in the aforementioned Y¹, and other symbols are as defined above) 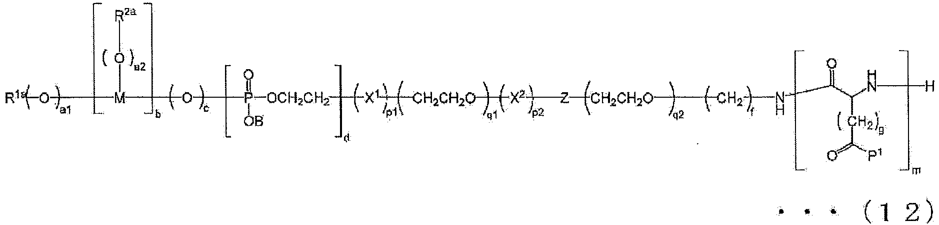 (in the formula (12), each symbol is as defined above)
[step (F1)]
   a step of obtaining a polymer represented by the following formula (13) by reacting a polymer represented by the aforementioned formula (12) obtained in the aforementioned step (E1) with a diethylenetriamine derivative represented by the aforementioned formula (6) 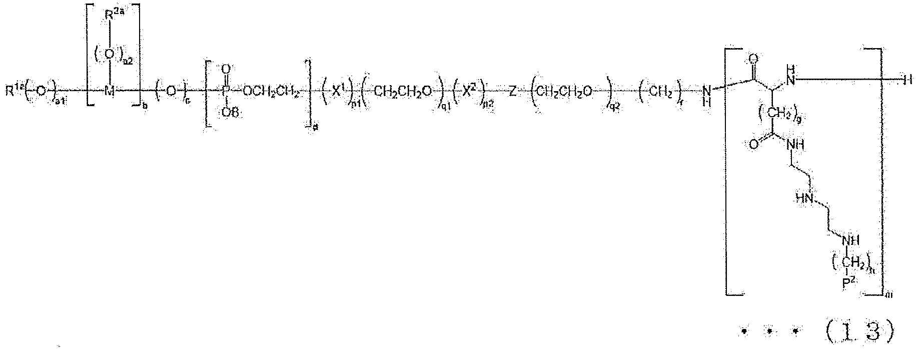 (in the formula (13), P² is a protected carboxy group or a protected sulfo group, and other symbols are as defined above)
[step (G1)]
   a step of obtaining a pH-responsive lipid derivative represented by the aforementioned formula (46) by deprotecting a polymer represented by the aforementioned formula (13) obtained in the aforementioned step (F1).

[22] A method for producing a pH-responsive lipid derivative represented by a structure represented by the following formula (47-1), comprising performing the following step (A3), step (B2), and step (C2) in this order: 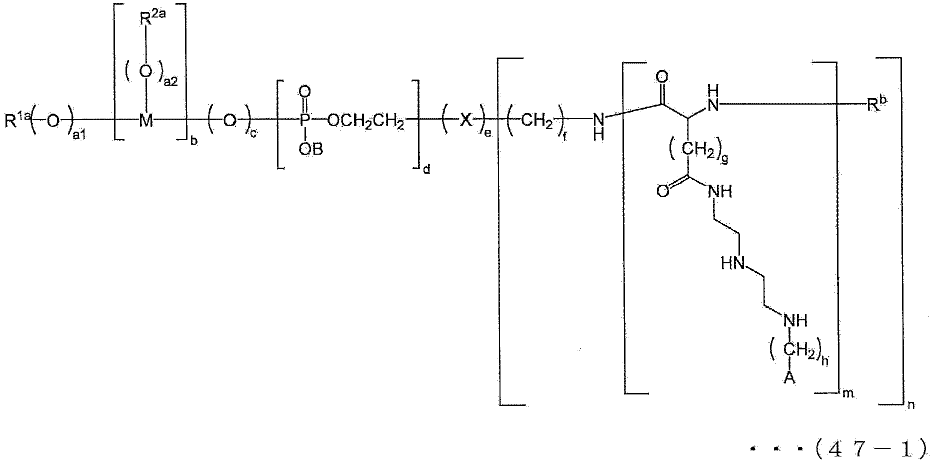 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{b} is -COCH₃, or -CO-(CH₂)_{b1}-COOH (wherein b1 is 2 or 3)
[step (A3)]
   a step of obtaining a polymer represented by the following formula (5) by a ring opening polymerization using a polymerization initiator represented by the following formula (3) and an α-amino acid-N-carboxylic acid anhydride represented by the following formula (4), and further introducing -COCH₃ or -CO-(CH₂)_{b1}-COOH (wherein b1 is 2 or 3) into a terminal amino group of the obtained polymer to obtain a polymer represented by the following formula (5-1) 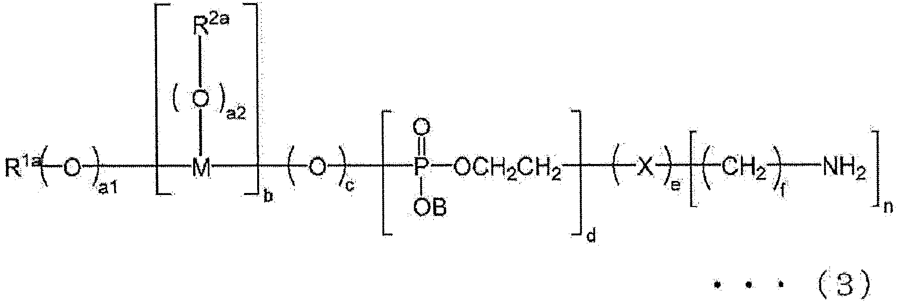 (in the formula (3), each symbol is as defined above) 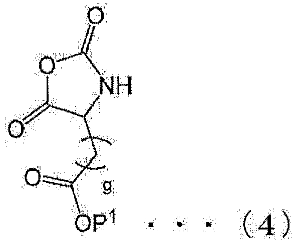 (in the formula (4), P¹ is a carboxy-protecting group, and g is 1 or 2) 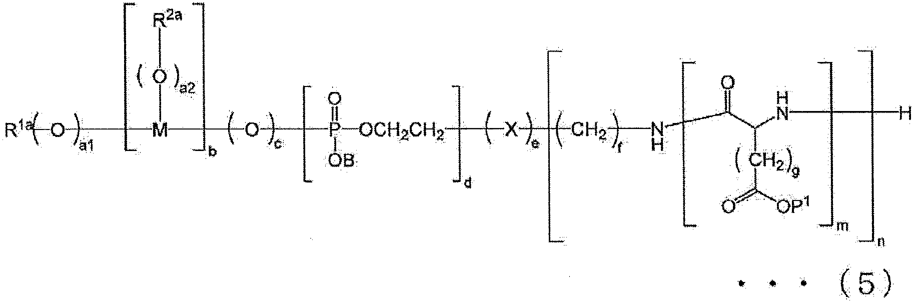 (in the formula (5), each symbol is as defined above) 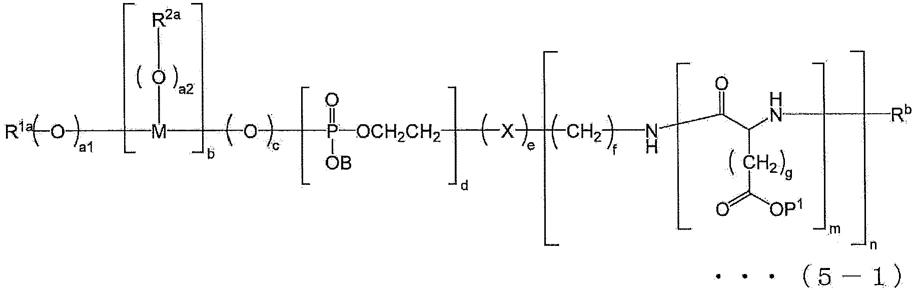 (in the formula (5-1), R^{b} is -COCH₃, or -CO-(CH₂)_{b1}-COOH (wherein b1 is 2 or 3), and other symbols are as defined above)
[step (B2)]
   a step of obtaining a polymer represented by the following formula (7-1) by reacting a polymer represented by the aforementioned formula (5-1) obtained in the aforementioned step (A3) with a diethylenetriamine derivative represented by the following formula (6) 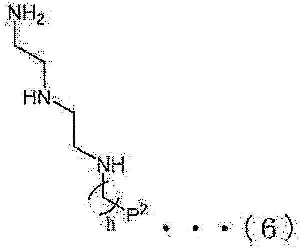 (in the formula (6), P² is a protected carboxy group or a protected sulfo group, and h is as defined above) 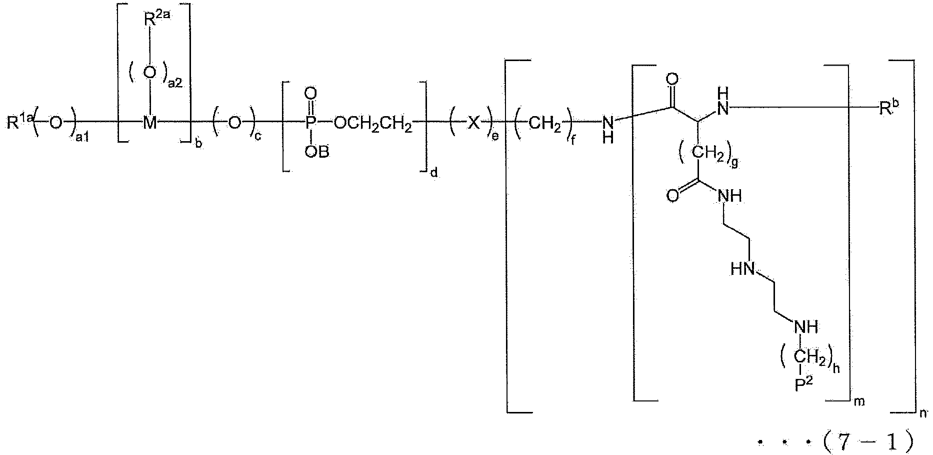 (in the formula (7-1), each symbol is as defined above)
[step (C2)]
   a step of obtaining a pH-responsive lipid derivative represented by the aforementioned formula (47-1) by deprotecting a polymer represented by the aforementioned formula (7-1) obtained in the aforementioned step (B2).

A method for producing a pH-responsive lipid derivative represented by a structure represented by the following formula (36-1), comprising performing the following step (D2): 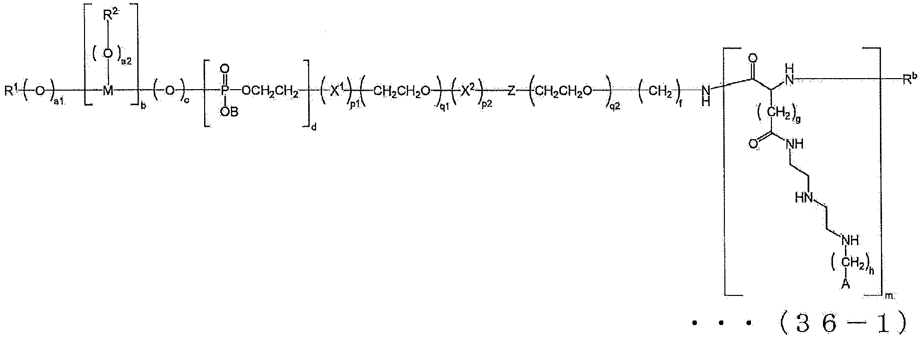 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
R^{b} is COCH₃, or -CO-(CH₂)_{b1}-COOH (wherein b1 is 2 or 3),
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1, and
q1 and q2 are each independently 0 to 50
[step (D2)]
   a step of obtaining a pH-responsive lipid derivative represented by the aforementioned formula (36-1) by reacting a functional group-containing lipid represented by the following formula (8) with a functional group-containing pH-responsive polymer represented by the following formula (9-1) 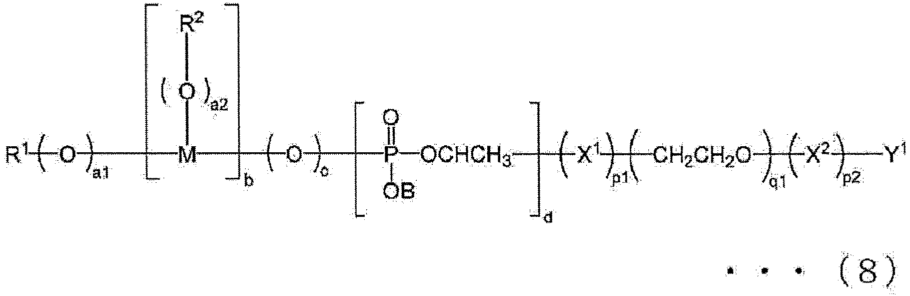 (in the formula (8), Y¹ is an atomic group containing a functional group capable of forming a covalent bond with a functional group contained in the following Y², and other symbols are as defined above) 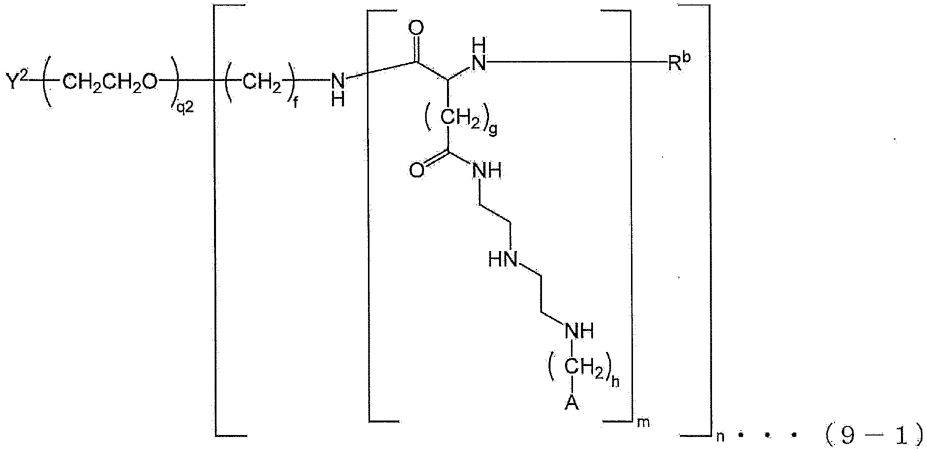 (in the formula (9-1), Y² is an atomic group containing a functional group capable of forming a covalent bond with a functional group contained in the aforementioned Y¹, and other symbols are as defined above).

A method for producing a pH-responsive lipid derivative represented by a structure represented by the following formula (46-1), comprising performing the following step (E2), step (F2), and step (G2) in this order: 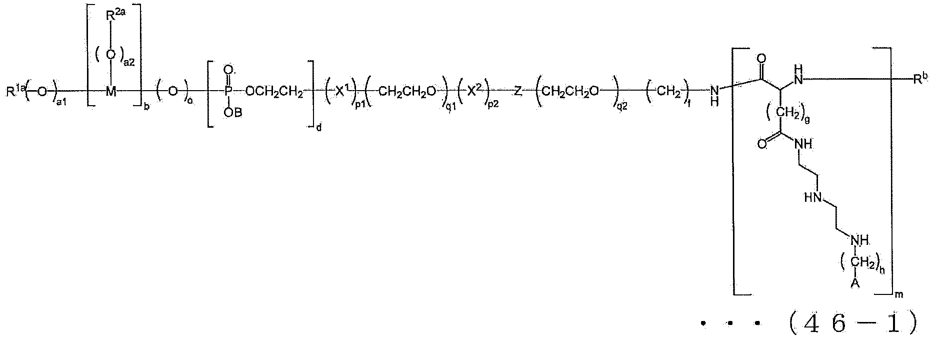 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
R^{b} is -COCH₃, or -CO-(CH₂)_{b1}-COOH (wherein b1 is 2 or 3),
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1, and
q1 and q2 are each independently 0 to 50
[step (E2)]
   a step of obtaining a polymer represented by the following formula (12-1) by reacting a functional group-containing lipid represented by the following formula (10) with a polymer represented by the following formula (11-1) 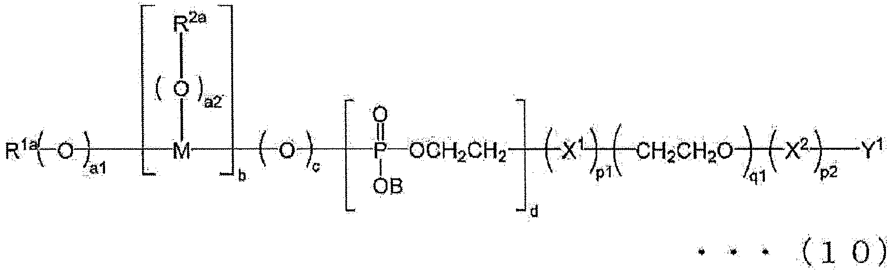 (in the formula (10), Y¹ is an atomic group containing a functional group capable of forming a covalent bond with a functional group contained in the following Y², and other symbols are as defined above) 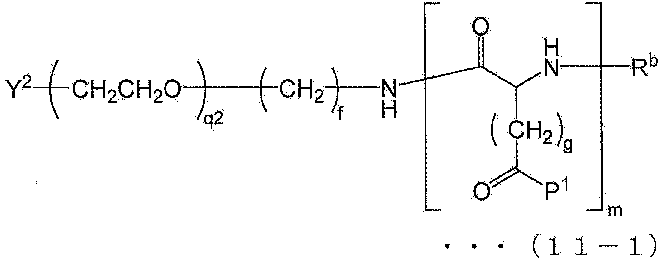 (in the formula (11-1), P¹ is a carboxy-protecting group, Y² is an atomic group containing a functional group capable of forming a covalent bond with a functional group contained in the aforementioned Y¹, and other symbols are as defined above) 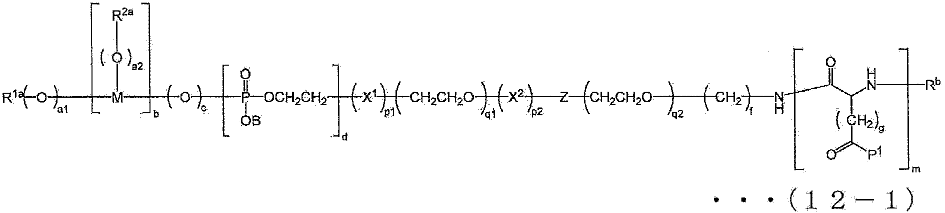 (in the formula (12-1), each symbol is as defined above)
[step (F2)]
   a step of obtaining a polymer represented by the following formula (13-1) by reacting a polymer represented by the aforementioned formula (12-1) obtained in the aforementioned step (E2) with a diethylenetriamine derivative represented by the aforementioned formula (6) 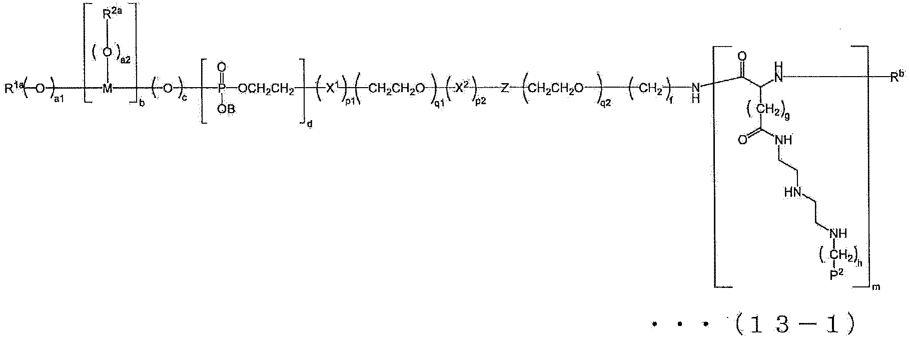 (in the formula (13-1), P² is a protected carboxy group or a protected sulfo group, and other symbols are as defined above)
[step (G2)]
   a step of obtaining a pH-responsive lipid derivative represented by the aforementioned formula (46-1) by deprotecting a polymer represented by the aforementioned formula (13-1) obtained in the aforementioned step (F2).

### 3. Transport carrier for drug delivery containing pH-responsive lipid derivative (i)

A transport carrier for drug delivery comprising 0.1 to 30 mol% of a pH-responsive lipid derivative of any of the above-mentioned [1] to [15].

The transport carrier for drug delivery of the above-mentioned [25], wherein the carrier is a solid lipid nanoparticle.

The transport carrier for drug delivery of the above-mentioned [25], wherein a difference (Q) in the zeta potential represented by the following formula (F1) is not less than 2.5 mV:
formula (F1): Q=(zeta potential at pH 6.5)-(zeta potential at pH 7.4)

The transport carrier for drug delivery of the above-mentioned [25], wherein the difference (Q) in the zeta potential represented by the aforementioned formula (F1) is not less than 2.5 mV and the zeta potential at pH 6.5 is higher than 0.

The transport carrier for drug delivery of the above-mentioned [25], wherein the particle size is 10 to 200 nm.

The transport carrier for drug delivery of the above-mentioned [25], further comprising a phospholipid.

The transport carrier for drug delivery of the above-mentioned [25], further comprising a cationic lipid.

The transport carrier for drug delivery of the above-mentioned [25], wherein the aforementioned cationic lipid is DOTAP (1,2-dioleoyloxy-3-trimethylammonium propane) or DODAP (dioleoyl-3-dimethylammonium propane).

The transport carrier for drug delivery of the above-mentioned [25], further comprising sterol.

The transport carrier for drug delivery of the above-mentioned [25], comprising 0.1 to 30 mol% of the pH-responsive lipid derivative of any of the above-mentioned [1] to [15], 0 to 60 mol% of the phospholipid, 1 to 60 mol% of the cationic lipid, and 0 to 40 mol% of sterol.

The transport carrier for drug delivery of the above-mentioned [25], wherein the transport drug is a nucleic acid derivative.

The transport carrier for drug delivery of the above-mentioned [25], wherein the transport drug is peptide.

The transport carrier for drug delivery of the above-mentioned [25], wherein the transport drug is a low-molecular-weight drug or an anticancer agent.

The transport carrier for drug delivery of the above-mentioned [25], wherein the pH-responsive lipid derivative is the pH-responsive lipid derivative of the above-mentioned [2].

The transport carrier for drug delivery of the above-mentioned [25], wherein the pH-responsive lipid derivative is the pH-responsive lipid derivative of the above-mentioned [3].

As another preferred embodiment of the pH-responsive lipid derivative of the present invention, for example, the following can be mentioned.
[1A] A pH-responsive lipid derivative represented by a structure represented by the following formula (1): 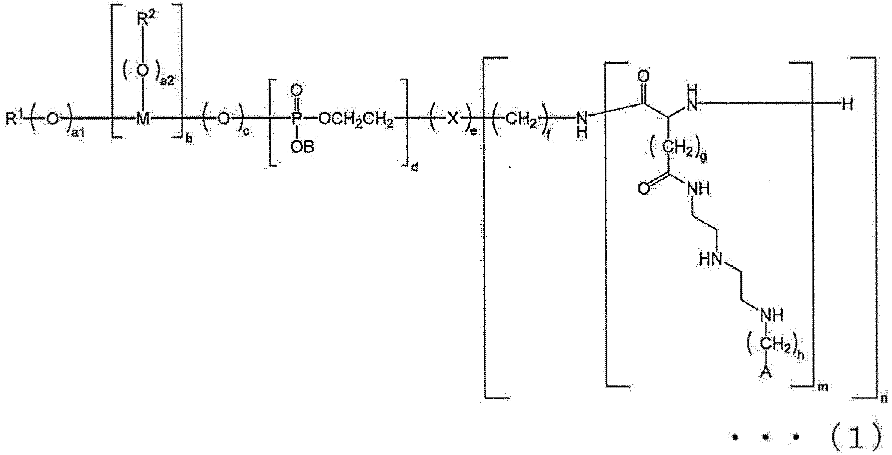 wherein
   R¹ and R² are each independently an alkyl group having 8 to 24 carbon atoms, or an acyl group having 8 to 24 carbon atoms, or a sterol residue,
   M is a trivalent group represented by>N-CH₂-CH₂- or an optionally branched hydrocarbon trivalent group having 3 to 7 carbon atoms,
   B is a hydrogen atom, an alkali metal, or an ammonium group,
   X is a linking group containing a bonding group formed by a nucleophilic substitution reaction, a Michael addition reaction, a thiol-ene reaction, or a cyclization addition reaction,
   A is a carboxy group or a sulfo group,
   a1, a2, b, c, d, and e are each independently 0 or 1,
   f is an integer of 0 to 5,
   h is an integer of 1 to 3,
   g and n are each independently 1 or 2, and
   m is 5 to 300.
[2A] The pH-responsive lipid derivative of the above-mentioned [1A], wherein X in the aforementioned formula (1) is a linking group represented by the following formula (2) 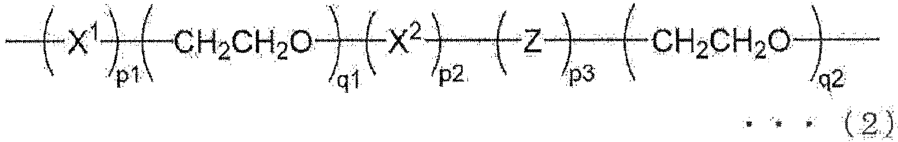 (in the formula (2), X¹ and X² are each independently a group having 1 to 5 carbon atoms and containing a bonding group resulting from a nucleophilic substitution reaction, Z is a group formed by a Michael addition reaction, a thiol-ene reaction, or a cyclization addition reaction, p1, p2 and p3 are each independently 0 or 1, and q1 and q2 are each independently 0 to 50).
[3A] The pH-responsive lipid derivative of the above-mentioned [1A] or [2A], wherein m in the aforementioned formula (1) is 5 to 150.
[4A] The pH-responsive lipid derivative of any of the above-mentioned [1A] to [3A], wherein, in the aforementioned formula (1), g and h are each 2, and A is a carboxy group.
[5A] The pH-responsive lipid derivative of any of the above-mentioned [1A] to [4A], wherein, in the aforementioned formula (1), R¹ and R² are each independently an alkyl group having 8 to 24 carbon atoms,
   b and n are each 1, and
   d is 0.
[6A] The pH-responsive lipid derivative of any of the above-mentioned [1A] to [5A], wherein, in the aforementioned formula (1),
   R¹ and R² are each independently an alkyl group having 8 to 24 carbon atoms,
   M is an optionally branched hydrocarbon trivalent group having 3 to 7 carbon atoms,
   A is a carboxy group,
   a1, a2, b, and n are each 1,
   c, d, e, and f are each 0, and
   g and h are each 2.
[7A] The pH-responsive lipid derivative of any of the above-mentioned [1A] to [4A], wherein, in the aforementioned formula (1), R¹ is a sterol residue, and a1, b, c, and d are each 0.
[8A] The pH-responsive lipid derivative of any of the above-mentioned [1A] to [4A], wherein, in the aforementioned formula (1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction, and p1, p2, and p3 are each 1.
[9A] The pH-responsive lipid derivative of any of the above-mentioned [1A] to [4A] and [8A], wherein, in the aforementioned formula (1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q2 is 0 to 5.
[10A] The pH-responsive lipid derivative of any of the above-mentioned [1A] to [4A], [8A] or [9A], wherein, in the aforementioned formula (1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group, a1, a2, b, c, d, e, and n are each 1,
   f is an integer of 2 to 5, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 is 0 to 5.
[11A] The pH-responsive lipid derivative of any of the above-mentioned [1A] to [4A], or [8A] to [10A], wherein, in the aforementioned formula (1),
   R¹ and R² are each independently an acyl group having 8 to 24 carbon atoms,
   M is an optionally branched hydrocarbon trivalent group having 3 to 7 carbon atoms,
   B is a hydrogen atom, an alkali metal, or an ammonium group,
   A is a carboxy group,
   a1, a2, b, c, d, e, and n are each 1,
   f is an integer of 2 to 5,
   g and h are each 2, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 and q2 are each independently 0 to 5.
[12A] The pH-responsive lipid derivative of any of the above-mentioned [1A] to [4A], wherein, in the aforementioned formula (1),
   R¹ and R² are each independently an alkyl group having 8 to 24 carbon atoms or an acyl group having 8 to 24 carbon atoms,
   b, e, and n are each 1,
   d is 0,
   f is an integer of 2 to 5, and
   for X in the aforementioned formula (2),
   X¹ and X² are each a group having 1 to 5 carbon atoms and containing a bonding group resulting from a nucleophilic substitution reaction, and
   p1, p2, and p3 are each 1.
[13A] The pH-responsive lipid derivative of any of the above-mentioned [1A] to [4A] or [12A], wherein, in the aforementioned formula (1),
   R¹ and R² are each independently an alkyl group having 8 to 24 carbon atoms or an acyl group having 8 to 24 carbon atoms,
   A is a carboxy group,
   b, e, and n are each 1,
   d is 0,
   f is an integer of 2 to 5,
   g and h are each 2, and
   for X in the aforementioned formula (2),
   X¹ and X² are each a group having 1 to 5 carbon atoms and containing a bonding group resulting from a nucleophilic substitution reaction,
   Z is a group formed by a cyclization addition reaction,
   p1, p2, and p3 are each 1, and
   q1 and q2 are each independently 0 to 5.

### [Advantageous Effects of Invention]

The pH-responsive lipid derivative of the present invention has the property of being electrically neutral under neutral conditions (pH 7.4) and changing to cationic under weakly acidic conditions (pH 6.5) around tumor tissues. A transport carrier for drug delivery which is represented by spherical micelles and liposomes containing the pH-responsive lipid derivative of the present invention exhibits stealth property in blood components and normal tissues which are under neutral environment in vivo, and increases cationicity in response to minute pH changes around tumor tissues, which improves the accumulation efficiency and uptake efficiency into tumor tissues. As a result, the low-molecular-weight drugs or nucleic acid pharmaceutical products encapsulated in the transport carrier can be released around the tumor tissues or introduced efficiently into tumor cells.

Furthermore, the pH-responsive lipid derivative of the present invention is composed of a polymer of amino acids contained in living organisms. Therefore, after delivery of a low-molecular-weight or nucleic acid pharmaceutical product, it is decomposed within the body and is not accumulated within the body.

According to the present invention, a pH-responsive transport carrier which is effective as a transport carrier capable of achieving an introduction system that can safely and efficiently perform delivery of low-molecular-weight drugs, nucleic acid pharmaceutical products, and the like to around tumor tissues and transfection thereof to tumor cells, and further, a pH-responsive lipid derivative useful for forming such transport carriers can be provided.

### [Brief Description of Drawings]

[Fig. 1]

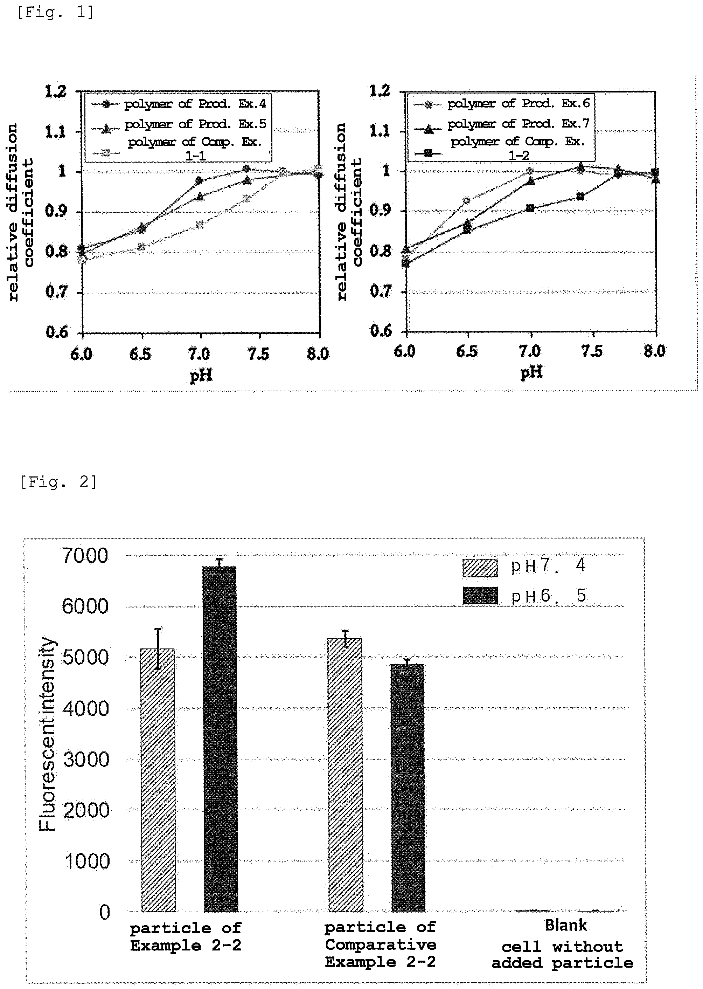

   Fig. 1 shows the evaluation results of the pH responsiveness of polymers subjected to the test in the presence of heparin in Experimental Example 1 described later.
[Fig. 2]
   Fig. 2 shows the degree of siRNA uptake into cells from particles subjected to the test in Experimental Example 3 described later.
[Fig. 3]

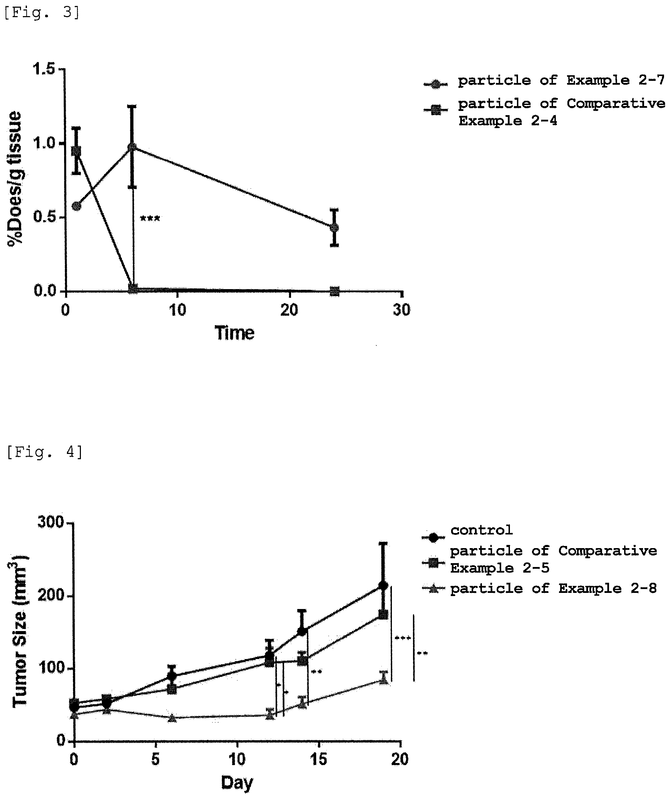

   Fig. 3 shows the evaluation results of tumor accumulation of siRNA from particles subjected to the test in Experimental Example 4 described later.
[Fig. 4]
   Fig. 4 shows the evaluation results of the antitumor effect of siPLK1 using particles subjected to the test in Experimental Example 5 described later.

### [Description of Embodiments]

Preferred embodiments of the present invention are explained in detail below.

### <pH-responsive lipid derivative>

The pH-responsive lipid derivative (i) of the present invention is represented by the following structure.

A pH-responsive lipid derivative represented by 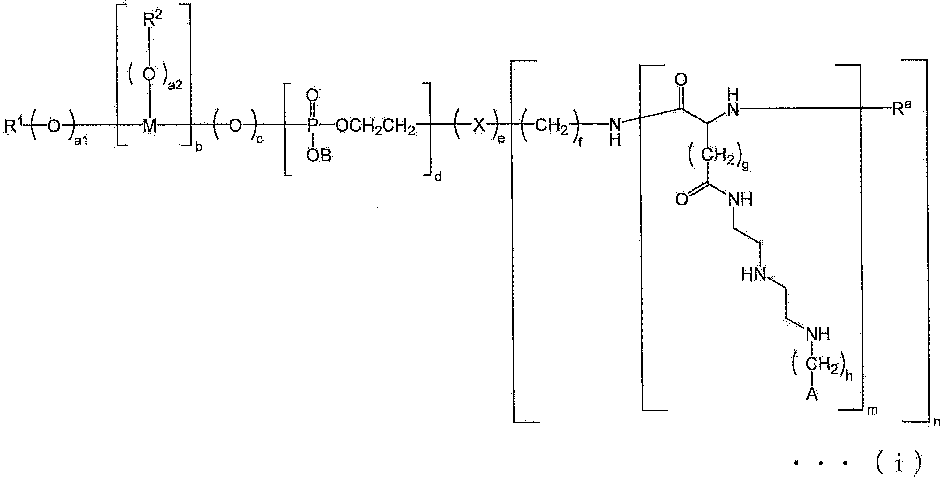 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{a} is H, -COCH₃, or -CO-(CH₂)_{b1}-COOH (wherein b1 is 2 or 3) .

The pH-responsive lipid derivative (i) includes two preferred embodiments: (A) pH-responsive lipid derivative (1) and (B) pH-responsive lipid derivative (1-1). These three derivatives differ structurally only at R^{a}. That is, (A) pH-responsive lipid derivative (1) is one of the preferred embodiments and R^{a} is H therein, and (B) pH-responsive lipid derivative (1-1) is another preferred embodiment and R^{a} is - COCH₃, or -CO-(CH₂)_{b1}-COOH (wherein b1 is 2 or 3) therein. The pH-responsive lipid derivative (i) includes the both and R^{a} is H, -COCH₃, or -CO-(CH₂)_{b1}-COOH (wherein b1 is 2 or 3). Other structural characteristics (R¹, R², M, B, X, A, a1, a2, b, c, d, e, f, h, g, n and m) are common in the three derivatives.

Accordingly, the pH-responsive lipid derivatives (i) of the present invention are described below, taking (A) pH-responsive lipid derivative (1) as a specific representative example thereof. A person skilled in the art can similarly understand pH-responsive lipid derivative (i) and pH-responsive lipid derivative (1-1) based on the description.

### (A) pH-responsive lipid derivative (1)

The pH-responsive lipid derivative of the present invention is represented by the following formula (1):

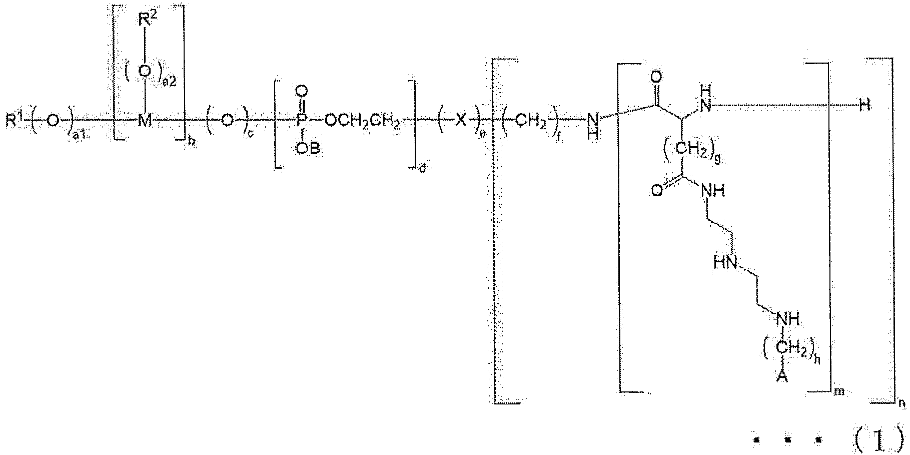

In the formula (1), R¹ and R² are each independently an alkyl group having 8 to 24 carbon atoms, or an acyl group having 8 to 24 carbon atoms, or a sterol residue.

In the formula (1), alkyl group having 8 to 24 carbon atoms for R¹, R² may be linear or branched and may contain an unsaturated bond. Specifically, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, octadienyl group, nonadienyl group, decadienyl group, undecadienyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, tetramethylhexadecenyl group (phytyl group) and the like can be mentioned. Aliphatic hydrocarbon groups having 10 to 20 carbon atoms such as decyl group, dodecyl group, tetradecyl group, hexadecyl group, octadecyl group, icosyl group, decenyl group, dodecenyl group, tetradecenyl group, hexadecenyl group, octadecenyl group, icosenyl group, decadienyl group, dodecadienyl group, tetradecadienyl group, hexadecadienyl group, octadecadienyl group, icosadienyl group and the like are preferred, and aliphatic hydrocarbon groups having 14 to 20 carbon atoms such as tetradecyl group, hexadecyl group, octadecyl group, icosyl group and the like are more preferred.

In the formula (1), an acyl group having 8 to 24 carbon atoms for R¹, R² may be linear or branched and may contain an unsaturated bond. Specifically, octanoyl group, nonanoyl group, decanoyl group, undecanoyl group, dodecanoyl group, tridecanoyl group, tetradecanoyl group, pentadecanoyl group, hexadecanoyl group, heptadecanoyl group, octadecanoyl group, nonadecanoyl group, icosanoyl group, henicosanoyl group, docosanoyl group, octaenoyl group, nonaenoyl group, decaenoyl group, undecaenoyl group, dodecaenoyl group, tridecaenoyl group, tetradecaenoyl group, pentadecaenoyl group, hexadecaenoyl group, heptadecaenoyl group, octadecaenoyl group, nonadecaenoyl group, icosaenoyl group, henicosaenoyl group, docosaenoyl group, octadienoyl group, nonadienoyl group, decadienoyl group, undecadienoyl group, dodecadienoyl group, tridecadienoyl group, tetradecadienoyl group, pentadecadienoyl group, hexadecadienoyl group, heptadecadienoyl group, octadecadienoyl group, nonadecadienoyl group, icosadienoyl group, henicosadienoyl group, docosadienoyl group, octadecatrienoyl group, icosatrienoyl group, icosatetraenoyl group, icosapentaenoyl group, docosahexaenoyl group, isostearoyl group, tetramethylhexadecanoyl group (phytanoyl group), retinoyl group, and the like can be mentioned. Acyl groups having 10 to 20 carbon atoms such as decanoyl group, dodecanoyl group, tetradecanoyl group, hexadecanoyl group, octadecanoyl group, icosanoyl group, decaenoyl group, dodecaenoyl group, tetradecaenoyl group, hexadecaenoyl group, octadecaenoyl group, icosaenoyl group, decadienoyl group, dodecadienoyl group, tetradecadienoyl group, hexadecadienoyl group, octadecadienoyl group, icosadienoyl group, and the like are preferred, and acyl groups having 14 to 20 carbon atoms such as tetradecanoyl group, hexadecanoyl group, octadecanoyl group, icosanoyl group, and the like are more preferred.

Examples of the sterol residue for R¹ or R² in the formula (1) include cholesteryl group (cholesterol residue), cholestaryl group (cholestanol residue), stigmasteryl group (stigmasterol residue), β-sitosteryl group (β-sitosterol residue), lanosteryl group (lanosterol residue), and ergosteryl group (ergosterol residue), and the like. The sterol residue is preferably a cholesteryl group or a cholestaryl group.

In the formula (1), a1 and a2 are each independently 0 or 1.

In the formula (1), b is 0 or 1. When b=0, the obtained lipid derivative is a single strand lipid derivative, and when b=1, it is a double-stranded lipid derivative having M as a branched moiety.

M is a trivalent group represented by>N-CH₂-CH₂- or an optionally branched hydrocarbon trivalent group having 3 to 7 carbon atoms (trivalent group derived from linear or branched hydrocarbon having 3 to 7 carbon atoms), and is not particularly limited as long as it is trivalent. For example, groups represented by the following formulas (14) to (16) can be mentioned. In the following formulas, other groups are also indicated in order to clarify the bonding relationships between group M and other groups. 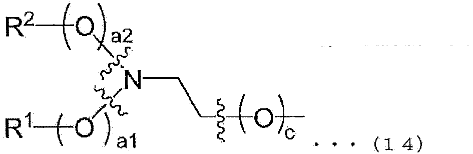 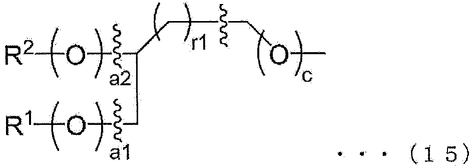 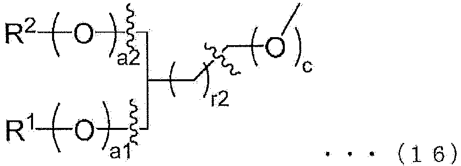 (in the above-mentioned formulas, r1 is an integer of 1 to 5, r2 is an integer of 0 to 4,
R¹ and R² are each as defined above
a1 and a2 are each as defined above,
c is as defined below, and 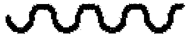 is a bonding position of trivalent group M to other three groups.)

In the formula (1), c is 0 or 1.

In the formula (1), d is 0 or 1. When d=1, the formula (1) shows a phospholipid. In the formula (1), B is a hydrogen atom, an alkali metal, or an ammonium group. As the alkali metal, lithium, sodium, potassium, or the like can be mentioned.

In the formula (1), e is 0 or 1.

In the formula (1), n is 1 or 2. When n=1, the obtained lipid derivative is a derivative having one pH-responsive polymer, and when n=2, it is a derivative having two pH-responsive polymers.

In the formula (1), X is a linking group containing a bonding group formed by a nucleophilic substitution reaction, a Michael addition reaction, a thiol-ene reaction, or a cyclization addition reaction.

### (When e=1 and n=1)

When e=1 and n=1, X is not particularly limited as long as it is a divalent organic group, and is preferably a linking group represented by the following formula (2):

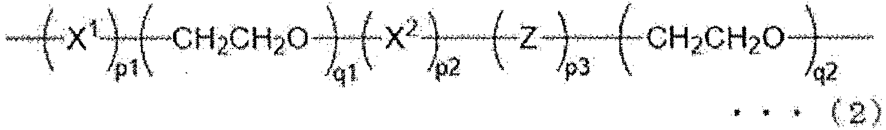

In the formula (2), p1 and p2 are each independently 0 or 1.

In the formula (2), X¹ and X² are each independently a group having 1 to 5 carbon atoms and containing a bonding group resulting from a nucleophilic substitution reaction, which is constituted of a bond formed by a nucleophilic substitution reaction and a spacer.

Examples of the bond that can be formed by a nucleophilic substitution reaction include ester bond, urethane bond, amide bond, ether bond, carbonate bond, divalent hydrocarbon group containing secondary amino group, single bond, and divalent hydrocarbon group. The spacer is not particularly limited as long as it is a site composed of a covalent bond. For example, a structure shown by the formula (17) or the formula (18) can be mentioned.

-(CH₂)ₛ₁- ... (17)

(in the formula (17), s1, is an integer of 1 to 4.)

- (CH₂)ₛ₂-O-(CH₂)ₛ₃- ··· (18)

(in the formula (18), s2 is an integer of 1 to 3, s3 is an integer that satisfies the formula 4-s2.)

In the formula (2), q1 and q2 show the degree of polymerization of the ethyleneglycol structure, and are each independently 0 to 50. When q1 or q2 becomes larger, the proportion of the hydrophilic portion of the pH-responsive lipid derivative also becomes larger. The setting can be arbitrarily determined according to the required performance and the like.

In the formula (2), p3 is 0 or 1. When p3 is 0, the below-mentioned polymerization initiator can be used as a starting material for production. When p3 is 1, production is possible by reacting the below-mentioned functional group-containing lipid with a functional group-containing pH-responsive polymer.

In the formula (2), Z is a group formed by a Michael addition reaction, a thiol-ene reaction, or a cyclization addition reaction and, for example, the following formulas (19) to (27) can be mentioned. In the following formulas, other groups are also indicated in order to clarify the bonding relationships between Z and other groups. 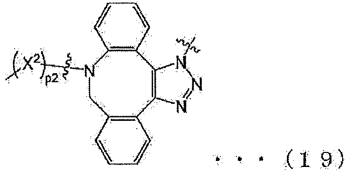 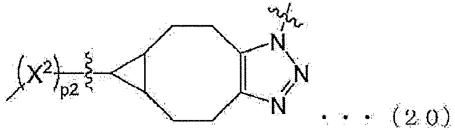 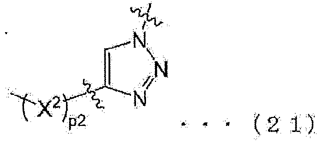 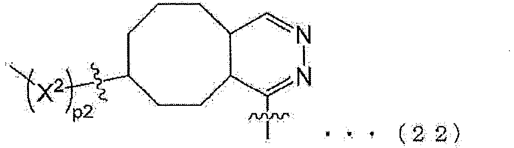 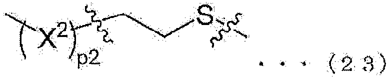 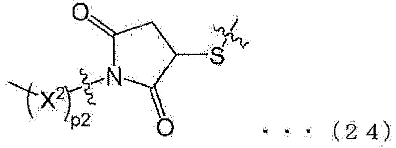 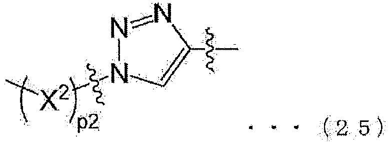 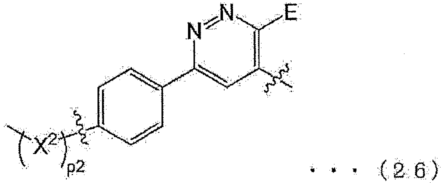 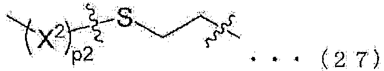 (in the above-mentioned formulas, E is a hydrogen atom or a methyl group,
X² and p2 are each as defined above, and 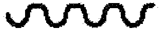 shows the bonding position of group Z to other two groups.)

### (When e=1 and n=2)

When e=1 and n=2, X is a trivalent organic group and is not particularly limited as long as it is a trivalent. For example, groups represented by the following formula (28) to formula (30) can be mentioned: 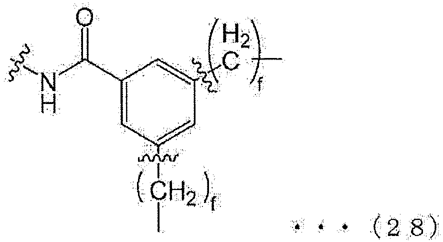 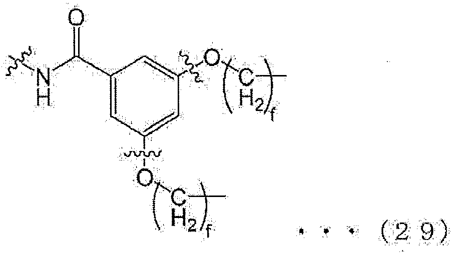 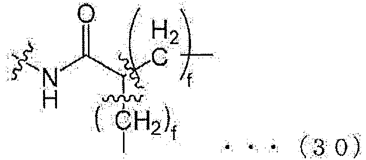 (in the above-mentioned formulas, f is an integer of 0 to 5, 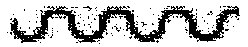 shows the bonding position of X to other three groups.)

In the formula (1), g is 1 or 2. When g=1, the pH-responsive lipid derivative is decomposed in vivo to aspartic acid or a derivative thereof, and when g=2, it is decomposed to glutamic acid or a derivative thereof. Therefore, it is excreted from the body without being accumulated in the body.

In the formula (1), h=1 to 3. When h=not less than 4, it becomes cationic even under neutral conditions of pH 7.4. Therefore, it is difficult to exhibit stealth property against blood components and normal tissues that are in a neutral environment in vivo.

In the formula (1), A is a carboxy group or a sulfo group.

The functional group represented by the following formula (31) including A and h in the formula (1) has property of exhibiting neutrality at pH 7.2 to 7.6 and changing to cationicity at pH 6.0 to 6.6. That is, polymers containing the pH-responsive functional group as a repeat unit also have similar pH-responsiveness. Furthermore, a lipid derivative represented by the formula (1) and having the pH-responsive polymer also has similar pH-responsiveness.

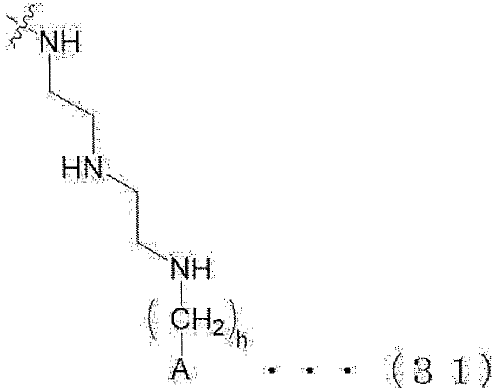

In the formula (1), m is 5 to 300, the range of m varies depending on the lipid to be bonded and is preferably 5 to 200, most preferably 5 to 150. It is particularly preferably 5 to 110.

When m is smaller than 5, the content of pH-responsive functional group in the pH-responsive lipid derivative may be low, making it difficult to form pH-responsive lipid nanoparticles. When m is larger than 300, the proportion of hydrophilic moiety in the pH-responsive lipid derivative becomes large, and the pH-responsive lipid derivative becomes easily detached from the produced lipid nanoparticles.

The degree of polymerization of the pH-responsive lipid derivative according to the present embodiment, i.e., m, can be adjusted according to the required property and the like, for example, by the polymerization conditions of the intermediate polymer.

While the N-terminal of the peptide structure in the formula (1) is described as a hydrogen atom, for example, an onium salt structure by pH change or a γ-lactam ring by an intramolecular nucleophilic substitution reaction may be formed instead of the terminal amino group, depending on the synthesis method of the peptide skeleton, the production method of the pH-responsive polymer, the storage conditions, and the like.

In addition, as described below, when there is a risk of side reaction in subsequent reactions, and the like, the amino group at the N-terminal may be protected or converted to a functional group such as an acetyl group or carboxylic acid, thereby leading to (B) pH-responsive polymer (1-1) represented by the following formula: 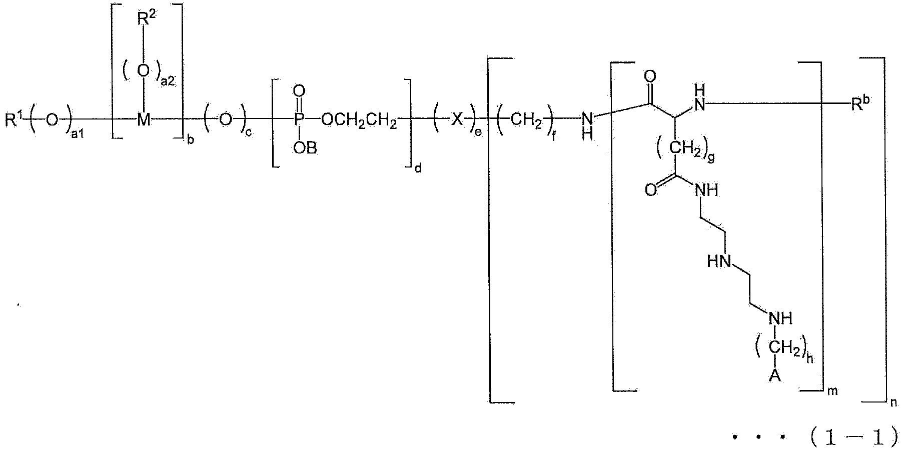 (in the above-mentioned formula, R^{b} is -COCH₃, or -CO-(CH₂)_{b1}-COOH (wherein b1 is 2 or 3), and for each other symbol, the detailed description of pH-responsive polymer (1) can be referred to).

The structure represented by the formula (1) is preferably a structure represented by any of the following formulas (32) to (34): 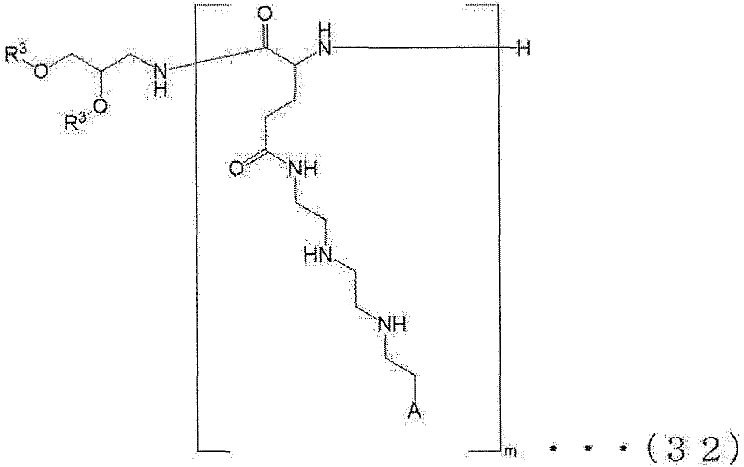
(R³ is an alkyl group having 14 to 20 carbon atoms, A is a carboxy group or a sulfo group, and m is 5 to 150) 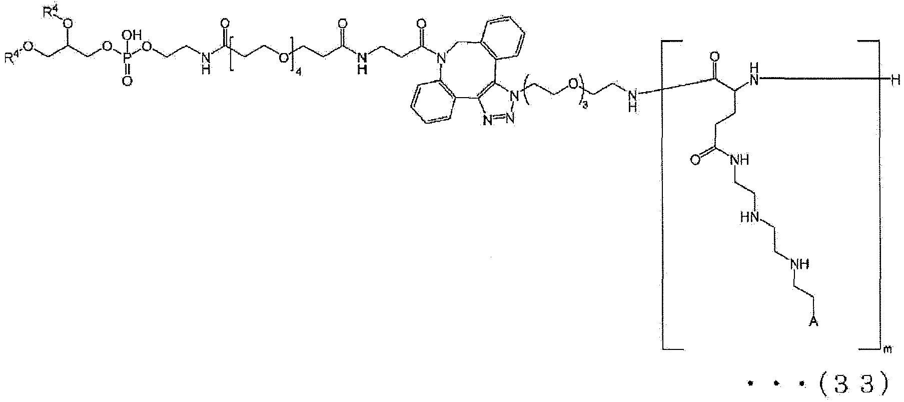
(R⁴ is an alkyl group having 14 to 20 carbon atoms or an acyl group having 14 to 20 carbon atoms, A is a carboxy group or a sulfo group, and m is 5 to 150) 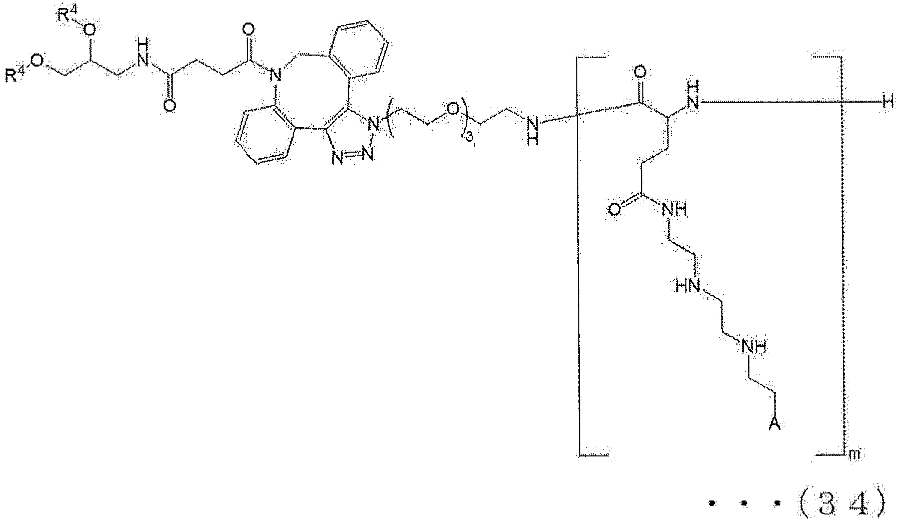
(R⁴ is an alkyl group having 14 to 20 carbon atoms or an acyl group having 14 to 20 carbon atoms, A is a carboxy group or a sulfo group, and m is 5 to 150.)

### <Production method of pH-responsive lipid derivative>

The pH-responsive lipid derivative (i) of the present invention can be produced by, as described above, performing step (A), step (B), and step (C) in this order when R^{a} in the aforementioned formula (47-i) is H, or performing step (A1), step (B), and step (C) in this order when R^{a} in the aforementioned formula (47-i) is -COCH₃ or -CO-(CH₂)_{b1}-COOH (wherein b1 is 2 or 3) to obtain a pH-responsive lipid derivative represented by the aforementioned formula (47-i), or performing step (D) to obtain a pH-responsive lipid derivative represented by the aforementioned formula (36-i), or performing step (E), step (F), and step (G) in this order to obtain a pH-responsive lipid derivative represented by the aforementioned formula (46-i).

More specifically, the pH-responsive lipid derivative (i) of the present invention can be produced by obtaining (A) pH-responsive lipid derivative (1) and (B) pH-responsive lipid derivative (1-1), which are preferred embodiments thereof. In the following, therefore, production methods of (A) pH-responsive lipid derivative (1) and (B) pH-responsive lipid derivative (1-1) are described.

### [Production method of (A) pH-responsive lipid derivative (1)]

The pH-responsive lipid derivative represented by the aforementioned formula (1) according to the present invention can be produced, for example, by a polymerization method, coupling method (1), or coupling method (2). The polymerization method is described below.

The following is a representative method for producing a pH-responsive lipid derivative represented by the aforementioned formula (1), and those skilled in the art can also produce a pH-responsive lipid derivative represented by the aforementioned formula (1) by using said production method with appropriate modifications or using other production methods known in the pertinent technique field or using them with appropriate modifications.

While the detail is described later, those skilled in the art can further produce a pH-responsive lipid derivative represented by the aforementioned formula (1-1) according to the present invention in which the terminal amino group has been protected or converted, by appropriately changing the production method and raw materials.

### <Production method of pH-responsive lipid derivative - polymerization method ->

A pH-responsive lipid derivative represented by the structure of the following formula (47) can be produced by a production method in which the following step (A2), step (B1), and step (C1) are performed in this order:

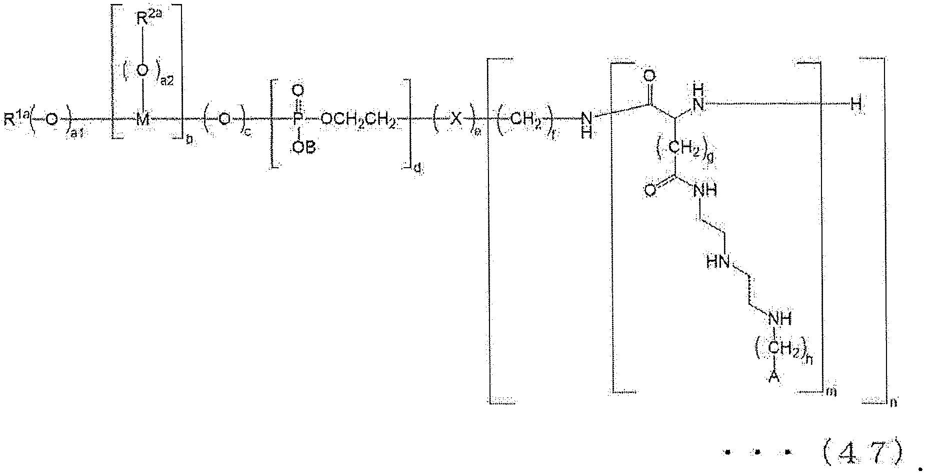

In the formula (1), R^{1a} and R^{2a} are each independently an alkyl group having 8 to 24 carbon atoms, or a sterol residue, M is a trivalent group represented by>N-CH₂-CH₂- or an optionally branched hydrocarbon trivalent group having 3 to 7 carbon atoms, B is a hydrogen atom, an alkali metal, or an ammonium group, X is a linking group containing a bonding group formed by a nucleophilic substitution reaction, a Michael addition reaction, a thiol-ene reaction, or a cyclization addition reaction, A is a carboxy group or a sulfo group, a1, a2, b, c, d, and e are each independently 0 or 1, f is an integer of 0 to 5, h is an integer of 1 to 3, g and n are each independently 1 or 2, and m is 5 to 300.

### [Step (A2)]

Step (A2) is a step of obtaining a polymer represented by the following formula (5) by a ring-opening polymerization using a polymerization initiator represented by the following formula (3) and an α-amino acid-N-carboxylic acid anhydride represented by the following formula (4):

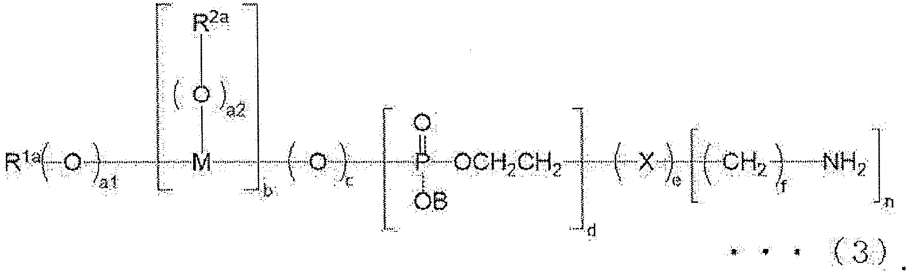

In the formula (3), R^{1a} and R^{2a} are each independently an alkyl group having 8 to 24 carbon atoms, or a sterol residue, the alkyl group may be a straight chain or branched, and may contain an unsaturated bond. Other symbols are as defined above.

In the formula (3), X is a linking group containing a bonding group formed by a nucleophilic substitution reaction, a Michael addition reaction, a thiol-ene reaction, or a cyclization addition reaction.
(when e=1 and n=1)

When e=1 and n=1, X is not particularly limited as long as it is a divalent organic group, and is preferably a linking group represented by the following formula (35):

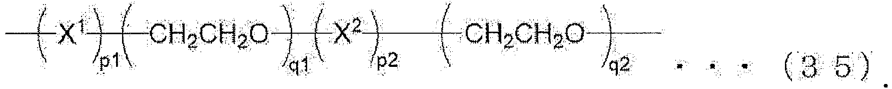

In the formula (35), p1 and p2 are each independently 0 or 1.

In the formula (35), X¹ and X² are each independently a group having 1 to 5 carbon atoms and containing a bonding group resulting from a nucleophilic substitution reaction, which is constituted of a bond formed by a nucleophilic substitution reaction and a spacer.

Examples of the bond that can be formed by a nucleophilic substitution reaction include urethane bond, amide bond, ether bond, divalent hydrocarbon group containing secondary amino group, single bond, and divalent hydrocarbon group. The spacer is not particularly limited as long as it is a site composed of a covalent bond. For example, a structure shown by the aforementioned formula (17) or the aforementioned formula (18) can be mentioned.

In the formula (35), q1 and q2 show the degree of polymerization of the ethyleneglycol structure, are each independently 0 to 50.

### (When e=1 and n=2)

When e=1 and n=2, X is a trivalent organic group and is not particularly limited as long as it is a trivalent. For example, groups represented by the aforementioned formula (28) to aforementioned formula (30) can be mentioned:

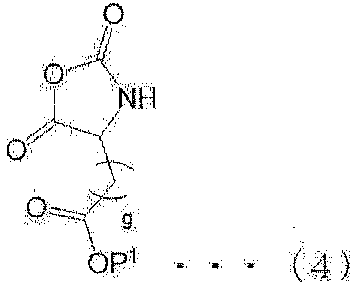

In the formula (4), g=1 or 2. It is preferably an acid anhydride derived from a naturally occurring L-amino acid, from the aspect of use in pharmaceutical applications.

In the formula (4), P¹ is a carboxy-protecting group, preferably a protecting group for a carboxy group having 1 to 8 carbon atoms. It is not particularly limited as long as it is a general carboxy-protecting group, and a methyl group, an ethyl group, a t-butyl group, an allyl group, a benzyl group, and the like can be specifically mentioned. A benzyl group is preferred from the aspects of easy availability and ease of reaction in the subsequent step (B).

As a method for ring-opening polymerization, known methods can be used.

Such methods are disclosed in, for example, Peptide-Basede Materials, 2011, 1-26, Angew. Chem., 2018, 5151-5155, Proceedings of the National Academy of Sciences of the United States of America, 2019, 10658-10663.

Examples of the solvent to be used for the ring-opening polymerization reaction include various organic solvents such as tetrahydrofuran, acetonitrile, N-methyl-2-pyrrolidone, N,N-dimethylformamide, N,N-dimethylacetamide, dichloromethane, chloroform, and the like and mixtures thereof. Preferably, N,N-dimethylformamide, N,N-dimethylacetamide, tetrahydrofuran, dichloromethane, and chloroform can be mentioned.

The total amount of the solvent used in the ring-opening polymerization reaction is 3- to 100-fold amount, preferably 5-to 50-fold amount, most preferably 7- to 25-fold amount, in volume ratio, relative to the α-amino acid-N-carboxylic anhydride represented by the formula (4). When a mixed solvent is used, the ratio is not particularly limited, and various compounds represented by the formula (3) and the formula (4) only need to be dissolved respectively.

The reaction temperature during the ring-opening polymerization reaction is generally 20 to 60°C, preferably 25 to 40°C. The reaction time at this time varies depending on the conditions such as reaction temperature and the like, and is generally preferably 3 hr to 24 hr.

By the above, a polymer represented by the formula (5) is obtained.

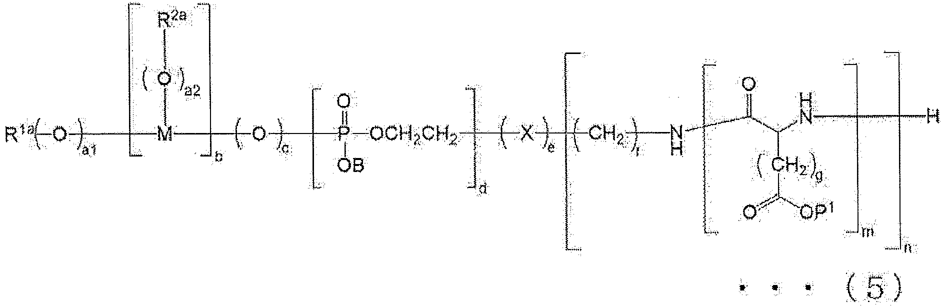

### (in the formula (5), each symbol is as defined above.)

In the formula (5), m can be defined in the same manner as m in the aforementioned formula (1). That is, in the formula (5), m is 5 to 300, the range of m varies depending on the lipid to be bonded and is preferably 5 to 200, most preferably 5 to 150. It is particularly preferably 5 to 110.

The obtained polymer may be used as it is unpurified, or may be isolated and purified by treatments such as reprecipitation, gel filtration chromatography, membrane purification and the like. Furthermore, as described below, a pH-responsive lipid derivative with the terminal amino group protected or converted can be obtained by further protecting or converting the terminal amino group.

### [Step (B1)]

Step (B1) is a step of obtaining a polymer represented by the following formula (7) by addition reaction of a polymer represented by the aforementioned formula (5) obtained in the aforementioned step (A2) with a diethylenetriamine derivative represented by the following formula (6)

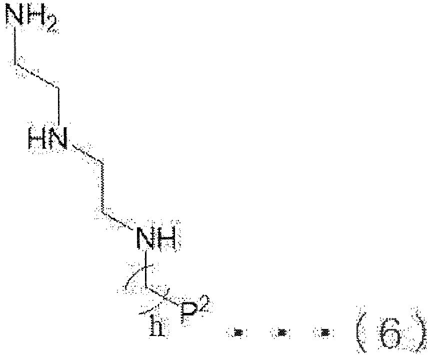

In the formula (6), h is an integer of 1 to 3. From the aspect of synthesis, h=2 or 3 is preferred.

In the formula (6), P² is a protected carboxy group or a protected sulfo group.

The protected carboxy group is not particularly limited as long as it is a derivative used generally. Specifically, a carboxylic acid methyl ester group, a carboxylic acid ethylester group, a carboxylic acid t-butyl ester group, a carboxylic acid allylester group, a carboxylic acid benzylester group, and the like can be mentioned. From the aspect of easy deprotection, a carboxylic acid t-butyl ester group is preferred.

The protected sulfo group is not particularly limited as long as it is a derivative used generally. Specifically, a sulfonic acid methyl ester group, a sulfonic acid ethylester group, a sulfonic acid 2,2,2-trifluoromethylester group, and the like can be mentioned. From the aspect of easy deprotection, a sulfonic acid 2,2,2-trifluoromethylester group is preferred.

Addition reaction of a polymer represented by the formula (5) and a primary amino group of a diethylenetriamine derivative represented by the formula (6) forms an amide bond, whereby a polymer represented by the formula (7) is obtained.

The amount of the diethylenetriamine derivative represented by the formula (6) is generally 2- to 10-fold amount, preferably 3- to 8-fold amount, most preferably 4- to 6-fold amount, in molar ratio, relative to m of the polymer represented by the formula (5).

When the amount is less than 2-fold amount, there is a risk that a structure in which the polymers are crosslinked by the diethylenetriamine derivative may be formed, or the addition rate of the diethylenetriamine derivative to the polymer may decrease. When the amount is more than 10-fold amount, the diethylenetriamine derivative not used for the reaction is wasted.

A catalyst can be used in this reaction and, for example, a catalyst such as 2-hydroxypyridine, pyridine, triethylamine, and the like can be used.

The addition reaction can be performed in various solvents. The solvent is not particularly limited as long as it has no reactivity with the polymer represented by the formula (5) and the diethylenetriamine derivative represented by the formula (6). For example, various organic solvents such as tetrahydrofuran, toluene, acetonitrile, chloroform, N-methyl-2-pyrrolidone, N,N-dimethylformamide, and the like, and mixtures thereof can be mentioned. N-methyl-2-pyrrolidone or tetrahydrofuran is preferred from the aspect of polymer solubility.

The total amount of the solvent used in the addition reaction is 1.5- to 70-fold amount, preferably 1.8- to 50-fold amount, most preferably 2- to 30-fold amount, in volume ratio, relative to the polymer represented by the formula (5).

The reaction temperature during the addition reaction varies depending on the solvent to be used, and is generally 0 to 100°C. The reaction time at this time the conditions such as reaction temperature and the like, and is generally preferably 3 hr to 72 hr.

By the above, a polymer represented by the formula (7) is obtained. 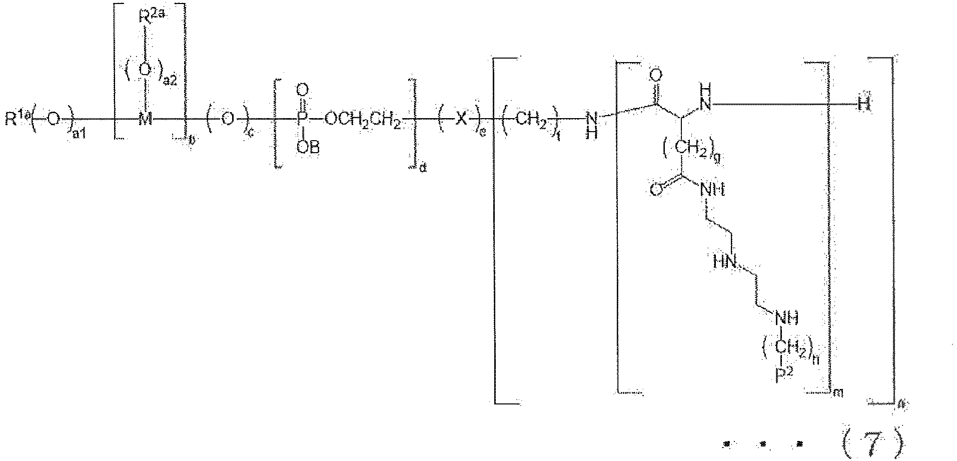 (in the formula (7), each symbol is as defined above).

The obtained polymer may be used as it is unpurified, or may be isolated and purified by treatments such as reprecipitation, gel filtration chromatography, membrane purification and the like.

### [Step (C1)]

Step (C1) is a step of obtaining a pH-responsive lipid derivative represented by the aforementioned formula (47) by deprotecting a polymer represented by the aforementioned formula (7) obtained in the aforementioned step (B1).

As the deprotection method, known methods can be used according to P² of the diethylenetriamine derivative introduced in step (B1). For example, when P² is a carboxylic acid t-butyl ester group, it is deprotected by hydrolysis under acidic conditions to be a carboxy group. Further, when P² is a sulfonic acid 2,2,2-trifluoromethyl ester group, it becomes a sulfo group by hydrolysis under basic conditions.

Specific production methods are shown below, to which the invention is not limited.

### (Specific Example 1)

The formula (7) wherein P² is a carboxylic acid t-butyl ester group is described. In this case, acid hydrolysis proceeds under acidic conditions to form a carboxy group.

The acid used in the acid hydrolysis reaction is not particularly limited as long as it is an acid. Inorganic acids are preferred, hydrochloric acid aqueous solution, nitric acid aqueous solution, and phosphoric acid aqueous solution are more preferred, and hydrochloric acid aqueous solution is most preferred.

The concentration of the acid varies depending on the type of acid. For example, in the case of an aqueous solution of hydrochloric acid, it is generally 1 to 11.2N, preferably 1.5 to 11.2N, and most preferably 2 to 11.2N.

When it is thinner than 1N, a longer time is required to complete the acid hydrolysis reaction.

An acid hydrolysis reaction can be performed in various solvents, with preference given to water-miscible solvents. Examples of the mixed solvent to be used in the acid hydrolysis reaction include water-THF and water-1,4-dioxane.

The total amount of the solvent used in the acid hydrolysis is 3- to 100-fold amount, preferably 4- to 75-fold amount, most preferably 5- to 50-fold amount, in volume ratio, relative to the polymer represented by the formula (7). When a mixed solvent is used, the ratio is not particularly limited, and various compounds represented by the formula (7) are preferably dissolved in an amount of an organic solvent to be used.

The reaction temperature during the acid hydrolysis is generally 20 to 80°C, preferably 25 to 60°C. The reaction time at this time varies depending on the conditions such as reaction temperature and the like, and is generally preferably 3 hr to 48 hr.

As the purification method of the obtained pH-responsive lipid derivative, isolation and purification can be performed by a treatment such as reprecipitation, gel filtration chromatography, membrane purification, or the like.

### <Production method of pH-responsive lipid derivative - coupling method (1) ->

A pH-responsive lipid derivative represented by the aforementioned formula (1) can also be synthesized by covalently bonding a lipid derivative and a pH-responsive polymer.

The pH-responsive polymer of the present invention represented by the formula (1) is as described above. As a specific example, the following formula (36) in which a lipid derivative and a pH-responsive polymer are covalently bonded at a molar ratio of 1:1 is explained here.

A pH-responsive lipid derivative represented by a structure of the following formula (36) can be produced via the production method shown in step (D1).

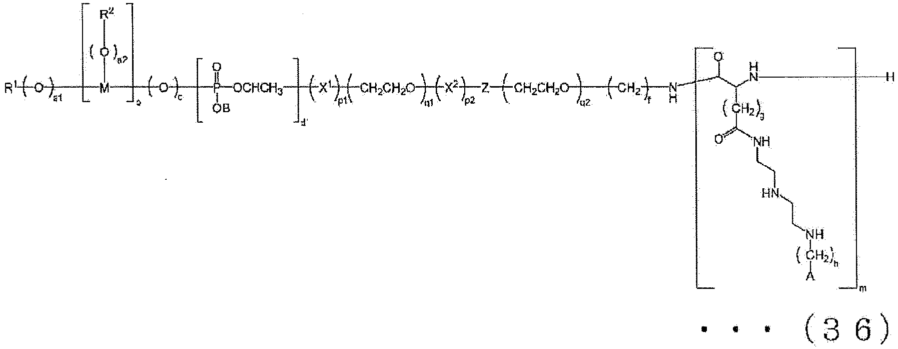

In the formula (36), R¹ and R² are each independently an alkyl group having 8 to 24 carbon atoms, or an acyl group having 8 to 24 carbon atoms, or a sterol residue, M is a trivalent group represented by>N-CH₂-CH₂- or an optionally branched hydrocarbon trivalent group having 3 to 7 carbon atoms, B is a hydrogen atom, an alkali metal, or an ammonium group, A is a carboxy group or a sulfo group, a1, a2, b, c, and d are each independently 0 or 1, f is an integer of 0 to 5, h is an integer of 1 to 3, g is 1 or 2, m is 5 to 300, X¹ and X² are each independently a group having 1 to 5 carbon atoms and containing a bonding group resulting from a nucleophilic substitution reaction, Z is a group formed by a Michael addition reaction, a thiol-ene reaction, or a cyclization addition reaction, p1 and p2 are each independently 0 or 1, and q1 and q2 are each independently 0 to 50.

### [Step (D1)]

Step (D1) is a step of obtaining a pH-responsive lipid derivative represented by the aforementioned formula (36) by reacting a functional group-containing lipid represented by the following formula (8) with a functional group-containing pH-responsive polymer represented by the following formula (9)

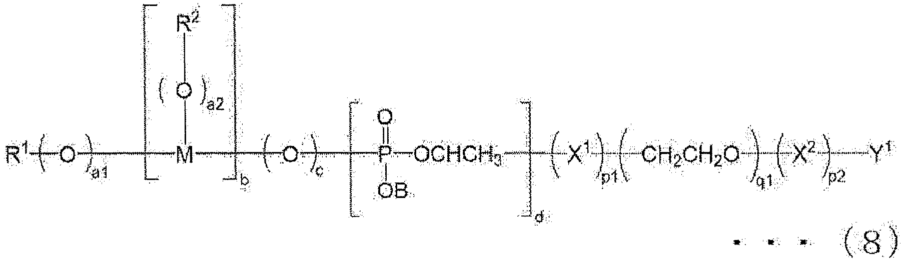

In the formula (8), p1 and p2 are each independently 0 or 1.

In the formula (8), X¹ and X² are each independently a group having 1 to 5 carbon atoms and containing a bonding group resulting from a nucleophilic substitution reaction, which is constituted of a bond formed by a nucleophilic substitution reaction and a spacer.

Examples of the bond that can be formed by a nucleophilic substitution reaction include ester bond, urethane bond, amide bond, ether bond, carbonate bond, divalent hydrocarbon group containing secondary amino group, single bond, and divalent hydrocarbon group. The spacer is not particularly limited as long as it is a site composed of a covalent bond. For example, a structure shown by the aforementioned formula (17) or the formula (18) can be mentioned.

In the formula (8), Y¹ is an atomic group containing a functional group capable of forming a covalent bond with a functional group contained in the following Y².

The aforementioned atomic group Y¹ is an atomic group contained in the lipid represented by the aforementioned formula (8), and includes a functional group (Y¹') obtained by forming a covalent bond with a functional group contained in Y² below. Y¹ may be only the aforementioned functional group (Y¹') or may consist of the binding site (W) between the aforementioned functional group (Y¹') and the aforementioned lipid, and is represented by the following formula:

Y¹-=Y¹'-W-.

In the aforementioned formula, Y¹' is a functional group capable of forming a covalent bond with a functional group contained in the following Y², and W is a bonding site to lipid or a single bond.

The bonding site W is not particularly limited as long as it is a linker responsible for bonding to a lipid and is a site composed of a covalent bond.

The bonding site W is preferably an ester bond, a urethane bond, an amide bond, an ether bond, a carbonate bond, a divalent hydrocarbon group containing secondary amino group, a single bond, a divalent hydrocarbon group, and a divalent oxyalkylene group and a repeat structure thereof. The aforementioned hydrocarbon group preferably has not more than 12 carbon atoms, and methylene group, ethylene group, trimethylene group, propylene group, isopropylene group, tetramethylene group, butylene group, isobutylene group, pentamethylene group, hexamethylene group, and the like can be mentioned. The aforementioned oxyalkylene group is, for example, an oxyethylene group or an oxypropylene group. The repeat unit of the aforementioned oxylene alkylene skeleton is preferably not more than 12.

In the formula (8), other symbols are as defined above.

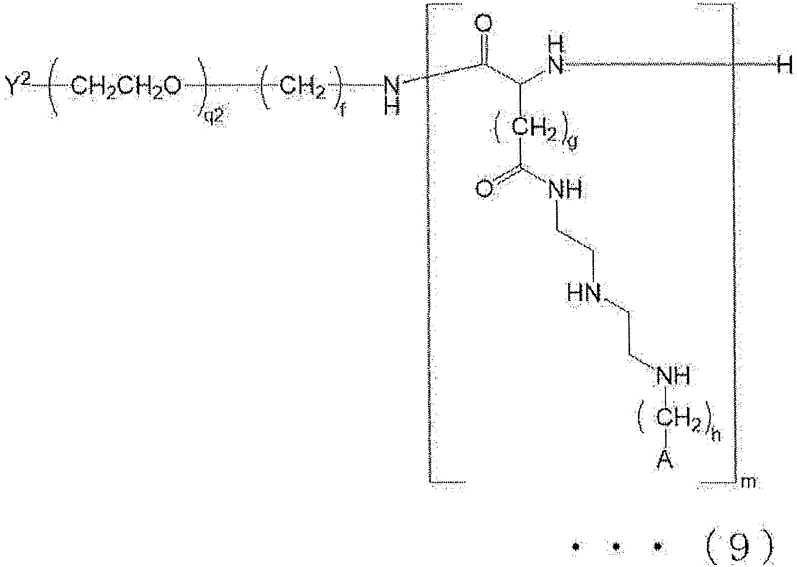

In the aforementioned formula (9), Y² is an atomic group containing a functional group that can form a covalent bond with the functional group contained in the aforementioned Y¹, and the functional group contained in the atomic group Y¹ in the aforementioned formula (8) and the functional group contained in the atomic group Y² are different from each other.

The aforementioned atomic group Y² is an atomic group contained in the functional group-containing pH-responsive polymer represented by the aforementioned formula (9), and includes a functional group (Y²') that can form a covalent bond with the functional group contained in the aforementioned Y¹. Y² may be the aforementioned functional group (Y²') alone or may consist of a bonding site (W') between the aforementioned functional group (Y²') and a pH-responsive polymer, and is represented by the following formula:

Y²-=Y²'-W'-.

In the aforementioned formula, Y²' is a functional group that can form a covalent bond with the functional group contained in the aforementioned Y¹, and W' is a bonding site to a pH-responsive polymer or a single bond.

The bonding site W' in the formula (9) is not particularly limited as long as it is a linker responsible for bonding to the pH-responsive polymer, and is a site composed of a covalent bond.

The bonding site W' is preferably a urethane bond, an amide bond, an ether bond, a divalent hydrocarbon group containing secondary amino group, a single bond, a divalent hydrocarbon group, and a divalent oxyalkylene group and a repeat structure thereof. The aforementioned hydrocarbon group preferably has not more than 12 carbon atoms, and methylene group, ethylene group, trimethylene group, propylene group, isopropylene group, tetramethylene group, butylene group, isobutylene group, pentamethylene group, hexamethylene group, and the like can be mentioned. The aforementioned oxyalkylene group is, for example, an oxyethylene group or an oxypropylene group. The repeat unit of the aforementioned oxylene alkylene skeleton is preferably not more than 12.

The functional group Y¹' in the aforementioned formula (8) and Y²' in the aforementioned formula (9) are different functional groups, and are not particularly limited as long as they are functional groups that can react with each other to form a covalent bond. By reacting functional group Y¹' and functional group Y²', a pH-responsive lipid derivative represented by the aforementioned formula (36) is obtained.

Preferred functional groups suitable as the functional group Y¹' include the following formulas (37) to (45). 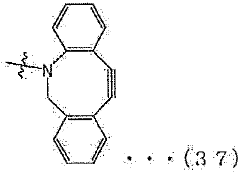 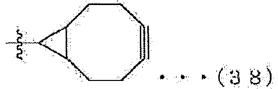 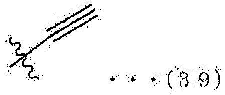 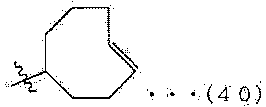 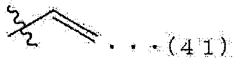 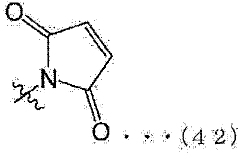 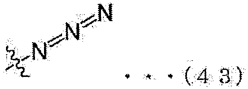 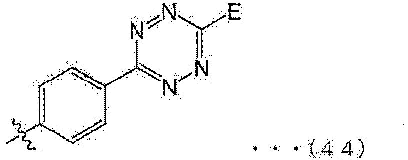 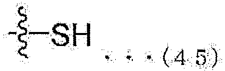 (in the above-mentioned formula (44), E is a hydrogen atom or a methyl group.)

As the functional group-containing lipid represented by the aforementioned formula (8) having an atomic group Y¹ containing the functional groups Y¹' represented by the aforementioned formulas (37) to (45), commercially available products may be used or they may also be separately synthesized and used.

The functional group-containing pH-responsive polymer represented by the aforementioned formula (9) having the atomic group Y² containing the functional group Y²' in the aforementioned formula (9) can be synthesized and used based on previous reports. Such method is disclosed in, for example, WO2018/110366.

As the reaction conditions for covalently bonding the functional group Y¹' and the functional group Y²', known methods can be used. By covalent bonding, Z is formed and a pH-responsive lipid derivative represented by the aforementioned formula (36) is obtained.

Here, preferable examples of the functional group Y²' include the formulas (37) to (41) and (43) to (45), among the functional groups exemplified for the functional group Y¹'.

In one embodiment, when the functional group Y¹' is an internal alkyne compound represented by the aforementioned formula (37), it reacts with the aforementioned formula (43) as the functional group Y²' to form Z represented by the aforementioned formula (19).

In another embodiment, when the functional group Y¹' is an internal alkyne compound represented by the aforementioned formula (38), it reacts with the aforementioned formula (43) as the functional group Y²' to form Z represented by the aforementioned formula (20).

In another embodiment, when the functional group Y¹' is an alkyne compound represented by the aforementioned formula (39), it reacts with the aforementioned formula (43) as the functional group Y²' to form Z represented by the aforementioned formula (21).

In another embodiment, when the functional group Y¹' is a trans cyclooctyne compound represented by the aforementioned formula (40), it reacts with tetrazine group and the like as the functional group Y²' to form Z represented by the aforementioned formula (22).

In another embodiment, when the functional group Y¹' is an alkene compound represented by the aforementioned formula (41), it reacts with the aforementioned formula (45) as the functional group Y²' to form Z represented by the aforementioned formula (23).

In another embodiment, when the functional group Y¹' is a maleimide compound represented by the aforementioned formula (42), it reacts with the aforementioned formula (45) as the functional group Y²' to form Z represented by the aforementioned formula (24).

In another embodiment, when the functional group Y¹' is the aforementioned formula (43), it reacts with the aforementioned formula (39) as the functional group Y²' to form Z represented by the aforementioned formula (25).

In another embodiment, when the functional group Y¹' is a tetrazine compound represented by the aforementioned formula (44), it reacts with the aforementioned formula (39) as the functional group Y²' to form Z represented by the aforementioned formula (26).

In another embodiment, when the functional group Y¹' is the aforementioned formula (45), it reacts with the aforementioned formula (41) as the functional group Y²' to form Z represented by the aforementioned formula (27).

As the purification method of the obtained pH-responsive lipid derivative, isolation and purification can be performed by a treatment such as reprecipitation, gel filtration chromatography, membrane purification, or the like.

In the formula (9), other symbols are as defined above.

Specific production methods are shown below, to which the invention is not limited.

### (Specific Example 2)

First, the case where Y¹' in the aforementioned formula (8) is a functional group represented by the aforementioned formula (37), and Y²' in the aforementioned formula (9) is a functional group represented by the aforementioned formula (43) is described.

In this case, the functional group represented by formula (37) and the functional group represented by formula (43) react by a cyclization addition reaction. As a result, a pH-responsive lipid derivative in which Z in the aforementioned formula (36) is the aforementioned formula (19) is obtained.

The amount of the functional group-containing lipid represented by the formula (8) used in the cyclization addition reaction is generally 0.8- to 3.0-fold amount, preferably 1.0-to 2.5-fold amount, most preferably 1.2- to 2.0-fold amount, in molar ratio, relative to a functional group-containing pH-responsive polymer represented by the formula (9).

When the amount is less than 0.8-fold amount, the proportion of the pH-responsive polymer in the resultant product increases, making purification difficult. When the amount is more than 3.0-fold amount, the proportion of the functional group-containing lipid in the resultant product increases, making purification difficult, and the functional group-containing lipid not used for the reaction is wasted.

The cyclization addition reaction can be performed in various solvents. The solvent to be used in the cyclization addition reaction may be any solvent as long as it can dissolve the functional group-containing lipid represented by the formula (8) and the functional group-containing pH-responsive polymer represented by the formula (9).

The solvent may be capable of dissolving the functional group-containing lipid represented by the formula (8) and the functional group-containing pH-responsive polymer represented by the formula (9), or a mixture of two or more solvents capable of dissolving each of them may be used.

Examples of the solvent used in the cyclization addition reaction include various organic solvents such as water, buffer, methanol, toluene, THF, dichloromethane, chloroform, and the like and mixtures thereof.

Examples of the mixed solvent used in the cyclization addition reaction include combinations of water-toluene, water-THF, water-dichloromethane, water-chloroform, and the like. The cyclization addition reaction may be a uniform solvent system or a two-layer separated solvent system. Generally, from the aspects that the functional group-containing lipid represented by the formula (8) is easily dissolved in an organic solvent and the functional group-containing pH-responsive polymer represented by the formula (9) is easily dissolved in water, a water-dichloromethane mixed solvent is preferred.

The total amount of the solvent used in the cyclization addition reaction is 50- to 150-fold amount, preferably 65- to 135-fold amount, most preferably 80- to 120-fold amount, in volume ratio, relative to a functional group-containing pH-responsive polymer represented by the formula (8). When a mixed solvent is used, the ratio is not particularly limited, and various compounds represented by the formula (8) and the formula (9) only need to be dissolved respectively.

The reaction temperature during the cyclization addition reaction is generally 20 to 60°C, preferably 25 to 45°C. The reaction time at this time varies depending on the conditions such as reaction temperature and the like, and is generally preferably 3 hr to 72 hr.

By the above, a pH-responsive lipid derivative wherein Z is the formula (19) is obtained. As the purification method of the obtained pH-responsive lipid derivative, isolation and purification can be performed by a treatment such as reprecipitation, gel filtration chromatography, membrane purification, or the like.

### (Specific Example 3)

Successively, the case where Y¹' in the aforementioned formula (8) is a functional group represented by the aforementioned formula (41), and Y²' in the aforementioned formula (9) is a functional group represented by the aforementioned formula (45) is described.

In this case, the functional group represented by formula (41) and the functional group represented by formula (45) react by a thiol-ene reaction. As a result, a pH-responsive lipid derivative in which Z in the aforementioned formula (36) is the aforementioned formula (23) is obtained.

The amount of the functional group-containing lipid represented by the formula (8) used in the thiol-ene reaction is generally 0.8- to 3.0-fold amount, preferably 1.0- to 2.5-fold amount, most preferably 1.2- to 2.0-fold amount, in molar ratio, relative to a functional group-containing pH-responsive polymer represented by the formula (9).

When the amount is less than 0.8-fold amount, the proportion of the pH-responsive polymer in the resultant product increases, making purification difficult. When the amount is more than 3.0-fold amount, the proportion of the functional group-containing lipid in the resultant product increases, making purification difficult, and the functional group-containing lipid not used for the reaction is wasted.

The thiol-ene reaction can be performed in various solvents. The solvent to be used in the thiol-ene reaction may be any solvent as long as it can dissolve the functional group-containing lipid represented by the formula (8) and the functional group-containing pH-responsive polymer represented by the formula (9).

The solvent may be capable of dissolving the functional group-containing lipid represented by the formula (8) and the functional group-containing pH-responsive polymer represented by the formula (9), or a mixture of two or more solvents capable of dissolving each of them may be used.

Examples of the solvent used in the thiol-ene reaction include various organic solvents such as water, buffer, methanol, toluene, THF, dichloromethane, chloroform, and the like and mixtures thereof.

Examples of the mixed solvent used in the thiol-ene reaction include combinations of water-toluene, water-THF, water-dichloromethane, water-chloroform, and the like. The cyclization addition reaction may be a uniform solvent system or a two-layer separated solvent system. Generally, from the aspects that the functional group-containing lipid represented by the formula (8) is easily dissolved in an organic solvent and the functional group-containing pH-responsive polymer represented by the formula (9) is easily dissolved in water, a water-dichloromethane mixed solvent is preferred.

The total amount of the solvent used in the thiol-ene reaction is 50- to 150-fold amount, preferably 65- to 135-fold amount, most preferably 80- to 120-fold amount, in volume ratio, relative to a pH-responsive polymer represented by the formula (9). When a mixed solvent is used, the ratio is not particularly limited, and various compounds represented by the formula (8) and the formula (9) only need to be dissolved respectively.

A thermal radical generator or a photoradical generator is used during the thiol-ene reaction between the functional group-containing lipid represented by the formula (8) and the functional group-containing pH-responsive polymer represented by the formula (9). As the thermal radical generator, a peroxide or an azo compound is preferred.

Examples of the peroxide include tert-butyl hydroperoxide, dibenzoyl peroxide, ammonium persulfate, hydrogen peroxide, tert-butyl-2-ethylhequinoate, and dilauroyl peroxide, and tert-butyl hydroperoxide, ammonium persulfate, and hydrogen peroxide are preferred.

Examples of the azo compound include azobis(isobutyronitrile), 2,2'-azobis(2-methylbutanenitrile), 2,2'-azobis(2-amidinopropane)hydrochloride, 4'4-azobis(4-cyanopentanoic acid), 2'2-azobis[2-methyl-N-(2-hydroxyethyl)propionamide], 2'2-azobis[N-(2-hydroxyethyl)-2-methylpropionamidine] 4 hydrate, 2'2-azobis(2-methylpropionamidine) dihydrochloride, 2'2-azobis[2-(2-imidazolin-2-yl)propane], and 2'2-azobis[2-(2-imidazolin-2-yl)propane] dihydrochloride.

Examples of the photoradical generator include lithium phenyl(2,4,6-trimethylbenzoyl)phosphinate, diphenyl(2,4,6-trimethylbenzoyl)phosphine oxide, 2-hydroxy-4'-(2-hydroxyethoxy)-2-methylpropiophenone, FOM-03011 (manufactured by FUJIFILM Wako Pure Chemical Corporation), CyracureUVI-6970, supra UVI-6974, supra UV-6990 (all manufactured by Union Carbide Corporation, USA), Irgacure264 (manufactured by BASF), and CIT-1682 (manufactured by Nippon Soda Co., Ltd.).

The amount of the thermal radical generator or photoradical generator used in the thiol-ene reaction is generally 0.05- to 10-fold amount, preferably 0.10- to 8-fold amount, most preferably 0.15- to 5-fold amount, in molar ratio, relative to a functional group-containing pH-responsive polymer represented by the formula (9).

When a thermal radical generator is used, the reaction temperature during the thiol-ene reaction between the functional group-containing lipid represented by the formula (8) and the functional group-containing pH-responsive polymer represented by the formula (9) is preferably the 10-hour half-life temperature of the thermal radical generator used ±20°C. The reaction time at this time varies depending on the conditions such as reaction temperature and the like, and is generally preferably 3 to 72 hr.

When a photoradical generator is used, the reaction temperature during the thiol-ene reaction between the functional group-containing lipid represented by the formula (8) and the functional group-containing pH-responsive polymer represented by the formula (9) is generally 0 to 60°C, and radicals are generated using a separate device that emits ultraviolet light, such as a high-pressure mercury lamp, an LED laser, or the like, depending on the absorption wavelength of the photoradical generator used. The ultraviolet radiation time varies depending on the type of photoradical generator and the conditions of the device that emits ultraviolet rays, and is preferably not more than 3 hours.

By the above, a pH-responsive lipid derivative wherein Z is the formula (23) is obtained. As the purification method of the obtained pH-responsive lipid derivative, isolation and purification can be performed by a treatment such as reprecipitation, gel filtration chromatography, membrane purification, or the like.

A pH-responsive lipid derivative having two pH-responsive polymers can be obtained by reacting a lipid having two atomic groups Y¹ with a pH-responsive polymer having one atomic group Y².

In addition, as described later, a pH-responsive lipid derivative with a protected or converted terminal group can be obtained by reacting a pH-responsive polymer with a protected or converted terminal amino group in place of the aforementioned formula (9) having a terminal amino group.

### <Production method of pH-responsive lipid derivative - coupling method (2) ->

A pH-responsive lipid derivative represented by the aforementioned formula (1) can also be synthesized by synthesizing a pH-responsive lipid derivative precursor by covalent bonding a lipid derivative and a pH-responsive polymer precursor, and then inducing the pH-responsive lipid derivative.

The pH-responsive lipid derivative of the present invention represented by the formula (1) is as described above. As a specific example, the following formula (46) in which a lipid derivative and a pH-responsive polymer precursor are covalently bonded at a molar ratio of 1:1 is explained here.

A pH-responsive lipid derivative represented by the structure of the following formula (46) can be produced by a production method in which the following step (E1), step (F1), and step (G1) are performed in this order:

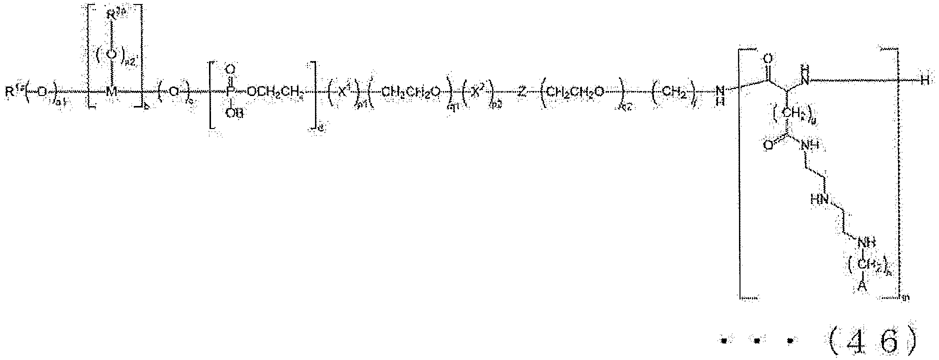

In the formula (46), R^{1a} and R^{2a} are each independently an alkyl group having 8 to 24 carbon atoms, or a sterol residue, M is a trivalent group represented by>N-CH₂-CH₂- or an optionally branched hydrocarbon trivalent group having 3 to 7 carbon atoms, B is a hydrogen atom, an alkali metal, or an ammonium group, A is a carboxy group or a sulfo group, a1, a2, b, c, and d are each independently 0 or 1, f is an integer of 0 to 5, h is an integer of 1 to 3, g is 1 or 2, m is 5 to 300, X¹ and X² are each independently a group having 1 to 5 carbon atoms and containing a bonding group resulting from a nucleophilic substitution reaction, Z is a group formed by a Michael addition reaction, a thiol-ene reaction, or a cyclization addition reaction, p1 and p2 are each independently 0 or 1, and q1 and q2 are each independently 0 to 50.

Similarly, as described later, a pH-responsive lipid derivative represented by the above-mentioned formula (46) wherein the terminal amino group is protected or converted can be obtained by performing the following step (E1), step (F1), and step (G1) in this order using a pH-responsive polymer precursor with a protected or converted terminal amino group in place of a pH-responsive polymer precursor having a terminal amino group.

### [Step (E1)]

Step (E1) is a step of obtaining a pH-responsive lipid derivative precursor represented by the following formula (12) by reacting a functional group-containing lipid represented by the following formula (10) with a polymer represented by the following formula (11)

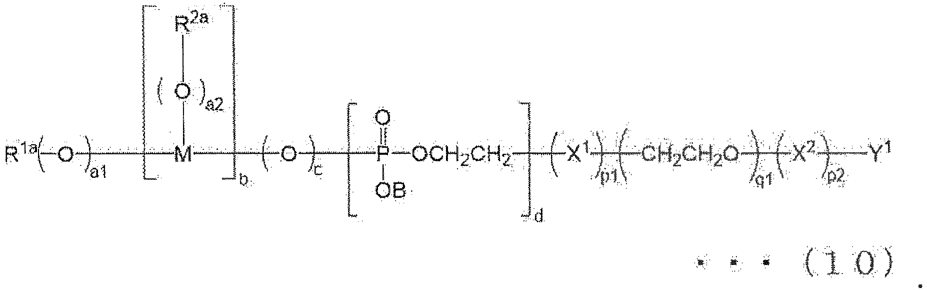

In the formula (10), R^{1a} and R^{2a} are each independently an alkyl group having 8 to 24 carbon atoms, or a sterol residue, the alkyl group may be a straight chain or branched, and may contain an unsaturated bond. Other symbols are as defined above.

X¹ and X² are each independently a group having 1 to 5 carbon atoms and containing a bonding group resulting from a nucleophilic substitution reaction, which is constituted of a bond formed by a nucleophilic substitution reaction and a spacer.

Examples of the bond that can be formed by a nucleophilic substitution reaction include urethane bond, amide bond, ether bond, divalent hydrocarbon group containing secondary amino group, single bond, and divalent hydrocarbon group. The spacer is not particularly limited as long as it is a site composed of a covalent bond. For example, a structure shown by the aforementioned formula (17) or the aforementioned formula (18) can be mentioned.

In the formula (10), Y¹ is an atomic group containing a functional group capable of forming a covalent bond with a functional group contained in the following Y².

The aforementioned atomic group Y¹ is an atomic group contained in the lipid represented by the aforementioned formula (10), and includes a functional group (Y¹') obtained by forming a covalent bond with a functional group contained in Y² below. Y¹ may be only the aforementioned functional group (Y¹') or may consist of the binding site (W) between the aforementioned functional group (Y¹') and the aforementioned lipid, and is represented by the following formula:

Y¹-=Y¹'-W-.

In the aforementioned formula, Y¹' is a functional group capable of forming a covalent bond with a functional group contained in the following Y², and W is a bonding site to lipid or a single bond.

The bonding site W is not particularly limited as long as it is a linker responsible for bonding to a lipid and is a site composed of a covalent bond.

The bonding site W is preferably a urethane bond, an amide bond, an ether bond, a divalent hydrocarbon group containing secondary amino group, a single bond, a divalent hydrocarbon group, and a divalent oxyalkylene group and a repeat structure thereof. The aforementioned hydrocarbon group preferably has not more than 12 carbon atoms, and methylene group, ethylene group, trimethylene group, propylene group, isopropylene group, tetramethylene group, butylene group, isobutylene group, pentamethylene group, hexamethylene group, and the like can be mentioned. The aforementioned oxyalkylene group is, for example, an oxyethylene group or an oxypropylene group. The repeat unit of the aforementioned oxylene alkylene skeleton is preferably not more than 12.

As a functional group appropriate as functional group Y¹', the aforementioned formula (37) to the aforementioned formula (45) can be preferably mentioned.

Each other symbols are as defined above.

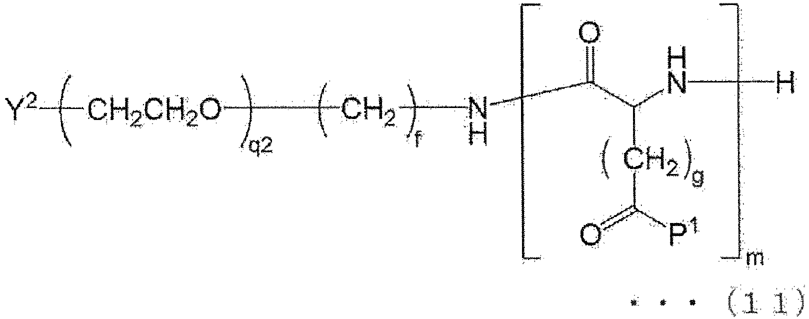

In the formula (11), P¹ is a protecting group for a carboxy group having 1 to 8 carbon atoms. It is not particularly limited as long as it is a general carboxy-protecting group, and a methyl group, an ethyl group, a t-butyl group, an allyl group, a benzyl group, and the like can be specifically mentioned. A benzyl group is preferred from the aspects of easy availability and ease of reaction in the subsequent step (F).

In the aforementioned formula (11), Y² is an atomic group containing a functional group that can form a covalent bond with the functional group contained in the aforementioned Y¹, and the functional group contained in the atomic group Y¹ in the aforementioned formula (10) and the functional group contained in the atomic group Y² are different from each other.

The aforementioned atomic group Y² is an atomic group contained in the polymer represented by the aforementioned formula (11), and includes a functional group (Y²') that can form a covalent bond with the functional group contained in the aforementioned Y¹. Y² may be the aforementioned functional group (Y²') alone or may consist of a bonding site (W') between the aforementioned functional group (Y²') and a pH-responsive polymer, and is represented by the following formula:

Y²-=Y²'-W'-.

In the aforementioned formula, Y²' is a functional group that can form a covalent bond with the functional group contained in the aforementioned Y¹, and W' is a bonding site to a pH-responsive polymer precursor or a single bond.

The bonding site W' in the formula (11) is not particularly limited as long as it is a linker responsible for bonding to the pH-responsive polymer precursor, and is preferably similar to the bonding site contained in Y² in the aforementioned formula (9).

Each other symbols are as defined above in the aforementioned formula (46).

The functional group Y¹' in the aforementioned formula (10) and Y²' in the aforementioned formula (11) are different functional groups, and are not particularly limited as long as they are functional groups that can react with each other to form a covalent bond. As the reaction conditions for covalently bonding the functional group Y¹' and the functional group Y²', known methods can be used. By covalent bonding, Z is formed and a pH-responsive lipid derivative precursor represented by the following formula (12) is obtained. 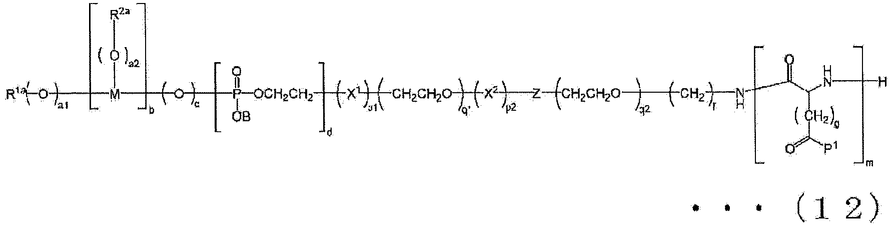 (in the formula (12), each symbol is as defined above.)

The obtained pH-responsive lipid derivative precursor may be used as it is unpurified, or may be isolated and purified by treatments such as reprecipitation, gel filtration chromatography, membrane purification and the like.

Specific production methods are shown below, to which the invention is not limited.

### (Specific Example 4)

The case where Y¹' in the aforementioned formula (10) is a functional group represented by the aforementioned formula (37), and Y²' in the aforementioned formula (11) is a functional group represented by the aforementioned formula (43) is described.

In this case, the functional group represented by formula (37) and the functional group represented by formula (43) react by a cyclization addition reaction. As a result, a polymer in which Z in the aforementioned formula (12) is the aforementioned formula (19) is obtained.

Regarding the amount of a functional group-containing lipid used in the cyclization addition reaction, the amount of solvent, and the reaction temperature, the same conditions as described above (Specific Example 2) can be used.

The cyclization addition reaction can be performed in various solvents. The solvent to be used in the cyclization addition reaction may be any solvent as long as it can dissolve the functional group-containing lipid represented by the formula (10) and the polymer represented by the formula (11).

The solvent may be capable of dissolving the functional group-containing lipid represented by the formula (10) and the polymer represented by the formula (11), or a mixture of two or more solvents capable of dissolving each of them may be used.

Examples of the solvent to be used in the cyclization addition reaction include various organic solvents such as THF, toluene, N-methyl-2-pyrrolidone, N,N-dimethylformamide, dichloromethane, chloroform, and the like, and mixture thereof.

By the above, a pH-responsive lipid derivative precursor wherein Z is the formula (19) is obtained. The obtained pH-responsive lipid derivative precursor may be used as it is unpurified, or may be isolated and purified by treatments such as reprecipitation, gel filtration chromatography, membrane purification and the like.

### [Step (F1)]

Step (F1) is a step of obtaining a polymer represented by the following formula (13) by addition reaction of a polymer represented by the aforementioned formula (12) obtained in the aforementioned step (E) with a diethylenetriamine derivative represented by the aforementioned formula (6).

Addition reaction of a pH-responsive lipid derivative precursor represented by the aforementioned formula (12) and a primary amino group of a diethylenetriamine derivative represented by the aforementioned formula (6) forms an amide bond, whereby a polymer represented by the following formula (13) is obtained.

The amount of the diethylenetriamine derivative represented by the formula (6) is generally 2- to 10-fold amount, preferably 3- to 8-fold amount, most preferably 4- to 6-fold amount, in molar ratio, relative to m of a polymer represented by the aforementioned formula (12).

When the amount is less than 2-fold amount, there is a risk that a structure in which the polymers are crosslinked by the diethylenetriamine derivative may be formed, or the addition rate of the diethylenetriamine derivative to the polymer may decrease. When the amount is more than 10-fold amount, the diethylenetriamine derivative not used for the reaction is wasted.

A catalyst can be used in this reaction and, for example, a catalyst such as 2-hydroxypyridine, pyridine, triethylamine, and the like can be used.

The addition reaction can be performed in various solvents. The solvent is not particularly limited as long as it has no reactivity with the polymer represented by the aforementioned formula (12) and the diethylenetriamine derivative represented by the formula (6). For example, various organic solvents such as tetrahydrofuran, toluene, acetonitrile, chloroform, N-methyl-2-pyrrolidone, N,N-dimethylformamide, and the like, and mixtures thereof can be mentioned. N-methyl-2-pyrrolidone or tetrahydrofuran is preferred from the aspect of polymer solubility.

The total amount of the solvent used in the addition reaction is 1.5- to 70-fold amount, preferably 1.8- to 50-fold amount, most preferably 2- to 30-fold amount, in volume ratio, relative to the polymer represented by the aforementioned formula (12).

The reaction temperature during the addition reaction varies depending on the solvent to be used, and is generally 0 to 100°C. The reaction time at this time varies depending on the conditions such as reaction temperature and the like, and is generally preferably 3 hr to 72 hr.

By the above, a polymer represented by the formula (13) is obtained. 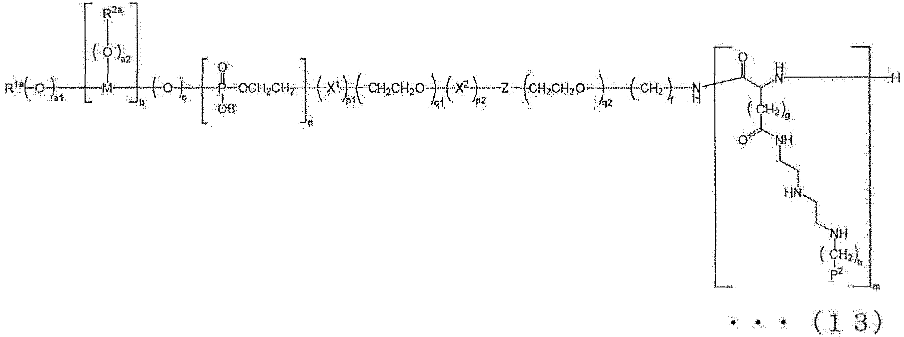 (in the formula (13), each symbol is as defined above.)

The obtained polymer may be used as it is unpurified, or may be isolated and purified by treatments such as reprecipitation, gel filtration chromatography, membrane purification and the like.

### [step (G1)]

Step (G1) is a step of obtaining a pH-responsive lipid derivative represented by the aforementioned formula (46) by deprotecting a polymer represented by the aforementioned formula (13) obtained in the aforementioned step (F1).

As the deprotection method, known methods can be used according to P² of the diethylenetriamine derivative introduced in step (F1). For example, when P² in the formula (13) is a carboxylic acid t-butyl ester group, the deprotection can be performed by a method similar to that in the aforementioned step (C1) except that the formula (13) is used in place of the polymer represented by the aforementioned formula (7).

A pH-responsive lipid derivative having two pH-responsive polymers can be obtained by reacting a lipid having two atomic groups Y¹ with a pH-responsive polymer precursor having one atomic group Y², and passing through the aforementioned steps (E1) to (G1).

### [Production method of (B) pH-responsive lipid derivative (1-1)]

The pH-responsive lipid derivative represented by the aforementioned formula (1-1) according to the present invention can be produced, for example, by a polymerization method, coupling method (1), or coupling method (2).

The following is a representative method for producing a pH-responsive lipid derivative represented by the aforementioned formula (1-1), and those skilled in the art can also produce a pH-responsive lipid derivative represented by the aforementioned formula (1-1) by using said production method with appropriate modifications or using other production methods known in the pertinent technique field or using them with appropriate modifications.

### <Production method of pH-responsive lipid derivative - polymerization method ->

A pH-responsive lipid derivative represented by a structure of the following formula (47-1) can be produced by performing the following step (A3), step (B2), and step (C2) in this order.

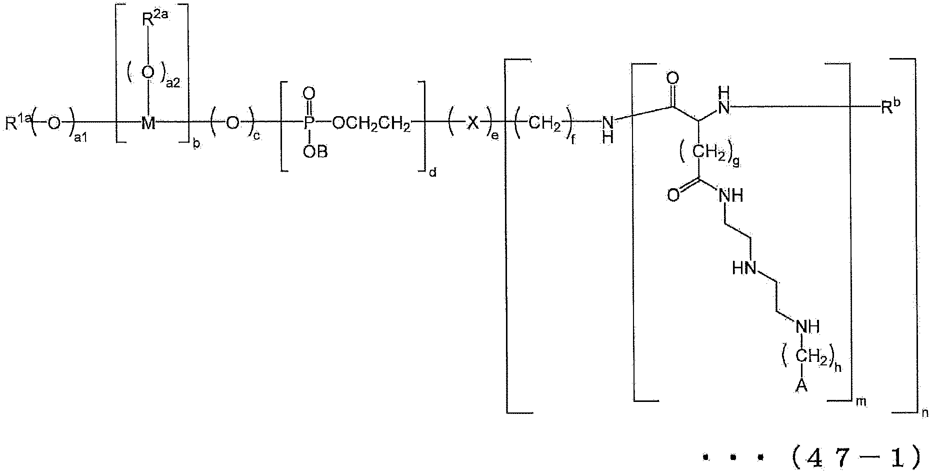

In the formula (47-1), R^{b} is -COCH₃ or -CO-(CH₂)_{b1}-COOH (wherein b1 is 2 or 3), and each other symbol is as defined in the aforementioned formula (47).

### [Step (A3)]

Step (A3) includes
(i) obtaining a polymer represented by the aforementioned formula (5) by a ring-opening polymerization using a polymerization initiator represented by the aforementioned formula (3) and an α-amino acid-N-carboxylic acid anhydride represented by the aforementioned formula (4) (step (i)),
(ii) further introducing COCH₃ or CO-(CH₂)_{b1}-COOH (wherein b1 is 2 or 3) into a terminal amino group of the obtained polymer to obtain a polymer represented by the aforementioned formula (5-1) (step (ii)).

### Step (i)

Those of ordinary skill in the art can perform step (i) by appropriately determining reaction conditions and the like by reference to the explanation of step (A2) described in detail above for the production of the pH-responsive lipid derivative (1).

### Step (ii)

Those of ordinary skill in the art can perform step (ii) by appropriately determining reaction conditions and the like by reference to specific descriptions in the below-mentioned Examples, polymers and methods known in the pertinent technique field, and technical common knowledge.

Specifically, polymer (5-1) can be synthesized by protecting or converting the terminal amino group of the polymer represented by the aforementioned formula (5) (also referred to as polymer (5)). Known reactions can be used to protect or convert the terminal amino group.

For example, a polymer (5-1) in which R^{b} is -CO-CH₃ can be obtained by reacting acetic anhydride as an electrophile with the terminal amino group of the polymer (5).

Also, for example, a polymer (5-1) in which R^{b} is -CO-(CH₂)₂-COOH can be obtained by reacting succinic anhydride as an electrophile with the terminal amino group of the polymer (5) .

The amount of the electrophile to be used is generally 2-to 10-fold amount, preferably 3- to 8-fold amount, most preferably 4- to 6-fold amount, in molar ratio, relative to the terminal amino group of the polymer (5).

In addition, a catalyst can be used in this reaction and, for example, catalysts such as DMAP and the like can be used.

The reaction can be performed in various solvents. The solvent is not particularly limited as long as it can dissolve polymer (5) and has no reactivity with electrophile. For example, various organic solvents such as tetrahydrofuran, 1,4-dioxane, toluene, acetonitrile, dichloromethane, chloroform, N-methyl-2-pyrrolidone, N,N-dimethylformamide, and the like, and mixture thereof can be used.

The total amount of the solvent used in the reaction is 1.5- to 70-fold amount, preferably 1.8- to 50-fold amount, most preferably 2- to 30-fold amount, in volume ratio, relative to the polymer (5).

The reaction temperature during the reaction varies depending on the solvent to be used, and is generally 0 to 100°C. The reaction time at this time varies depending on the conditions such as reaction temperature and the like, and is generally preferably 3 hr to 72 hr.

### [Step (B2)]

Step (B2) is a step of obtaining a polymer represented by the aforementioned formula (7-1) by reacting a polymer represented by the aforementioned formula (5-1) obtained in the aforementioned step (A3) with a diethylenetriamine derivative represented by the aforementioned formula (6).

Those of ordinary skill in the art can perform step (B2) by appropriately determining reaction conditions and the like by reference to the explanation of step (B1) described in detail above for the production of the pH-responsive lipid derivative (1).

### [Step (C2)]

Step (C2) is a step of obtaining a pH-responsive lipid derivative represented by the aforementioned formula (47-1) by deprotecting a polymer represented by the aforementioned formula (7-1) obtained in the aforementioned step (B2).

Those of ordinary skill in the art can perform step (C2) by appropriately determining reaction conditions and the like by reference to the explanation of step (C1) described in detail above for the production of the pH-responsive lipid derivative (1).

### <Production method of pH-responsive lipid derivative - coupling method (1) ->

In the pH-responsive lipid derivative (1-1), derivative (36-1) in which the hydrogen atom of the terminal amino group in the derivative represented by the aforementioned formula (36) is substituted by R^{b} (wherein R^{b} is -COCH₃ or -CO-(CH₂)_{b1}-COOH (wherein b1 is 2 or 3)) can be obtained by reacting, instead of the functional group-containing pH-responsive polymer represented by the formula (9) and having a terminal amino group, a functional group-containing pH-responsive polymer (9-1) in which hydrogen atom of the terminal amino group has been substituted with R^{b}, with a functional group-containing lipid represented by the formula (8), in the aforementioned step (D1) in the production method of pH-responsive lipid derivative (1). The reaction can be performed by appropriately determining by reference to the explanation of step (D1) described in detail above for the production of the pH-responsive lipid derivative (1).

Those of ordinary skill in the art can appropriately synthesize functional group-containing pH-responsive polymer (9-1) by reference to polymers and methods known in the pertinent technique field, technical common knowledge, and step (ii) in step (A3), and use same to perform step (D2).

### <Production method of pH-responsive lipid derivative - coupling method (2) ->

A pH-responsive lipid derivative represented by the aforementioned formula (1-1) can also be synthesized by synthesizing a pH-responsive lipid derivative precursor by covalent bonding a lipid derivative and a pH-responsive polymer precursor, and then inducing the pH-responsive lipid derivative.

More specifically, in the pH-responsive lipid derivatives (1-1), a pH-responsive lipid derivative represented by a structure of the following formula (46-1) can be produced by performing the corresponding step (E2), step (F2), and step (G2) in this order according to the following step (E1), step (F1), and step (G1) described above with respect to the production of pH-responsive lipid derivative (1) and using a pH-responsive polymer precursor in which hydrogen atom of the terminal amino group is substituted by R^{b} (wherein R^{b} is -COCH₃ or -CO-(CH₂)_{b1}-COOH (wherein b1 is 2 or 3)) in place of a pH-responsive polymer precursor having a terminal amino group.

Those of ordinary skill in the art can appropriately synthesize a pH-responsive polymer precursor in which hydrogen atom of the terminal amino group is substituted by R^{b} (wherein R^{b} is -COCH₃ or -CO-(CH₂)_{b1}-COOH (wherein b1 is 2 or 3)), by reference to polymers and methods known in the pertinent technique field, technical common knowledge, and step (ii) in step (A3).

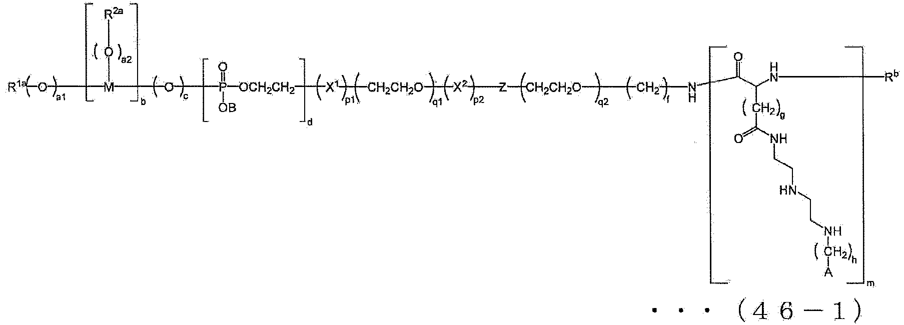

The production methods of the pH-responsive lipid derivatives (1) and (1-1) of the present invention have been described above. The pH-responsive lipid derivative (i) can be produced by appropriately performing step (A), step (A1), step (B), step (C), step (D), step (E), step (F), and step (G) based on the description.

The pH-responsive lipid derivative of the present invention has been described above. In order to further clearly show each structure of a pH-responsive lipid derivative represented by a structure represented by the formula (i), a pH-responsive lipid derivative represented by a structure represented by the formula (1), a pH-responsive lipid derivative represented by a structure represented by the formula (1-1), a pH-responsive lipid derivative represented by a structure represented by the formula (47), a pH-responsive lipid derivative represented by a structure represented by the formula (36), and a pH-responsive lipid derivative represented by a structure represented by the formula (46), respective structures represented by the following formula (i), the formula (1), the formula (1-1), the formula (47), the formula (36), and the formula (46) are enlarged and described in this order.

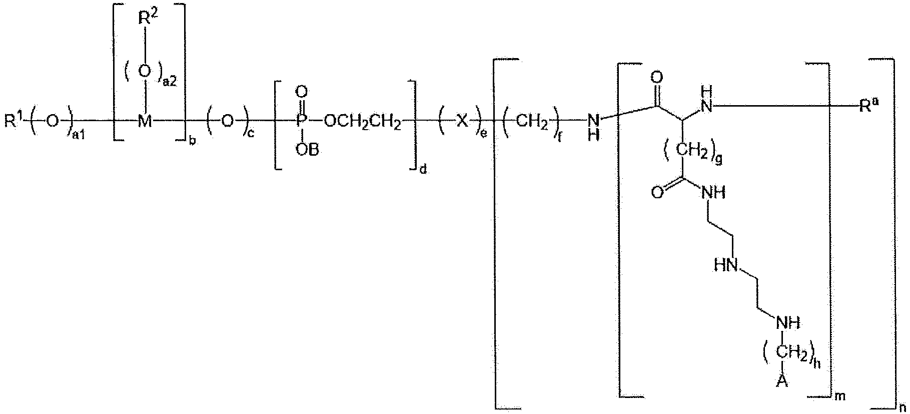

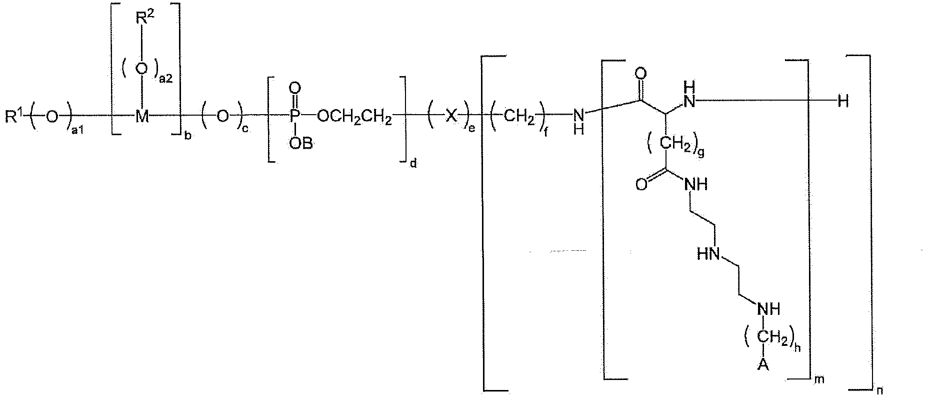

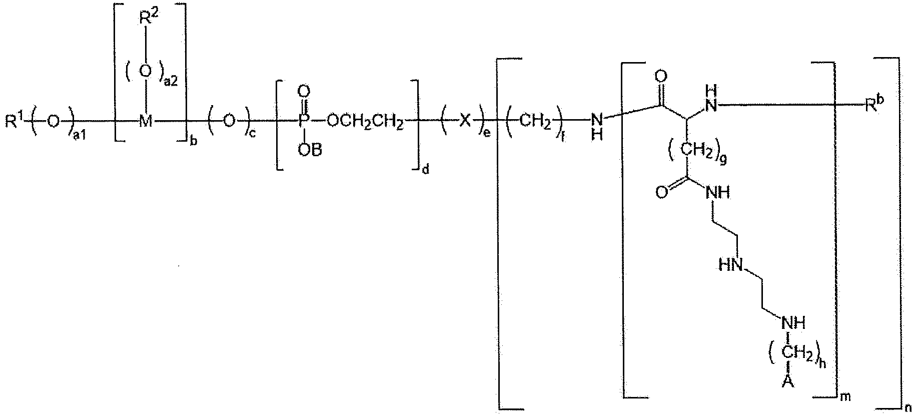

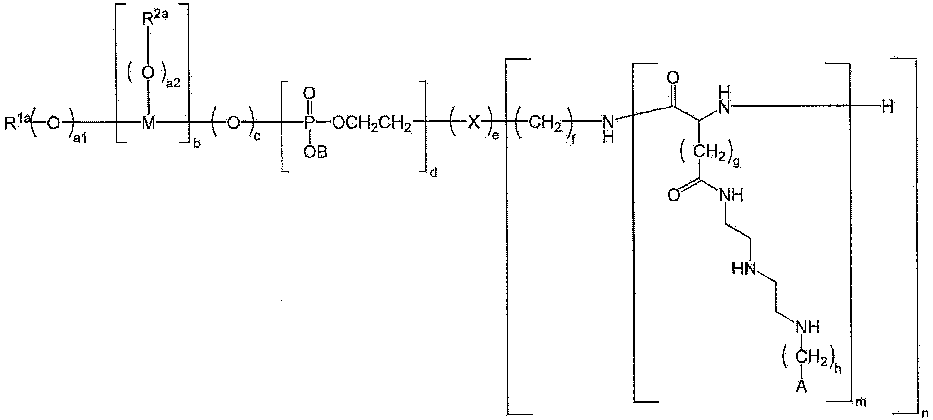

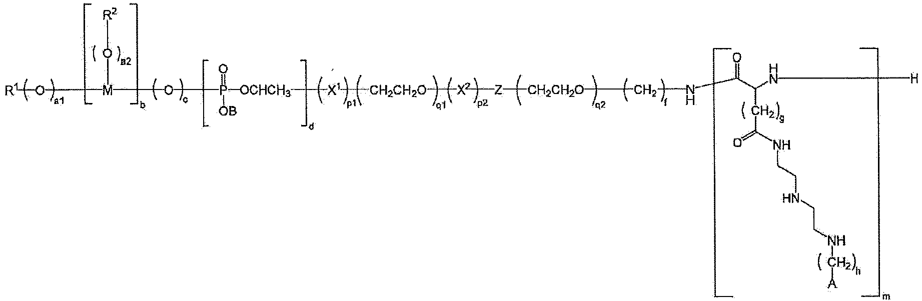

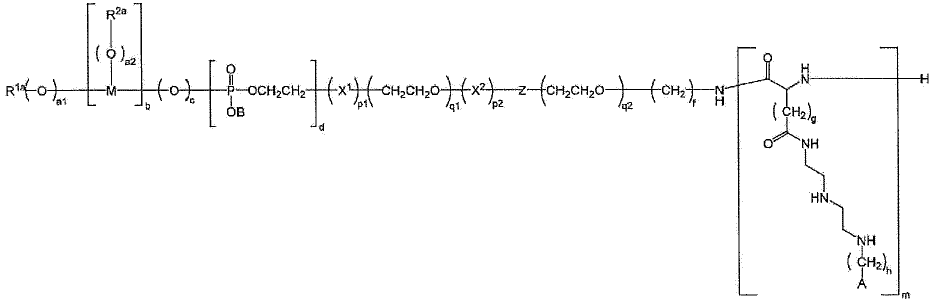

### <Utilization of pH-responsive lipid derivative as transport carrier for drug delivery>

Utilization of the pH-responsive lipid derivatives of the present invention (specifically, pH-responsive lipid derivative (i), pH-responsive lipid derivative (1), and pH-responsive lipid derivative (1-1)) as a transport carrier for drug delivery is now explained. In the following, (A) pH-responsive lipid derivative (1) as a representative pH-responsive lipid derivative (i) of the present invention is explained as a specific example. Those of ordinary skill in the art can similarly utilize the pH-responsive lipid derivative (i) and the pH-responsive lipid derivative (1-1) as transport carriers for drug delivery, based on the explanation.

The transport carrier for drug delivery of the present invention contains a pH-responsive lipid derivative represented by the aforementioned formula (1), and lipid nanoparticles whose basic constituents are phospholipids or their derivatives, or sterols or lipids other than phospholipids can be recited as examples.

The form of the lipid nanoparticles according to the present invention is not particularly limited. Examples of a form dispersed in an aqueous solvent include unilamellar liposome, multilamellar liposome, O/W emulsion, W/O/W emulsion, spherical micelle, solid lipid nanoparticle, amorphous layered structure, and the like. As the lipid nanoparticles according to the present invention, spherical micelle, solid lipid nanoparticles, and liposomes are preferred.

The lipid nanoparticle according to the present invention is a particle having a structure in which hydrophilic portions of a pH-responsive lipid derivative are aligned toward the aqueous phase side of the interface. In the pH-responsive lipid derivative, the lipid portion is hydrophobic and the polymer portion containing the pH-responsive functional group is hydrophilic, so that the pH-responsive portion is exposed on the surface of the lipid nanoparticle. Therefore, the lipid nanoparticles according to the present invention are pH-responsive like pH-responsive lipid derivatives. In other words, it becomes electrically neutral in a neutral environment (pH 7.4) and becomes more cationic under weakly acidic conditions (pH 6.5) around tumor tissues.

Among the constitutive lipids of the lipid nanoparticle according to the present invention, lipids used in forming general lipid nanoparticles can be used as the lipid other than the pH-responsive lipid derivative of the present invention. Examples of such lipid include phospholipid, neutral lipid, cationic lipid, sterol, saturated or unsaturated fatty acid, and the like. One kind or two or more kinds of these can be used in combination.

As the phospholipid, phosphatidyl choline, glycerophospho lipid, sphingophospho lipid, phosphatidylserine, phosphatidylethanolamine, phosphatidylinositol, phosphatidylglycerol, phosphatidic acid, and the like can be mentioned, and specifically dioleylphosphatidylethanolamine (DOPE), dioleylphosphatidic acid (DOPA), and the like can be mentioned. Examples of the sterol include animal-derived sterols such as cholesterol, cholesterol succinic acid, lanosterol, dihydrosterol, desmosterol, dihydrocholesterol, and the like; plant-derived sterols (phytosterols) such as stigmasterol, sitosterol, campesterol, brassicasterol, and the like; microorganism-derived sterols such as thymosterol, ergosterol, and the like, and the like. As the phospholipid, DOPE is preferred, and as the sterol, cholesterol is preferred.

As the neutral lipid, glycerolipid or sphingolipid can be mentioned. Fatty acid residue in these glycerol lipid or sphingo lipid is not particularly limited and examples thereof include saturated or unsaturated fatty acid residues having a carbon number of 12 to 24. Specifically, acyl groups derived from fatty acids such as lauric acid, myristic acid, palmitic acid, palmitoleic acid, stearic acid, oleic acid, linolenic acid, arachidic acid, arachidonic acid, behenic acid, lignoceric acid, and the like can be mentioned.

Examples of the cationic lipid include DODAC (dioctadecyldimethylammonium chloride), DOTMA (N-[2,3-bis(oleoyloxy)propyl]-N,N,N-trimethylammonium chloride), DDAB (didodecyldimethylammonium bromide), DOTAP (1,2-dioleyloxy-3-trimethylammonium propane), DC-Chol (3β-[N-(N',N'-dimethylaminoethane)carbamoyl]cholesterol), DMRIE (1,2-dimyristoyloxypropyl-3-dimethylhydroxyethylammonium bromide), DOSPA (2,3-dioleyloxy-N-[2-(sperminecarboxamido)ethyl]-N,N-dimethyl-1-propanaminum trifluoroacetate), DSTAP (1,2-distearoyl-3-trimethylammonium propane), DODAP (dioleoyl-3-dimethylammonium-propane), and the like. As the cationic lipid, DOTAP and DODAP are preferred.

As the fatty acid and the like, caprylic acid, capric acid, lauric acid, myristic acid, palmitic acid, palmitoleic acid, stearic acid, isostearic acid, oleic acid, linoleic acid, arachidic acid, behenic acid, erucic acid, lignoceric acid, derivatives thereof, and the like can be mentioned.

In the constituent lipids of the lipid nanoparticles according to the present invention, mixing ratios used in forming general lipid nanoparticles can be used as the mixing ratio of the lipid other than the pH-responsive lipid derivative of the present invention.

In the lipid nanoparticle of the present invention, it is preferable that the pH-responsive lipid derivative represented by the aforementioned formula (1) is blended in an amount of 0.1 to 30 mol%, preferably 0.5 to 20 mol%, of the total lipid during production of lipid nanoparticles. When it is less than 0.1 mol %, the proportion of pH-responsive portion on the surface of lipid nanoparticles decreases, and there is a risk that they may not be cationic enough to accumulate in and be taken up by tumor tissues under weakly acidic conditions. When it is more than 30 mol%, stable production of lipid nanoparticles may become difficult.

The size of the lipid nanoparticles according to the present invention is preferably not more than 500 nm in average particle size, more preferably not more than 400 nm in average particle size, and further preferably not more than 200 nm in average particle size. The particle size distribution is preferably not more than 0.5, more preferably not more than 0.45, most preferably not more than 0.4. The average particle size of lipid nanoparticles means the number base mean diameter measured by dynamic light scattering (DLS). Measurement by dynamic light scattering can be performed using commercially available DLS equipment, or the like.

The lipid nanoparticles of the invention contain a pH-responsive lipid derivative that is neutral at pH 7.2 to 7.6 and changes to cationic at pH 6.0 to 6.6 as a constituent component. The surface charge (zeta potential) of lipid nanoparticles also changes in response to changes in pH. The surface charge of lipid nanoparticles at each pH can be measured using a commercially available zeta potential measuring device.

The zeta potential of the lipid nanoparticle of the present invention at pH 7.4 varies depending on the constituent components, a drug to be encapsulated, the content thereof, and the like, and is preferably electrically neutral. For lipid nanoparticles encapsulating nucleic acid, for example, lipid nanoparticles with a range of -10 mV to +10 mV are mainly used.

The pH-responsiveness of the lipid nanoparticle of the present invention can be calculated from the difference between the zeta potential value at pH 6.5 and the zeta potential value at pH 7.4. The difference in the zeta potential of the lipid nanoparticle of the present invention can be calculated from the following formula (F1): Q=(zeta potential at pH 6.5)-(zeta potential at pH 7.4) wherein Q is preferably not less than 2.5 mV, more preferably not less than 5 mV.

Since the lipid nanoparticle of the present invention is electrically neutral under neutral environment of pH 7.4, it exhibits stealth property in blood components and normal tissues, and increases cationicity in response to minute pH change (pH 6.5) around tumor tissues.

Since cell surface is negatively charged, an increase in the cationicity is expected to improve accumulation efficiency and uptake efficiency into tumor tissues. As a result, the low-molecular-weight drugs or nucleic acid pharmaceutical products encapsulated in the transport carrier can be released around the tumor tissues or introduced efficiently into tumor cells.

When the difference (Q) in zeta potential of lipid nanoparticles is smaller than 2.5 mV, the amount of change in zeta potential due to pH change is small. Therefore, there is a possibility that the accumulation in tumor tissues or the efficiency of uptake into cells cannot be improved.

The zeta potential of the lipid nanoparticle of the present invention at pH 6.5 is more preferably a positive value. Since the cell surface has a negative charge, a positive value promotes electrostatic interaction and further promotes uptake into cells.

The production method of the lipid nanoparticles according to the present invention is not particularly limited, and any method available to those skilled in the art may be employed. In one embodiment, all lipid components are dissolved in an organic solvent such as chloroform or the like, the mixture is dried under reduced pressure using an evaporator or by spray drying using a spray dryer to form a lipid membrane, an aqueous solvent is added to the above-mentioned mixture after being dried, and the mixture is emulsified by an emulsifying equipment such as homogenizer or the like, an ultrasonic emulsifying equipment, a high-pressure injection emulsifying equipment, or the like. In addition, it can be produced by methods well known as methods for producing liposomes such as a reversed-phase normal pressure method and the like. When control of the size of lipid nanoparticles is desired, extrusion (extrusion filtration) can be performed under high pressure using a membrane filter with uniform pore size, or the like.

The kind of the aqueous solvent (dispersion medium) is not particularly limited. For example, a buffer such as phosphate buffer, citrate buffer, phosphate buffered physiological saline, or the like, saline, a cell culture medium, or the like can be used. These aqueous solvents (dispersion media) can stably disperse lipid nanoparticles. Sugar (e.g., glucose, lactose, sucrose, and the like) aqueous solution, polyhydric alcohol (e.g., glycerin, propylene glycol, and the like), and the like may be added in addition to water. To stably store for a long time a lipid membrane structure dispersed in an aqueous solvent, it is desirable to reduce the amount of electrolyte in the aqueous solvent as much as possible and also desirable to remove dissolved oxygen by nitrogen bubbling, from the viewpoint of physical stability such as aggregation. Furthermore, when storing by freeze-drying or spray-drying, for example, use of an aqueous sugar solution or a polyhydric alcohol aqueous solution in cryopreserving a lipid membrane structure dispersed in an aqueous solvent enables long-term preservation. The concentration of the aqueous solvent is not particularly limited. For example, it is preferably 2 to 20%(W/V), more preferably 5 to 10%(W/V), in an aqueous sugar solution. It is preferably 1 to 5%(W/V), more preferably 2 to 2.5%(W/V), in a polyhydric alcohol aqueous solution. In buffers, the concentration of a buffering agent is preferably 5 to 50 mM, more preferably 10 to 20 mM. The concentration of a lipid membrane structure in an aqueous solvent is not particularly limited, and the total concentration of lipid in an aqueous solvent is preferably 0.1 to 500 mM, more preferably 1 to 100 mM.

As a method of further drying lipid nanoparticles dispersed in an aqueous solvent, general freeze-drying and spray drying can be mentioned. In this case, as the aqueous solvent, as described above, an aqueous sugar solution, preferably a sucrose aqueous solution or a lactose aqueous solution, can be used. When lipid nanoparticles dispersed in an aqueous solvent are produced and further dried, the lipid nanoparticles can be stored for a long term. In addition, when an aqueous pharmaceutical solution is added to the dried lipid nanoparticles, the lipid mixture is efficiently hydrated and the drug can be efficiently retained in the lipid nanoparticles.

The lipid nanoparticle according to the present invention is particularly useful as a carrier for delivering a medicinal component to tumor tissues. The components that the lipid nanoparticles according to the present invention encapsulate inside the particles are not particularly limited as long as they are of a size permitting the encapsulation. For example, any substances such as low molecular weight drugs, nucleic acids, saccharides, peptides, metal compounds, or the like can be encapsulated. The component that the lipid nanoparticle according to the present invention encapsulates is preferably one or more kinds selected from the group consisting of low molecular weight drugs, nucleic acids, and peptides. Examples of the nucleic acid include antisense oligonucleotide, siRNA, miRNA, shRNA, mRNA, nucleic acid aptamer, decoy nucleic acid, ribozyme, CpGoligo nucleic acid, and the like.

As the low molecular weight medicament, medicaments having a molecular weight of not more than about 1000 can be mentioned. The low molecular weight drug may be, for example, an anticancer agent or a contrasting agent. Examples of the anticancer agent include paclitaxel, doxorubicin, cisplatin, gemcitabine, and the like.

As the peptides, antibody medicaments (e.g., herceptin, avastin, cyramza, and the like) and the like can be mentioned.

### [Example]

The present invention is explained in more detail in the following by referring to Examples and Experimental Examples; however, the present invention is not limited to the following Examples and Experimental Examples.

### [Measurement of molecular weight by gel filtration chromatography (GPC) analysis]

Trade name "Prominence" manufactured by Shimadzu Corporation or trade name "Infinity Lab 2D-LC" manufactured by Agilent Technologies (hereinafter Agilent) was used.

The measurement of poly(β-benzyl L-glutamic acid) (hereinafter to be abbreviated as pBLG) was performed under conditions of column: PL gel (trade name "MIXED-D") (two columns combined) manufactured by Agilent, column temperature: 40°C, sample concentration: 0.2 wt%, injection volume: 100 µL, eluent: 10 mM LiBr-added N-methyl-2-pyrrolidone, flow rate: 0.6 ml/min, detector; refractive index detector (RI), standard: polymethyl methacrylate.

The measurement of poly(glutamic acid diethylenetriamine carboxylic acid) (hereinafter to be abbreviated as pGlu (DET-Car)) and poly(glutamic acid diethylenetriamine sulfonic acid) (hereinafter to be abbreviated as pGlu (DET-Sul)) was performed under conditions of column: manufactured by Tosoh Corporation, two kinds of TSK gel (trade name "TSKgel G3000PW_{XL}" and trade name "TSKgel G5000PW_{XL}") combined, column temperature: 40°C, sample concentration: 0.2 wt%, injection volume: 100 µL, eluent: 10 mM HEPES buffer (pH 7.4) added with NaCl to 500 mM, flow rate: 0.4 ml/min, detector: refractive index detector (RI), standard: polyethylene glycol.

### [Production Example 1] Synthesis of N-(2-(2-aminoethylamino)ethyl)-3-aminopropionic acid t-butyl ester (DET-Car tBu, compound 3))

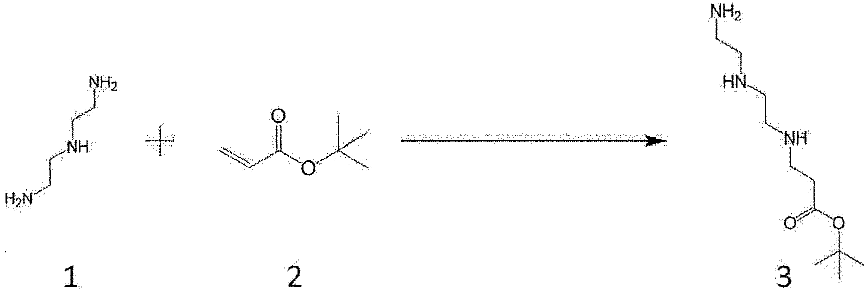

Diethylenetriamine (hereinafter to be abbreviated as DET, 474.9 g, 4.6 mol, compound 1) was dissolved in 500 mL of methanol under ice-cooling, and separately, acrylic acid t-butyl ester (5.9 g, 46 mol, compound 2) was dissolved in 270 mL of methanol to prepare various solutions. To the DET solution under stirring, the acrylic acid t-butyl ester solution was added dropwise over 3 hr while maintaining at 15-25°C.

After the completion of the dropwise addition, the mixture was stirred at 15-25°C for 1 hr and evaporated under reduced pressure after stirring to obtain a yellow transparent oil (109.7 g).

The obtained oil was subjected to column purification using a silica gel column to obtain DET-Car tBu (compound 3) as a colorless transparent oil (66.0 g).

### ¹H NMR(MeOD 400MHz):δ 2.60-2.85(m,10H),2.42(t,2H),1.44(s,9H). ESI-MS:calcd for C₁₁H₂₆N₃O₂⁺[M+H⁺] :232.2020; found 232.1995.

### [Production Example 2] Synthesis of AZ-pGlu(DET-C2-Car)20 (compound 7, m=22)

### (AZ-pBLG20 (compound 6))

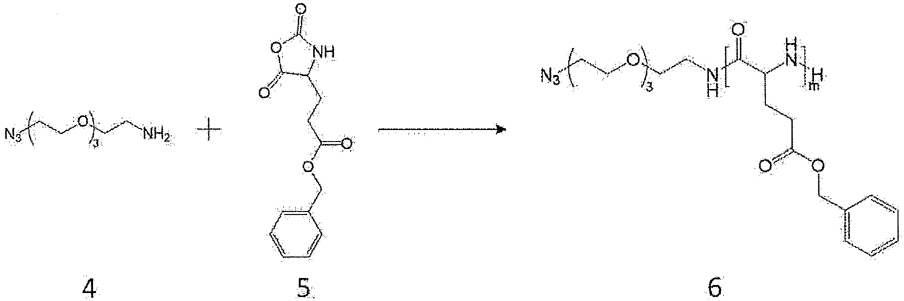

BLG-N-carboxyanhydride (hereinafter to be abbreviated as BLG-NCA, 8.2 g, 31 mmol, compound 5) was dissolved under a nitrogen atmosphere in 45 mL of N,N-dimethylformamide (hereinafter to be abbreviated as DMF, 5.5-fold amount relative to weight of BLG-NCA) and 45 mL of dichloromethane (hereinafter to be abbreviated as DCM, 5.5-fold amount relative to weight of BLG-NCA). To the obtained solution was added 900 mg of 11-azido-3,6,9-trioxa undecane-1-amine (0.9 g, 4.1 mmol, compound 4), and the mixture was stirred under a nitrogen atmosphere for 30 min at 30°C. The completion of the reaction was confirmed by the disappearance of the peak (-NH-CH(CO-)-CH₂-),δ=4.35-4.60 ppm) derived from the amide α-position of compound 5 by ¹H-NMR measurement in a deuterated DMSO solvent at 25°C.

After completion of the reaction, DCM was evaporated under reduced pressure, and 57 mL of methanol (7.0-fold amount relative to weight of BLG-NCA) and 114 mL of ion exchange water (14.0-fold amount relative to weight of BLG-NCA) were added while stirring the obtained solution to precipitate a white solid. The obtained solid was collected by filtration, dissolved in 23 mL of tetrahydrofuran (hereinafter to be abbreviated as THF), and 208 mL of ethanol (EtOH) was added to precipitate a white solid. The obtained solid was collected by filtration and dried under reduced pressure to give AZ-pBLG20 (compound 6) as a white powder (5.6 g).

The object product (10 mg) was dissolved in a deuterated dimethyl sulfoxide (DMSO) solvent (1.0 mL) added with trimethylsilane (TMS) and, using the sample, the structure was confirmed by ¹H-NMR analysis, and it was confirmed to be AZ-pBLG20.

¹H NMR(deuterated DMSO 400MHz):δ7.00-7.60(m, degree of polymerization×5H),4.80-5.30(m, degree of polymerization×2H),3.75-4.40(m, degree of polymerization×1H),3.00-3.65(m,16H),1.55-2.70(m, degree of polymerization×4H).

In addition, the molecular weight was calculated by size-exclusion chromatography (SEC) analysis, and it was confirmed to be Mn=3,600, Mw=5,200, PDI=1.44.

### Synthesis of (AZ-pGlu(DET-C2-Car)20 (compound 7, m=22))

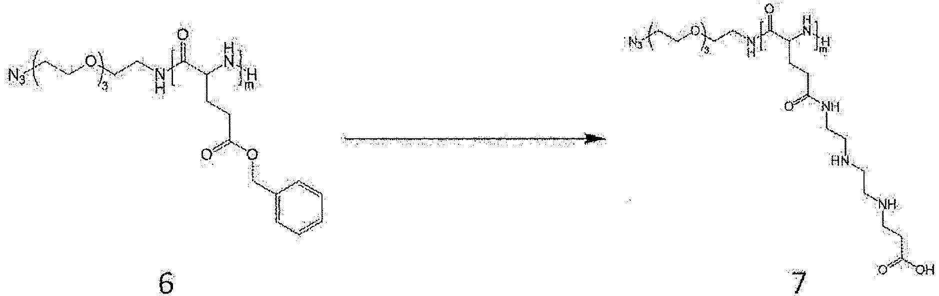

AZ-pBLG20 (3.0 g, number of moles of benzyl group in polymer 13.6 mmol, compound 6) and 2-hydroxypyridine (3.9 g, 41 mmol, 3-fold amount relative to number of moles of benzyl group in polymer) were uniformly dissolved using compound 3 (15.7 g, 68 mmol, 5-fold amount relative to number of moles of benzyl group in polymer) synthesized in Production Example 1 and 15 mL of THF and the mixture was reacted at 50°C for 21 hr. The completion of the reaction was confirmed by the disappearance of the peak (CH₂-C₆H₅,δ=5.02 ppm) at the benzyl-position in the pBLG side chain by ¹H-NMR measurement in a deuterated DMSO solvent at 25°C.

After completion of the reaction, the mixture was evaporated under reduced pressure to give a yellow oily solution. To the obtained solution was added dropwise 46.5 g of 6N hydrochloric acid solution (15.5-fold amount relative to weight of compound 6), and the mixture was reacted at 30°C for 17 hr. The completion of the reaction was confirmed by the disappearance of the peak (C-(CH₃)₃,δ=1.45 ppm) of tBu group in the side chain by ¹H-NMR measurement in an aqueous deuterium chloride solvent at 25°C.

After completion of the reaction, 5N sodium hydroxide solution (24.9 g) was added to adjust pH to 3-4, and dialysis purification and freeze-drying were performed to obtain AZ-pGlu(DET-C2-Car)20 (compound 7) as a white yellow powder (1.9 g).

To 0.65% deuterium chloride deuterium oxide solution was added trimethylsilyl propionic acid (TMSP). To the object product (10 mg) was added the above-mentioned deuterated solvent (1.0 mL). Using the resulting dissolved sample, the structure was confirmed by ¹H-NMR analysis, and it was confirmed to be AZ-pGlu(DET-C2-Car)20.

¹H NMR(deuterium chloride+deuterium oxide 400MHz):δ 4.30-4.50(m, degree of polymerization×1H),3.65-3.80(m,14H),3.20-3.65(m, degree of polymerization×10H+2H),2.85-3.00(t, degree of polymerization×2H),2.35-2.55(m, degree of polymerization×2H),1.90-2.25(m, d, degree of polymerization×2H).

In addition, the molecular weight was calculated by SEC analysis, and it was confirmed to be Mn=6,300,Mw=7,800,PDI=1.24 at pH 7.4.

In the following, a specific calculated method for AZ-pGlu(DET-Car)20 is recited as one embodiment.

### (Calculation of m)

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 22.

Hereinafter, the final target polymer of each Production Example or Example is sometimes referred to as the polymer of Production Example O or the polymer of Example O. As an example, AZ-pGlu(DET-C2-Car) 20 is the polymer of Production Example 2.

### [Production Example 3] Synthesis of AZ-pGlu(DET-C2-Car)30 (compound 7, m=32)

### Synthesis of (AZ-pBLG30 (compound 6))

By an operation similar to that in Production Example 2 except that 10.0 g of compound 5 and 256 mg of compound 4 were used, AZ-pBLG30 (compound 6) was obtained as a white powder (7.9 g).

By a method similar to that in Production Example 2, the structure was confirmed by ¹H-NMR analysis, and it was confirmed to be AZ-pBLG30.

¹H NMR(deuterated DMSO 400MHz):δ 7.00-7.60(m, degree of polymerization×5H),4.80-5.30(m, degree of polymerization×2H),3.75-4.40(m, degree of polymerization×1H),3.00-3.65(m,16H),1.55-2.70(m, degree of polymerization×4H).

In addition, the molecular weight was calculated by SEC analysis, and it was confirmed to be Mn=7,200, Mw=9,900, PDI=1. 38.

Successively, by an operation similar to that in Production Example 2 except that 3.5 g of compound 6 was used, AZ-pGlu(DET-C2-Car)30 (compound 7) was obtained as a white-yellow powder (3.8 g).

By a method similar to that in Production Example 2, the structure was confirmed by ¹H-NMR analysis, and it was confirmed to be AZ-pGlu(DET-C2-Car)30.

¹H NMR(deuterium chloride +deuterium oxide 400MHz):δ 4.30-4.50(m, degree of polymerization×1H),3.65-3.80(m,14H),3.20-3.65(m, degree of polymerization×10H+2H),2.85-3.00(t, degree of polymerization×2H),2.35-2.55(m, degree of polymerization×2H),1.90-2.25(m, d, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 32.

In addition, the molecular weight was calculated by SEC analysis, and it was confirmed to be Mn=9,100, Mw=11,400, PDI=1.25 at pH 7.4.

### [Production Example 4] Synthesis of AZ-pGlu(DET-C2-Car)100 (compound 7, m=98)

### Synthesis of (AZ-pBLG100 (compound 6))

By an operation similar to that in Production Example 2 except that 39.8 g of compound 5 and 308 mg of compound 4 were used to obtain AZ-pBLG100 (compound 6) as a white powder (31.4 g).

By a method similar to that in Production Example 2, the structure was confirmed by ¹H-NMR analysis, and it was confirmed to be AZ-pBLG100.

¹H NMR(deuterated DMSO 400MHz):δ 7.00-7.60(m, degree of polymerization×5H),4.80-5.30(m, degree of polymerization×2H),3.75-4.40(m, degree of polymerization×1H),3.00-3.65(m,16H),1.55-2.70(m, degree of polymerization×4H).

In addition, the molecular weight was calculated by SEC analysis, and it was confirmed to be Mn=19,000, Mw=22,100, PDI=1.16.

Successively, by an operation similar to that in Production Example 2 except that 30.8 g of compound 6 was used, AZ-pGlu(DET-C2-Car)100 (compound 7) was obtained as a white-yellow powder (30.3 g).

By a method similar to that in Production Example 2, the structure was confirmed by ¹H-NMR analysis, and it was confirmed to be AZ-pGlu(DET-C2-Car)100(m=98).

¹H NMR(deuterium chloride +deuterium oxide 400MHz):δ 4.30-4.50(m, degree of polymerization×1H),3.65-3.80(m,14H),3.20-3.65(m, degree of polymerization×10H+2H),2.85-3.00(t, degree of polymerization×2H),2.35-2.55(m, degree of polymerization×2H),1.90-2.25(m, d, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=17,500, Mw=19,300, PDI=1.10 at pH 7.4.

### [Production Example 5] Synthesis of AZ-pGlu(DET-C3-Car)100 (compound 11, m=98)

### Synthesis of (N-(2-(2-aminoethylamino)ethyl)-4-aminobutyric acid t-butyl ester (DET-C3-Car tBu, compound 10))

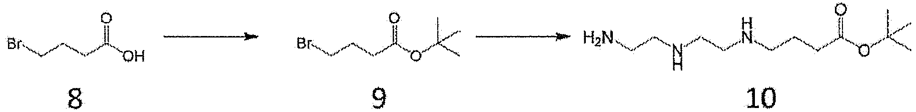

30 g of magnesium sulfate and 2 mL of sulfuric acid were mixed with 200 mL of DCM, and the mixture was stirred for 10 min. Then, 4-bromobutyric acid (10.0 g, 59.9 mmol, compound 8) and t-butanol (22.2 g, 132.9 mmol) were added. The reaction solution was stirred under an argon atmosphere at 25°C for 72 hr, and quenched by adding dropwise to saturated aqueous sodium hydrogen carbonate.

After completion of the reaction, the organic layer was partitioned and washed with saturated aqueous sodium hydrogen carbonate and saturated brine, and dried over magnesium sulfate. After filtration, the filtrate was evaporated under reduced pressure to obtain 4-bromobutyric acid t-butyl ester (compound 9) as a colorless transparent oil (11.1 g).

¹H NMR(CDCl₃ 400MHz)δ
3.45(t,2H),2.40(t,2H),2.13(q,2H),1.45(s,9H) .
¹³C NMR(CDCl₃ 100MHz)δ 27.9,28.0,32.8,33.7,80.5,171.7

DET (36.9 g, 357.7 mmol, compound 1) was dissolved in 100 mL of DCM. To the obtained DET solution was added dropwise compound 9 (10.0 g, 40.8 mmol), and the mixture was reacted at room temperature for 24 hr.

After completion of the reaction, the organic layer was washed twice with 500 mM NaCl solution, dried over sodium sulfate. After filtration and evaporation under reduced pressure, the obtained crude product was diluted with methanol and acidified with 5 M HCl. To this solution was added dropwise diethyl ether to precipitate a white solid. The obtained solid was collected by filtration and dried under reduced pressure to obtain DET-C3-Car tBu (compound 10) as a white solid (3.8 g).

¹H NMR(D₂O, 400MHz) δ 3.42-3.64(m,8H),3.07(t,2H),2.43(t,2H),1.954(q,2H),1.45(s,9H).

ESI-MS:calcd for C₁₂H₂₈N₃O₂⁺[M+H⁺] :246.2176; found:246.2147.

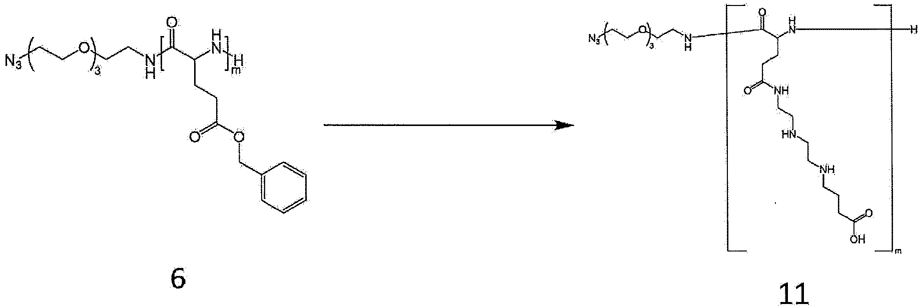

AZ-pBLG100 (50 mg, number of moles of benzyl group in polymer 0.23 mmol, compound 6) synthesized in Production Example 4 and 2-hydroxypyridine (107 mg, 1.1 mmol, 5-fold amount relative to number of moles of benzyl group in polymer) were uniformly dissolved using DET-C3-Car tBu (852 mg, 3.5 mmol, 15-fold amount relative to number of moles of benzyl group in polymer, compound 10) and 5 mL of THF, and the mixture was reacted at 50°C for 21 hr. The completion of the reaction was confirmed by the disappearance of the peak (CH₂-C₆H₅, δ=5.02 ppm) at the benzyl-position in the pBLG side chain by ¹H-NMR measurement in a deuterated DMSO solvent at 25°C.

After completion of the reaction, the mixture was evaporated under reduced pressure to give a yellow oily solution. To the obtained solution was added dropwise 40 mL of 1N hydrochloric acid solution (15.5-fold amount relative to weight of compound 6), and the mixture was reacted at 40°C for 2 days. The completion of the reaction was confirmed by the disappearance of the peak (C-(CH₃)₃,δ=1.45 ppm) of tBu group in the side chain by ¹H-NMR measurement in an aqueous deuterium chloride solvent at 25°C.

After completion of the reaction, dialysis purification and freeze-drying were performed to obtain AZ-pGlu(DET-C3-Car)100 as a white yellow powder (52.7 mg).

The object product (10 mg) was dissolved in deuterium oxide (0.6 mL) added with trimethylsilyl propionic acid (TMSP) and, using the sample, the structure was confirmed by ¹H-NMR analysis, and it was confirmed to be AZ-pGlu(DET-C3-Car)100.

¹H NMR(deuterium oxide 400MHz)δ 4.30-4.50(b, degree of polymerization×1H),3.65-3.80(m,14H),3.00-3.65(m, degree of polymerization×10H+2H),2.30-2.55(b, degree of polymerization×4H),1.90-2.25(b, d, degree of polymerization×2H),1.82(b, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=17,700, Mw=20,400, PDI=1.15 at pH 7.4.

### [Production Example 6] Synthesis of AZ-pGlu(DET-C2-Sul)100 (compound 15, m=98)

### Synthesis of (N-(2-(2-aminoethylamino)ethyl)-2-aminoethanesulfonic acid 2,2,2-trifluoroethyl ester (compound 14))

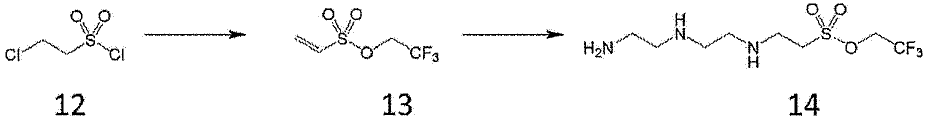

2,2,2-Trifluoroethanol (hereinafter to be abbreviated as TFE, 18.4 g, 183.9 mmol) and triethylamine (27.9 g, 275.7 mmol) were dissolved in 100 mL of DCM and then ice-cooled. To the mixed solution was added dropwise 2-chloroethanesulfonic acid chloride (15.0 g, 92 mmol, compound 12), and the mixture was stirred at 20-30°C for 2 hr.

After completion of the reaction, filtration was performed, and the obtained filtrate was washed with 1 M HCl and ion exchange water. The organic layer was dried over magnesium sulfate. After filtration, the filtrate was evaporated under reduced pressure to obtain vinylsulfonic acid 2,2,2-trifluoroethyl ester (compound 13) as a colorless transparent oil (14.5 g).

¹H NMR(CDCl₃, 400MHz)δ
6.60(m,1H),6.51(d,1H),6.23(d,1H),4.42(q,2H).
¹³C NMR(CDCl₃, 100MHz)δ 64.80,120.55,123.09,131.87.
¹⁹F NMR(CDCl₃, 376MHz,δ)-74.0.

DET (60.7 g, 588 mmol, compound 1) was dissolved in 100 mL of DCM and ice-cooled. Thereto was added dropwise compound 13 (12.0 g, 63.1 mmol), and the mixture was reacted at room temperature for 24 hr.

After completion of the reaction, the organic layer was washed twice with 500 mM NaCl solution, dried over sodium sulfate. After filtration and evaporation under reduced pressure, the obtained crude product was diluted with methanol and acidified with 5 M HCl. To this solution was added dropwise diethyl ether to precipitate a white solid. The obtained solid was collected by filtration and dried under reduced pressure to obtain DET-C2-Sul TFE (compound 14) as a white solid (9.5 g).

¹H NMR(D₂O, 400MHz)δ 4.92(q,2H),4.02(t,2H),3.74(t,2H),3.45-3.61(m,8H).

ESI-MS:calcd for C₈H₂₀N₃O₃S⁺[M+H⁺] :294.1094; found:294.1101.

### Synthesis of (AZ-pGlu(DET-C2-Sul)100 (compound 15))

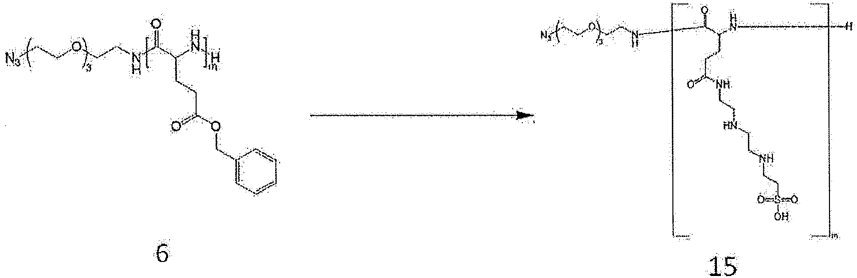

AZ-pBLG100 (50 mg, number of moles of benzyl group in polymer 0.23 mmol, compound 6) synthesized in Production Example 4 and 2-hydroxypyridine (107 mg, 1.1 mmol, 5-fold amount relative to number of moles of benzyl group in polymer) were uniformly dissolved using compound 14 (1.02 g, 3.5 mmol, 15-fold amount relative to number of moles of benzyl group in polymer) and 5 mL of THF, and the mixture was reacted at 50°C for 21 hr. The completion of the reaction was confirmed by the disappearance of the peak (CH₂-C₆H₅,δ=5.02 ppm) at the benzyl-position in the pBLG side chain by ¹H-NMR measurement in a deuterated DMSO solvent at 25°C.

To the reaction solution was added 40 mL of 0.3 M NaOH NMP/MeOH solution and the mixture was stirred for 24 hr at room temperature.

After completion of the reaction, dialysis purification was performed using 0.01 M HCl aqueous solution and ion exchange water, the mixture was freeze-dried, and 5N sodium hydroxide solution (24.9 g) was added to adjust pH to 3-4. Then, dialysis purification and freeze-drying were performed to obtain AZ-pGlu(DET-C2-Sul)100 as a white yellow powder (49.1 mg) .

By a method similar to that in Production Example 4, the structure was confirmed by ¹H-NMR analysis, and it was confirmed to be AZ-pGlu(DET-C2-Sul)100.

¹H NMR(deuterium oxide 400MHz):δ 4.30-4.50(b, degree of polymerization×1H),3.90(t, degree of polymerization×2H),3.65-3.80(m,14H),3.00-3.65(m, degree of polymerization×10H+2H),2.40(b, degree of polymerization×2H),1.90-2.25(b, d, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=19,300, Mw=21,600, PDI=1.1.2 at pH 7.4.

### [Production Example 7] Synthesis of AZ-pGlu(DET-C3-Sul)100 (compound 20, m=98)

### Synthesis of (N-(2-(2-aminoethylamino)ethyl)-3-aminopropanesulfonic acid 2,2,2-trifluoroethyl ester (DET-C3-Sul) TFE (compound 19))

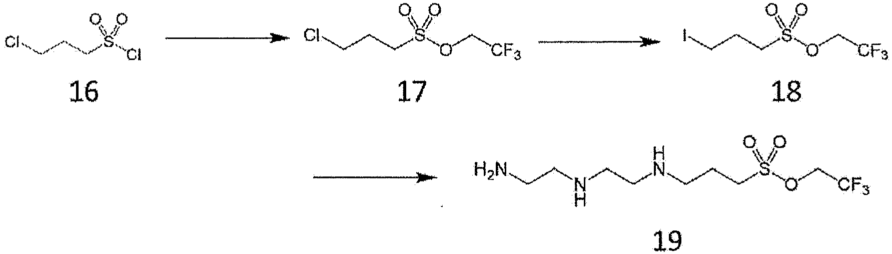

TFE (16.9 g, 168.9 mmol) and triethylamine (17.1 g, 169.0 mmol) were dissolved in 100 mL of DCM and then ice-cooled. To the mixed solution was added dropwise 3-chloropropanesulfonic acid chloride (15.0 g, 84.7 mmol, compound 16), and the mixture was stirred at 20-30°C for 2 hr.

After completion of the reaction, filtration was performed, and the obtained filtrate was washed with 1 M HCl and ion exchange water. The organic layer was dried over magnesium sulfate. After filtration, the filtrate was evaporated under reduced pressure to obtain 3-chloropropanesulfonic acid 2,2,2-trifluoroethyl ester (compound 17) (16.9 g).

¹H NMR (CDCl₃, 500MHz)δ
4.52(q,2H),3.68(t,2H),3.43(t,2H),2.37(m,3H).
¹³C NMR(CDCl₃, 100MHz)δ 121.8,63.8,48.7,42.2,28.3.
¹⁹F NMR(CDCl₃, 376MHz)δ -73.9

18.7 g of sodium iodide (18.7 g, 124.8 mmol) was prepared into a suspension using 40 mL of acetone, compound 17 (15.0 g, 62.3 mmol) was added, and the mixture was stirred at 56°C for 3 days.

After completion of the reaction, the mixture was filtered and the filtrate was evaporated under reduced pressure. The mixture was diluted with dichloromethane, and partitioned and washed with ion exchange water. The organic layer was dried over magnesium sulfate. The mixture was filtered and the filtrate was evaporated under reduced pressure to obtain 3-iodo propanesulfonic acid 2,2,2-trifluoroethyl ester (compound 18) (16.8 g).

¹H NMR (CDCl₃, 400MHz) δ
4.52(q,2H),3.36(t,2H),3.31(t,2H),2.39(m,3H).
¹³C NMR(CDCl3, 100MHz)δ 121.9,63.8,51.9,27.0,1.8.
¹⁹F NMR(CDCl3, 376MHz)δ -73.9

DET (39.8 g, 385.8 mmol, compound 1) was dissolved in 100 mL of DCM and ice-cooled. Thereto was added dropwise compound 18 (16.0 g, 48.2 mmol), and the mixture was reacted at room temperature for 24 hr.

After completion of the reaction, the organic layer was washed twice with 500 mM NaCl solution, dried over sodium sulfate. After filtration and evaporation under reduced pressure, the obtained crude product was diluted with methanol and acidified with 5 M HCl. To this solution was added dropwise diethyl ether to precipitate a white solid. The obtained solid was collected by filtration and dried under reduced pressure to obtain DET-C3-Sul TFE (compound 19) as a white solid (7.0 g).

¹H NMR(D₂O, 400MHz)δ 4.92(q,2H),4.02(t,2H),3.74(t,2H),3.45-3.61(m,8H).

ESI-MS:calcd for C₈H₂₀N₃O₃S⁺ [M+H⁺]:308.1250; found:308.1272.

### Synthesis of (AZ-pGlu(DET-C3-Sul)100 (compound 20, m=98))

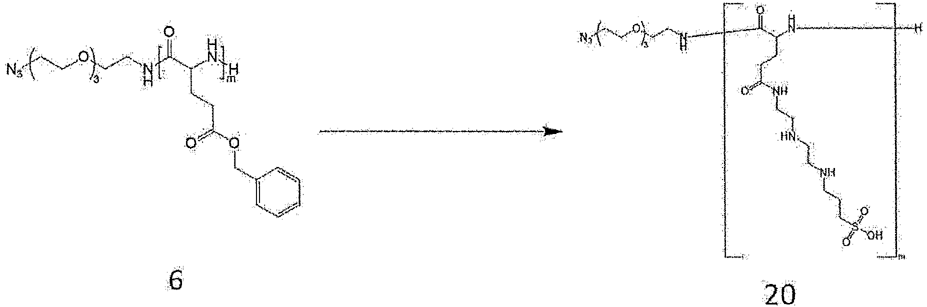

By an operation similar to that in Production Example 6 except that DET-C3-Sul TFE (1.06 g, 3.4 mmol, compound 19) was used instead of DET-C2-Sul TFE (compound 14) to obtain AZ-pGlu(DET-C3-Sul)100 (compound 20) as a white-yellow powder (53.1 mg).

By a method similar to that in Production Example 4, the structure was confirmed by ¹H-NMR analysis, and it was confirmed to be AZ-pGlu(DET-C3-Sul)100.

¹H NMR(deuterium oxide 400MHz)δ 4.30-4.50(b, degree of polymerization×1H),3.65-3.80(m,14H),3.20-3.50(m, degree of polymerization×10H+2H),3.05(b, degree of polymerization×2H),2.45(b, degree of polymerization×2H),1.80-2.35(b, m, degree of polymerization×4H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=20,800, Mw=23,100, PDI=1.11 at pH 7.4.

### [Production Example 8] Synthesis of SH-pGlu(DET-C2-Car)30 (compound 23, m=33)

### Synthesis of (SH-pBLG30 (compound 22))

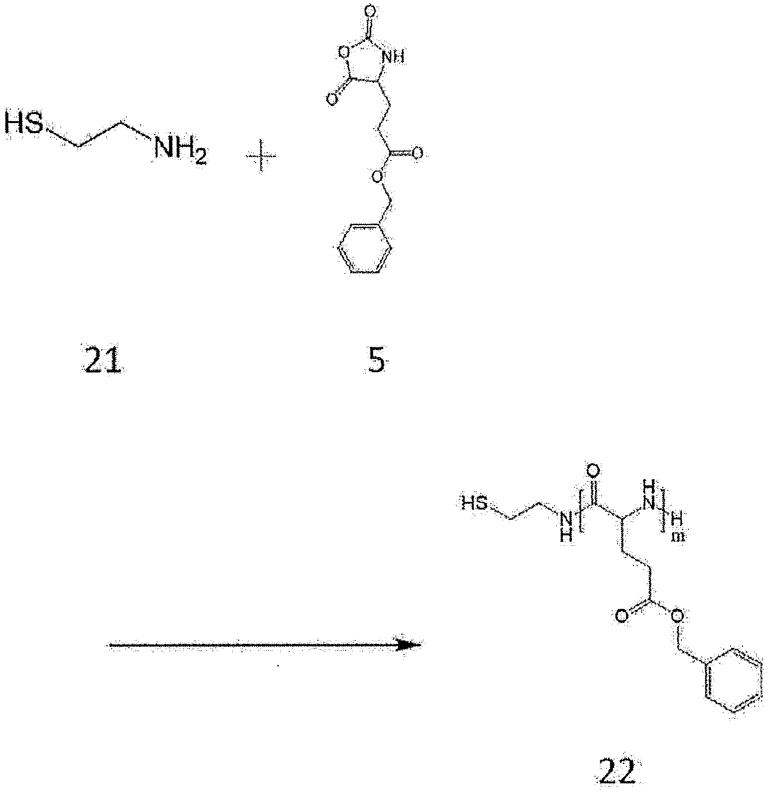

By an operation similar to that in Production Example 2 except that aminoethanethiol (90 mg, compound 21) was used instead of compound 4, SH-pBLG30(compound 22) was obtained as a white powder (7.8 g).

By a method similar to that in Production Example 2, the structure was confirmed by ¹H-NMR analysis, and it was confirmed to be SH-pBLG30.
¹H NMR(deuterated DMSO 400MHz)δ 7.00-7.60(m, degree of polymerization×5H),4.80-5.30(m, degree of polymerization×2H+1H),3.75-4.40(m, degree of polymerization×1H),2.65-3.20(m,4H),1.55-2.65(m, degree of polymerization×4H).

In addition, the molecular weight was calculated by SEC analysis, and it was confirmed to be Mn=7,600, Mw=10,600, PDI=1.39.

### Synthesis of ((SH-pGlu(DET-C2-Car)30 (compound 23), m=33))

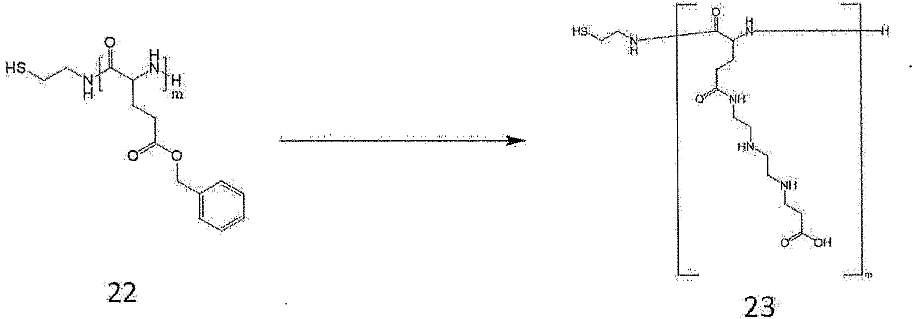

Successively, by an operation similar to that in Production Example 2 except that SH-pBLG30 (3.5 g, compound 22) was used instead of compound 6, SH-pGlu(DET-C2-Car)30 (compound 23) was obtained as a white-yellow powder (3.8 g).

By a method similar to that in Production Example 2, the structure was confirmed by ¹H-NMR analysis, and it was confirmed to be SH-pGlu(DET-C2-Car)30.

¹H NMR(deuterium chloride +deuterium oxide 400MHz)δ 4.30-4.50(m, degree of polymerization×1H),3.20-3.85(m, degree of polymerization×10H),3.10-3.20(m,2H),2.65-3.00(t+m, degree of polymerization×2H+2H),2.35-2.55(m, degree of polymerization×2H),1.90-2.25(m, d, degree of polymerization×2H).

With a peak (3.10-3.20 ppm) derived from the initiator as the standard value (2H), the degree of polymerization was calculated from the integration value of a peak (4.30-4.50 ppm) derived from the amide group α-position of polypeptide, and the degree of polymerization was confirmed to correspond to 33.

In addition, the molecular weight was calculated by SEC analysis, and it was confirmed to be Mn=9,500, Mw=11,800, PDI=1.24 at pH 7.4.

### [Production Example 9] Synthesis of AZ-Ph-(pGlu(DET-C2-Car)30)2 (compound 31, m=29, n=2)

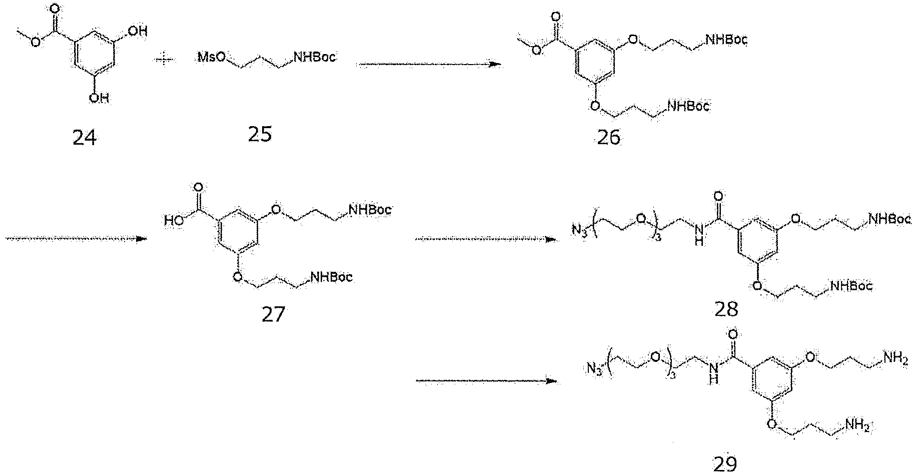

### Synthesis of (3,5-bis [(3-Boc-amino)propyloxy]benzoic acid methyl ester (compound 26))

3,5-Dihydroxy benzoic acid methyl (3.0 g, 17.8 mmol, compound 24) and 3-Boc-aminopropylmesylate (10.2 g, 40.2 mmol, compound 25) were completely dissolved in 30 mL of DMF. Then, potassium carbonate (9.5 g, 68.7 mmol) was added, and the mixture was stirred at 40°C for 48 hr.

After completion of the reaction, the reaction solvent was evaporated under reduced pressure, which was followed by redissolution in ethyl acetate. The mixture was partitioned and washed with aqueous citric acid solution and dried over magnesium sulfate.

After filtration, the filtrate was evaporated under reduced pressure to give a brown solid which was recrystallized to obtain 3,5-bis [(3-Boc-amino)propyloxy]benzoic acid methyl ester (compound 26) as a white solid (5.0 g).

### Synthesis of (3,5-bis [(3-Boc-amino)propyloxy]benzoic acid (compound 27))

Compound 26 (3.0 g, 6.2 mmol) was completely dissolved in 42 mL of THF and 15 mL of MeOH. Then, 6 mL of 4N NaOH was added, and the mixture was stirred at 30°C for 5 hr.

After completion of the reaction, the reaction solvent was evaporated under reduced pressure, which was followed by redissolution in aqueous citric acid solution. The mixture was extracted with dichloromethane, partitioned and washed with saturated brine, and dried over magnesium sulfate.

After filtration, the filtrate was evaporated under reduced pressure to obtain 3,5-bis[(3-Boc-amino)propyloxy]benzoic acid (compound 27) as a white solid (2.8 g).

### Synthesis of (AZ-EG₃-Ph-DiBocPr, compound 28)

Compound 27 (1.1 g, 2.3 mmol) and N-hydroxysuccinimide (0.4 g, 3.4 mmol) were completely dissolved in 8 mL of THF and 15 mL of dichloromethane. Then, 1-(3-dimethylaminopropyl)-3-ethylcarbodiimide hydrochloride (0.7 g, 3.4 mmol) was added, and the mixture was stirred at 30°C for 48 hr.

Successively, 11-azido-3,6,9-trioxaundecane-1-amine (0.6 g, 2.5 mmol) and triethylamine (480 µL, 3.4 mmol) were added, and the mixture was stirred for 24 hr.

After completion of the reaction, the mixture was evaporated under reduced pressure to give a brown viscous substance which was purified by a column to obtain AZ-EG₃-Ph-DiBocPr (compound 28) as a colorless transparent viscous oil (1.1 g).

### Synthesis of (AZ-EG₃-Ph-DiPA, compound 29)

Compound 28 (1.1 g, 1.6 mmol) was completely dissolved in 6 mL of ethyl acetate and 6 mL of EtOH. Then, 12 mL of 4N HCl ethyl acetate solution was added, and the mixture was stirred at room temperature for 4 hr.

After completion of the reaction, the mixture was evaporated under reduced pressure to give AZ-EG₃-Ph-DiPA·HCl as a white solid 0.8 g.

The obtained hydrochloride was dissolved in methanol, desalted with anion exchange resin, and evaporated under reduced pressure to obtain AZ-EG₃-Ph-DiPA (compound 29) as a white solid (0.5 g).

¹H NMR(deuterated chloroform + deuterated dimethyl sulfoxide 400MHz)δ 7.20-7.30(m,1H),6.85-7.00(s,2H),6.50-6.60(s,1H),4.10-4.25(t,4H),3.50-3.75(m,14H),3.10-3.20(t,2H),2.80-3.00(t,4H),1.80-2.05(m,4H).

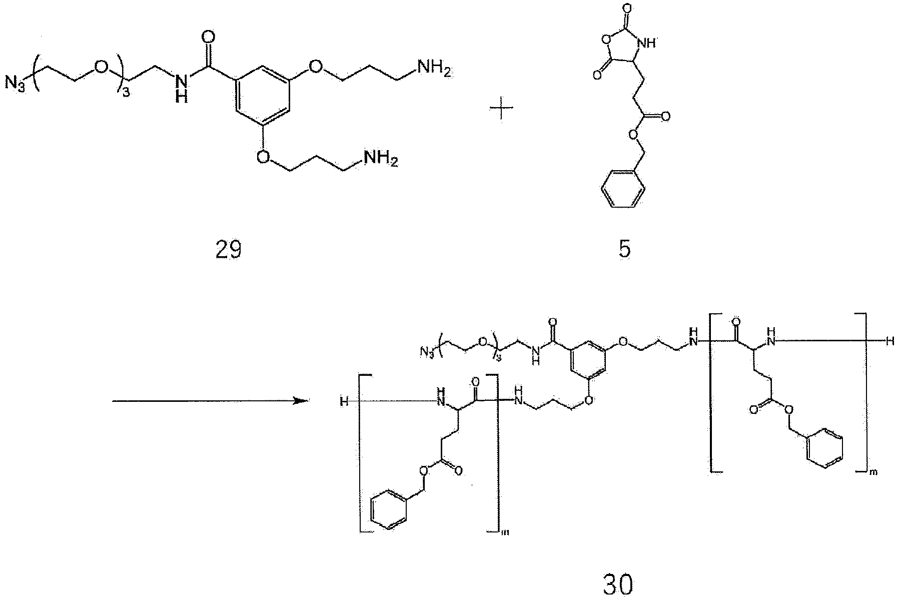

### Synthesis of (AZ-Ph-(pBLG30)2 (compound 30))

By an operation similar to that in Production Example 2 except that 6.0 g of compound 5 and 178 mg of compound 29 were used, AZ-Ph-(pBLG30)2 (compound 30) was obtained as a white powder (4.5 g).

By a method similar to that in Production Example 2, the structure was confirmed by ¹H-NMR analysis, and it was confirmed to be AZ-Ph-(pBLG30)2.

¹H NMR(deuterated DMSO 400MHz)δ 6.80-7.60(m, degree of polymerization×5H+3H),6.50-6.6(s,1H),4.80-5.30(m, degree of polymerization×2H),3.75-4.40(m, degree of polymerization×1H+4H),3.50-3.75(m,14H),3.00-3.50(m,6H),1.55-2.70(m, degree of polymerization×4H+4H).

In addition, the molecular weight was calculated by size-exclusion chromatography (SEC) analysis, and it was confirmed to be Mn=11,500, Mw=16,600, PDI=1.44.

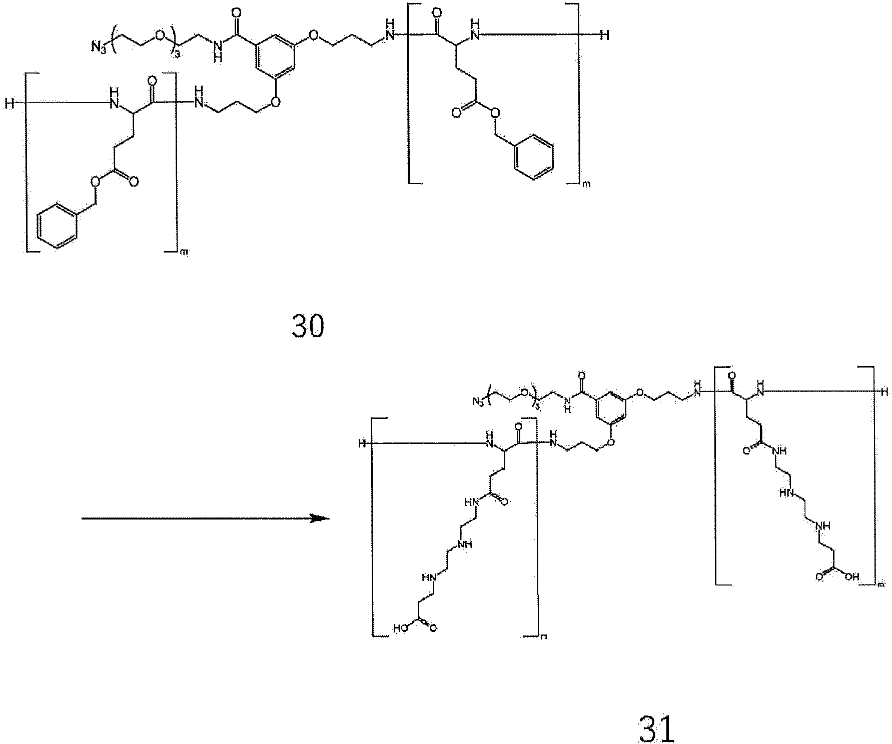

### Synthesis of (AZ-Ph-(pGlu(DET-C2-Car)30)2(compound 31))

By an operation similar to that in Production Example 2 except that AZ-Ph-(pBLG30)2 (500 mg, compound 30) was used instead of compound 6, AZ-Ph-(pGlu(DET-C2-Car)30)2 (compound 31) was obtained as a white powder (220 mg).

By a method similar to that in Production Example 2, the structure was confirmed by ¹H-NMR analysis, and it was confirmed to be AZ-Ph-(pGlu(DET-C2-Car)30)2 from the shift value thereof.

¹H NMR(deuterium chloride +deuterium oxide 400MHz)δ 6.80-7.10(m,2H),6.5-6.65(m,1H),4.30-4.50(m, degree of polymerization×1H),4.10-4.30(m,4H),3.65-3.80(m,14H),3.20-3.65(m, degree of polymerization×10H+8H),2.85-3.00(t, degree of polymerization×2H),2.35-2.55(m, degree of polymerization×2H),1.90-2.25(m, d, degree of polymerization×2H+4H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 29.

In addition, the molecular weight was calculated by SEC analysis, and it was confirmed to be
Mn=13,6000,Mw=17,000,PDI=1.24 at pH 7.4.

### [Production Example 10] Synthesis of terminal acetylated AZ-pGlu(DET-C2-Car)30 (compound 56, m=32)

### Synthesis of (terminal acetylated AZ-pBLG30 (compound 55))

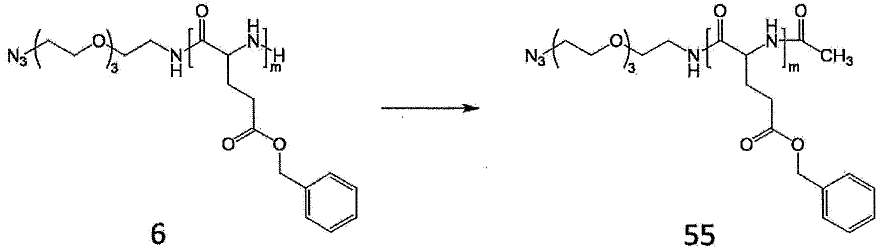

AZ-pBLG30 (50 mg, 6.91 µmol, compound 6) synthesized in Production Example 3 and acetic anhydride (3.5 mg, 0.035 mmol, 5-fold amount relative to number of moles of polymer), dimethylaminopyridine (0.04 mg, 0.35 µmol, 0.05-fold amount relative to number of moles of polymer) were uniformly dissolved in 0.5 mL of dichloromethane, and the mixture was reacted at room temperature for 16 hr. The completion of the reaction was confirmed by the disappearance of ninhydrin color development derived from starting materials by TLC spot.

After completion of the reaction, reprecipitation and purification was performed using THF and ethanol, and the mixture was dried to obtain terminal acetylated AZ-pBLG30 as a white powder (37 mg).

### Synthesis of (terminal acetylated AZ-pGlu(DET-C2-Car)30 (compound 56))

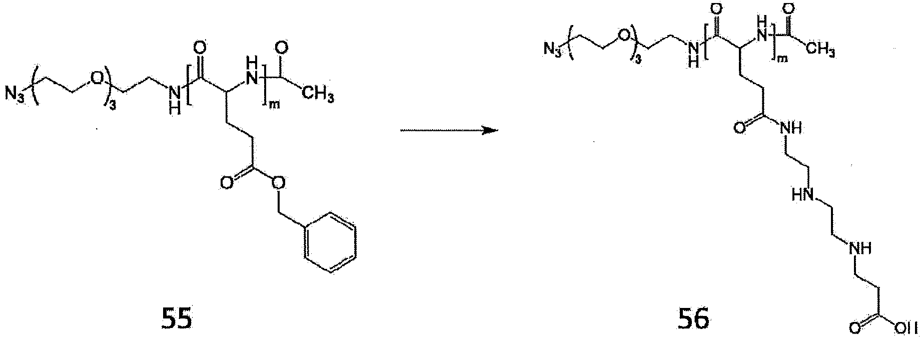

By an operation similar to that in Production Example 2 except that terminal acetylated AZ-pBLG30 (30 mg, number of moles of benzyl group in polymer 0.14 mmol, compound 55) was used instead of AZ-pBLG20 (compound 6), terminal acetylated AZ-pGlu(DET-C2-Car)30 (compound 56) was obtained as a white yellow powder (32 mg).

By a method similar to that in Production Example 2, the structure was confirmed by ¹H-NMR analysis, and it was confirmed to be terminal acetylated AZ-pGlu(DET-C2-Car)30.

¹H NMR(deuterium chloride +deuterium oxide 400MHz):δ 4.30-4.50(m, degree of polymerization×1H),3.65-3.80(m,14H),3.20-3.65(m, degree of polymerization×10H+2H),2.85-3.00(t, degree of polymerization×2H),2.35-2.55(m, degree of polymerization×2H),1.90-2.25(m, d, degree of polymerization×2H+3H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 32.

### [Comparative Example 1-1] Synthesis of AZ-pGlu(DET-C4-Car)100 (compound 35, m=98)

### Synthesis of (N-(2-(2-aminoethylamino)ethyl)-5-aminopentanoic acid t-butyl ester (DET-C4-Car) tBu (compound 34))

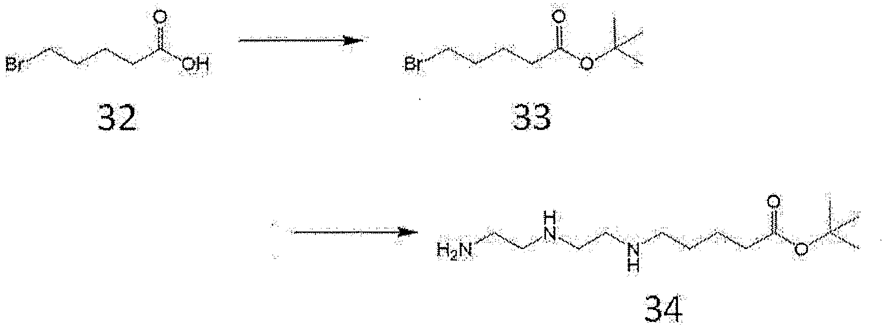

By an operation similar to that in Production Example 5 except that 5-bromopentanoic acid (10.0 g, 55.2 mmol, compound 32) was used instead of compound 8, 5-bromopentanoic acid t-butyl ester (compound 33) was obtained as a colorless transparent oil (11.8 g).

¹H NMR(CDCl₃, 400MHz)δ
3.42(t,2H),2.26(t,2H),1.89(m,2H),1.74(m,2H),1.45(s,9H).

By an operation similar to that in Production Example 5 except that 5-bromopentanoic acid t-butyl ester (10.0 g, 42.1 mmol, compound 33) was used instead of compound 9, DET-C4-Car tBu (compound 34) was obtained as a white solid (3.8 g).

¹H NMR(D₂O, 400MHz) 5 3.41-3.59(m,8H),3.16(t,2H),2.45(t,2H),1.65-1.81(m,4H),1.45(s,9H).
ESI-MS:calcd for C₁₃H₃₀N₃O₂⁺[M+H⁺]:260.2332; found:260.2297

### Synthesis of (AZ-pGlu(DET-C4-Car)100 (compound 35, m=98))

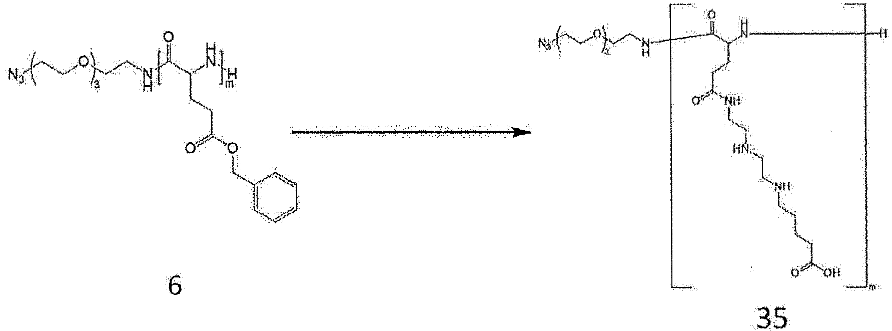

By an operation similar to that in Production Example 2 except that DET-C4-Car tBu (901 mg, 3.5 mmol, compound 34) was used instead of DET-C2-Car tBu (compound 3), AZ-pGlu(DET-C4-Car)100 was obtained as a white yellow powder (53.6 mg).

By a method similar to that in Production Example 2, the structure was confirmed by ¹H-NMR analysis, and it was confirmed to be AZ-pGlu(DET-C4-Car)100.
¹H NMR(deuterium oxide 400MHz)δ 4.30-4.50(b, degree of polymerization×1H),3.65-3.80(m,14H),3.20-3.65(m, degree of polymerization×10H+2H),2.42(b, degree of polymerization×2H),2.31(t, degree of polymerization×2H),1.90-2.20(b, d, degree of polymerization×2H),1.65(q, degree of polymerization×2H),1.55(q, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=18,500, Mw=20,600, PDI=1.11 at pH 7.4.

### [Comparative Example 1-2] Synthesis of AZ-pGlu(DET-C4-Sul)100 (compound 41, m=98)

### Synthesis of (N-(2-(2-aminoethylamino)ethyl)-4-aminobutanesulfonic acid 2,2,2-trifluoroethyl ester (DET-C4-Sul) TFE (compound 40))

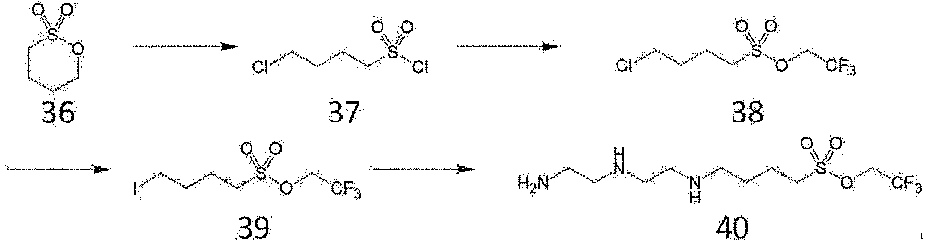

11.3 mL of thionyl chloride (11.3 mL, 155.8 mmol) was dissolved in 2 mL of DMF. Then, to the obtained mixed solution was added dropwise 1,4-butanesultone (15.0 g, 110.2 mmol, compound 36), and the mixture was stirred under an argon atmosphere at 70°C for 3 days. Thionyl chloride (3 mL, 41.4 mmol) was added to the reaction mixture and the mixture was stirred at 70°C for additional 3 days.

After completion of the reaction, toluene was added and the mixture was evaporated under reduced pressure. This step was repeated twice, and the obtained residue was vacuum dried to give 4-chlorobutanesulfonic acid chloride (compound 37) (17.9 g).

¹H NMR(CDCl₃, 400MHz)δ 3.75(t,2H),3.61(t,2H),2.19-2.29(m,2H),1.96-2.05 (m, 2H) .

By an operation similar to that in Production Example 7 except that 4-chlorobutanesulfonic acid chloride (15.0 g, 78.5 mmol, compound 37) was used instead of compound 16, 4-chlorobutanesulfonic acid 2,2,2-trifluoroethyl ester (compound 38) (16.3 g) was obtained.

¹H NMR(CDCl₃, 400MHz)δ 4.53(q,2H),3.58(t,2H),3.26(t,2H),2.09(q,2H),1.96(q,2H).

By an operation similar to that in Production Example 7 except that 4-chlorobutanesulfonic acid 2,2,2-trifluoroethyl ester (15.0 g, 58.9 mmol, compound 38) was used instead of compound 17, 4-iodo butanesulfonic acid 2,2,2-trifluoroethyl ester (compound 39) (17.3 g) was obtained.

¹H NMR(CDCl₃, 400MHz)δ 4.53(q,2H),3.26(t,2H),3.19(t,2H),1.92-2.09(m,2H).

By an operation similar to that in Production Example 7 except that 4-iodo butanesulfonic acid 2,2,2-trifluoroethyl ester (16.0 g, 46.2 mmol, compound 39) was used instead of compound 18, DET-C4-Sul TFE (compound 40) was obtained as a white solid (7.0 g).

¹H NMR(D₂O, 400MHz)δ 4.81(q,2H),3.56(t,2H),3.12-3.31(m,10H),1.84-2.02(m,4H).
ESI-MS:calcd for C₈H₂₀N₃O₃S⁺[M+H⁺] :322.1497; found:322.1463.

### Synthesis of (AZ-pGlu(DET-C4-Sul)100 (compound 41, m=98))

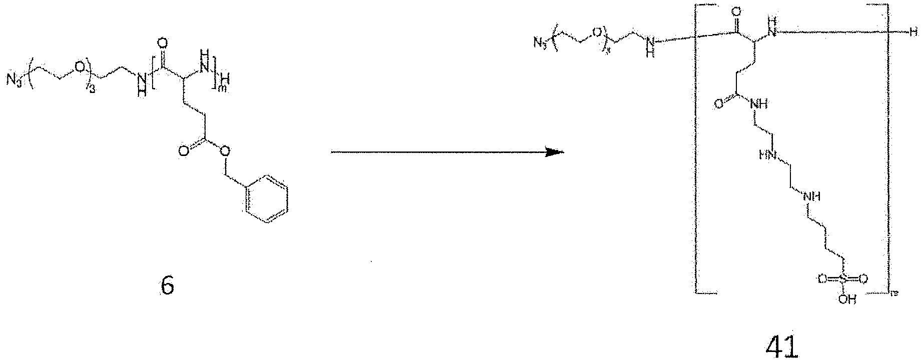

By an operation similar to that in Production Example 6 except that DET-C4-Sul TFE (1.12 g, 3.5 mmol, compound 40) was used instead of DET-C2-Sul TFE (compound 14), AZ-pGlu(DET-C4-Sul)100 (compound 41) was obtained as a white yellow powder (51.3 mg).

By a method similar to that in Production Example 2, the structure was confirmed by ¹H-NMR analysis, and it was confirmed to be AZ-pGlu(DET-C4-Sul)100.

¹H NMR(deuterium oxide 400MHz)δ 4.30-4.50(b, degree of polymerization×1H),3.65-3.80(m,14H),3.20-3.65(m, degree of polymerization×10H+2H),2.92(t, degree of polymerization×2H),2.42(b, degree of polymerization×2H),1.70-2.30((b, m)+q, degree of polymerization×4H),1.60-1.70(q, degree of polymerization×2H).

With a peak (3.65-3.80 ppm) derived from the initiator as the standard value (14H), the degree of polymerization was calculated from the integration value of a peak (4.30-4.50 ppm) derived from the amide group α-position of polypeptide, and the degree of polymerization was confirmed to correspond to 98.

In addition, the molecular weight was calculated by SEC analysis, and it was confirmed to be Mn=21,700, Mw=23,900, PDI=1.10 at pH 7.4.

### [Example 1-1] Synthesis of DSGE-pGlu(DET-C2-Car)30 (compound 44, m=31)

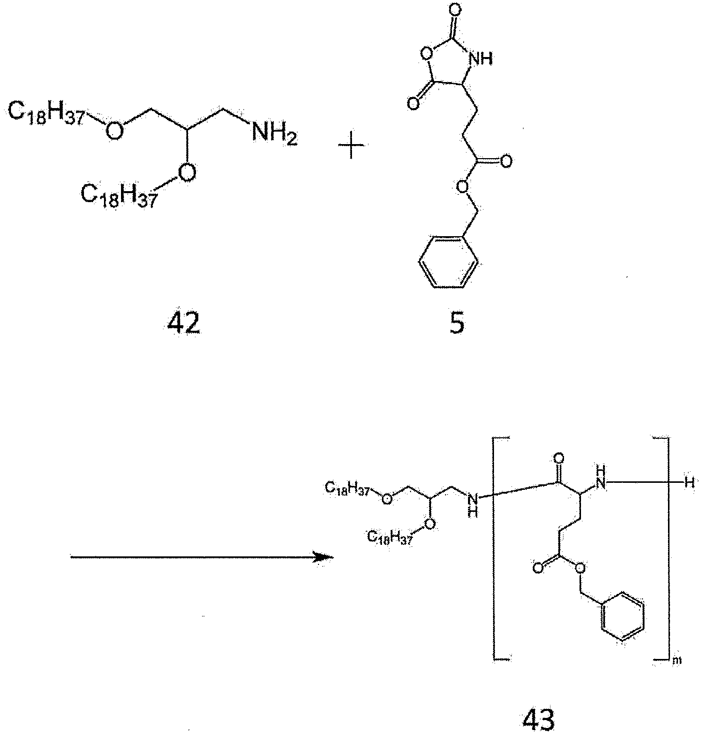

### Synthesis of (DSGE-pBLG30 (compound 43))

By an operation similar to that in Production Example 2 except that 1.43 g of compound 5 was used and DSGE-amine (100 mg, 0.17 mmol, compound 42) was used instead of compound 4, DSGE-pBLG30(compound 43) was obtained as a white powder (0.9 g).

By a method similar to that in Production Example 2 except that the measurement solvent was deuterated chloroform, the structure was confirmed by ¹H-NMR analysis, and it was confirmed to be DSGE-pBLG30.

¹H NMR (deuterated chloroform 400MHz)δ 7.10-7.50(m, degree of polymerization×5H),4.80-5.30(m, degree of polymerization×2H),3.80-4.30(m, degree of polymerization×1H),3.20-3.80(m,9H),1.90-2.90(m, degree of polymerization×4H),1.25(b,64H),0.88(t,6H).

With a peak (0.88 ppm) derived from a methyl group at the lipid terminal as the standard value (6H), the degree of polymerization was calculated from the integration value of a peak (3.80-4.30 ppm) derived from the amide group α-position of polypeptide, and the degree of polymerization was confirmed to correspond to 31.

In addition, the molecular weight was calculated by SEC analysis, and it was confirmed to be Mn=9,800, Mw=12,900, PDI=1.32.

### Synthesis of (DSGE-pGlu(DET-C2-Car)30 (compound 44, m=31))

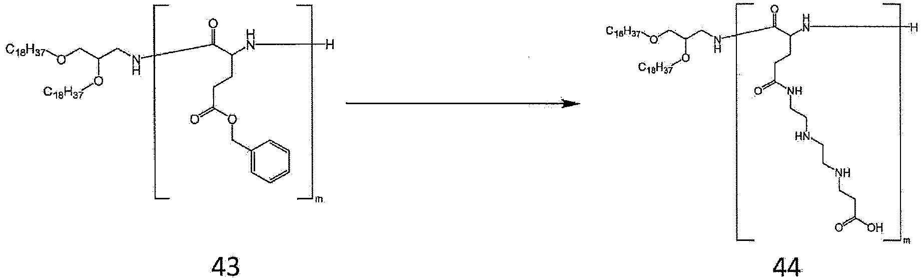

Successively, by an operation similar to that in Production Example 2 except that DSGE-pBLG30 (0.9 g, compound 43) was used instead of compound 6, and the deprotection reaction using 6N hydrochloric acid solution was carried out at 50°C, DSGE-pGlu(DET-C2-Car)30 (compound 44) was obtained as a white-yellow powder (0.8 g).

By a method similar to that in Production Example 2, the structure was confirmed by ¹H-NMR analysis, and it was confirmed to be DSGE-pGlu(DET-C2-Car)30.

¹H NMR (aqueous deuterium chloride 400MHz)δ 4.30-4.50(b, degree of polymerization×1H),3.20-3.90(m, degree of polymerization×10H+9H),2.90(b, degree of polymerization×2H),2.45(b, degree of polymerization×2H),1.80-2.25(m, degree of polymerization×2H),1.20-1.40(b,64H),0.80-0.95 (b,6H).

With a peak (0.80-0.95 ppm) derived from the methyl group at the lipid terminal as the standard value (6H), the degree of polymerization was calculated from the integration value of a peak (4.30-4.50 ppm) derived from the amide group α-position of polypeptide, and the degree of polymerization was confirmed to correspond to 31.

### [Example 1-2] Synthesis of DSGE-pGlu(DET-C2-Car)100 (compound 44, m=98)

### Synthesis of (DSGE-pBLG100 (compound 43))

By an operation similar to that in Production Example 2 except that 2.86 g of compound 5 was used, DSGE-amine (60 mg, 0.10 mmol, compound 42) was used instead of compound 4, and the deprotection reaction using 6N hydrochloric acid solution was carried out at 50°C, DSGE-pBLG100 (compound 43) was obtained as a white powder (2.1 g).

By a method similar to that in Example 1-1, the structure was confirmed by ¹H-NMR analysis, and it was confirmed to be DSGE-pBZG100.

¹H NMR (deuterated chloroform 400MHz)δ 7.10-7.50(m, degree of polymerization×5H),4.80-5.30(m, degree of polymerization×2H),3.80-4.30(m, degree of polymerization×1H),3.20-3.80(m,9H),1.90-2.90(m, degree of polymerization×4H),1.25(b,64H),0.88(t,6H).

With a peak (0.88 ppm) derived from the methyl group at the lipid terminal as the standard value (6H), the degree of polymerization was calculated from the integration value of a peak (3.80-4.30 ppm) derived from the amide group α-position of polypeptide, and the degree of polymerization was confirmed to correspond to 98.

In addition, the molecular weight was calculated by SEC analysis, and it was confirmed to be Mn=15,200, Mw=18,600, PDI=1.22.

### Synthesis of (DSGE-pGlu(DET-C2-Car)100 (compound 44, m=98))

Successively, by an operation similar to that in Example 1-1 except that DSGE-pBLG100 (1.0 g, compound 43) was used instead of DSGE-pBLG30, DSGE-pGlu(DET-C2-Car)100(compound 44) was obtained as a white-yellow powder (1.30 g).

By a method similar to that in Production Example 2, the structure was confirmed by ¹H-NMR analysis, and it was confirmed to be DSGE-pGlu(DET-C2-Car)100.

¹H NMR (aqueous deuterium chloride 400MHz)δ 4.30-4.50(b, degree of polymerization×1H),3.20-3.90(m, degree of polymerization×10H+9H),2.90(b, degree of polymerization×2H),2.45(b, degree of polymerization×2H),1.80-2.25(m, degree of polymerization×2H),1.20-1.40(b,64H),0.80-0.95(b,6H).

With a peak (0.80-0.95 ppm) derived from the methyl group at the lipid terminal as the standard value (6H), the degree of polymerization was calculated from the integration value of a peak (4.30-4.50 ppm) derived from the amide group α-position of polypeptide, and the degree of polymerization was confirmed to correspond to 98.

### [Example 1-3] Synthesis of DSPE-DBCO-AZ-pGlu(DET-C2-Car)20 (compound 46, m=22)

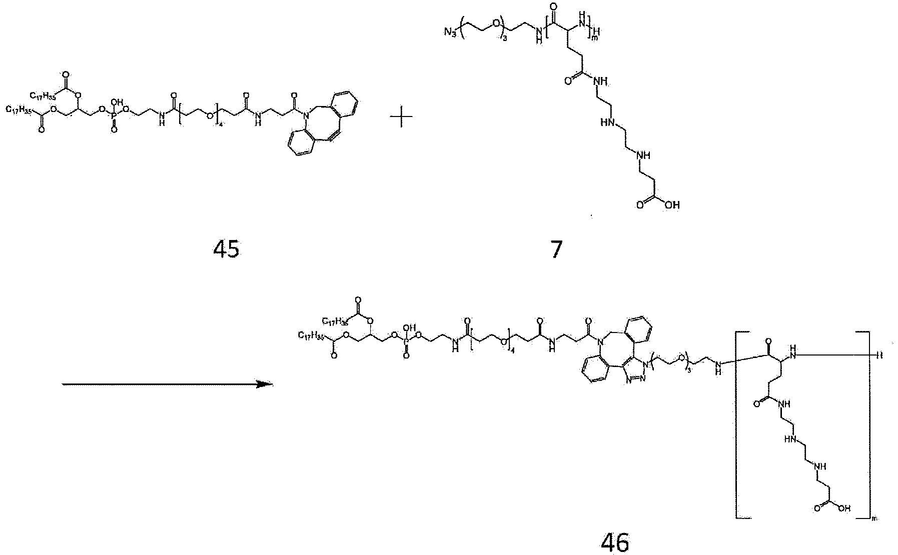

AZ-pGlu(DET-C2-Car)20 (396 mg, 63 µmol, compound 7, m=22) synthesized in Production Example 2 was uniformly dissolved in 19.8 mL of ion exchange water (50-fold amount relative to polymer weight). Separately, DSPE-EG₄-DBCO (241 mg, 188 µmol, 3-fold amount relative to number of moles of polymer, compound 45) was uniformly dissolved in 19.8 mL of dichloromethane (50-fold amount relative to polymer weight) and added to the aqueous solution. This two-layer reaction solution was stirred under shading at room temperature for 2 days.

After completion of the reaction, the obtained reaction solution was added dropwise to 800 mL of acetonitrile to obtain a white precipitate. The supernatant was discarded, the precipitate was washed twice with acetonitrile and vacuum dried at room temperature to obtain DSPE-DBCO-AZ-pGlu(DET-C2-Car)20 (compound 46) as a white yellow powder (626 mg).

Trimethylsilyl propionic acid (TMSP) was added to prepare a deuterated solvent added with 20% deuterium chloride (20 mg) and THF (0.4 mL) per deuterium oxide 0.6 mL. The above-mentioned deuterated solvent (0.6 mL) was added to the target product (5 mg) to dissolve same. Using the sample, the structure was confirmed by ¹H-NMR analysis, and it was confirmed to be DSPE-DBCO-AZ-pGlu(DET-C2-Car)20 from the shift value thereof.

¹H NMR(40% deuterated THF aqueous deuterium chloride 400MHz)δ 7.20-7.80(m,8H),5.20(d,1H),4.30-4.50(m, degree of polymerization×1H),3.30-4.25(m, degree of polymerization×10H+54H),2.90(b, degree of polymerization×2H),2.00-2.60(m, degree of polymerization×4H),1.28(b,60H),0.85(t,6H).

### [Example 1-4] Synthesis of DSPE-DBCO-AZ-pGlu(DET-C2-Car)30 (compound 46, m=32)

By an operation similar to that in Example 1-3 except that, instead of AZ-pGlu(DET-C2-Car)20, AZ-pGlu(DET-C2-Car)30 (700 mg, 76 µmol, compound 7, m=32) synthesized in Production Example 3 was used, and DSPE-EG₄-DBCO (142 mg, 114 µmol, 1.5-fold amount relative to number of moles of polymer, compound 45) was used, DSPE-DBCO-AZ-pGlu(DET-C2-Car)30 (compound 46) was obtained as a white-yellow powder (781 mg).

By a method similar to that in Example 1-3, the structure was confirmed by ¹H-NMR analysis, and it was confirmed to be DSPE-DBCO-AZ-pGlu(DET-C2-Car)30 from the shift value thereof.

¹H NMR(40% deuterated THF aqueous deuterium chloride 400MHz)δ 7.20-7.80(m,8H),5.20(d,1H),4.30-4.50(m, degree of polymerization×1H),3.30-4.25(m, degree of polymerization×10H+54H),2.90(b, degree of polymerization×2H),2.00-2.60(m, degree of polymerization×4H),1.28(b,60H),0.85(t,6H).

### [Example 1-5] Synthesis of DSPE-alkehyl-HS-pGlu(DET-C2-Car)30 (compound 48, the formula (1), m=33)

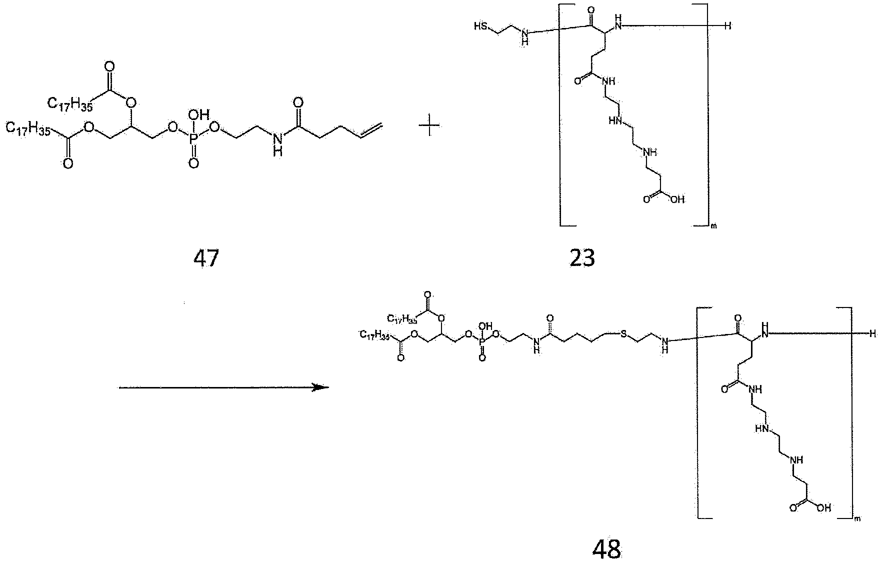

700 mg of SH-pGlu(DET-C2-Car)30 (700 mg, 74 µmol, compound 23, m=33) synthesized in Production Example 8 was uniformly dissolved in 35.0 mL of water. Separately, DSPE-alkenyl (101 mg, 122 µmol, 1.6-fold amount relative to number of moles of polymer, compound 47) was uniformly dissolved in 35.0 mL of dichloromethane and added to the aqueous solution, and the mixture was stirred under nitrogen. To this two-layer solution was added 10.3 mg of ammonium persulfate and the mixture was stirred under nitrogen at room temperature for 2 days.

After completion of the reaction, the reaction solution was added dropwise to 1400 mL of acetonitrile to obtain a white precipitate. The supernatant was discarded, reprecipitation and purification with water and acetonitrile was repeated twice and vacuum drying was performed at room temperature to obtain DSPE-alkenyl-HS-pGlu(DET-C2-Car)30(compound 48) as a white yellow powder (632 mg).

By a method similar to that in Example 1-3, the structure was confirmed by ¹H-NMR analysis, and it was confirmed to be DSPE-alkenyl-HS-pGlu(DET-C2-Car)30 from the shift value thereof.

¹H NMR(40% deuterated THF aqueous deuterium chloride 400MHz)δ 4.30-4.50(m, degree of polymerization×1H),3.30-4.25(m, degree of polymerization×10H+17H),2.65-3.10(m, degree of polymerization×2H+4H),2.00-2.60(m, degree of polymerization×4H),1.55-1.85(q+q,4H),1.28(b,60H),0.85(t,6H).

### [Example 1-6] Synthesis of DPPE-DBCO-AZ-pGlu(DET-C2-Car)20 (compound 50, m=22)

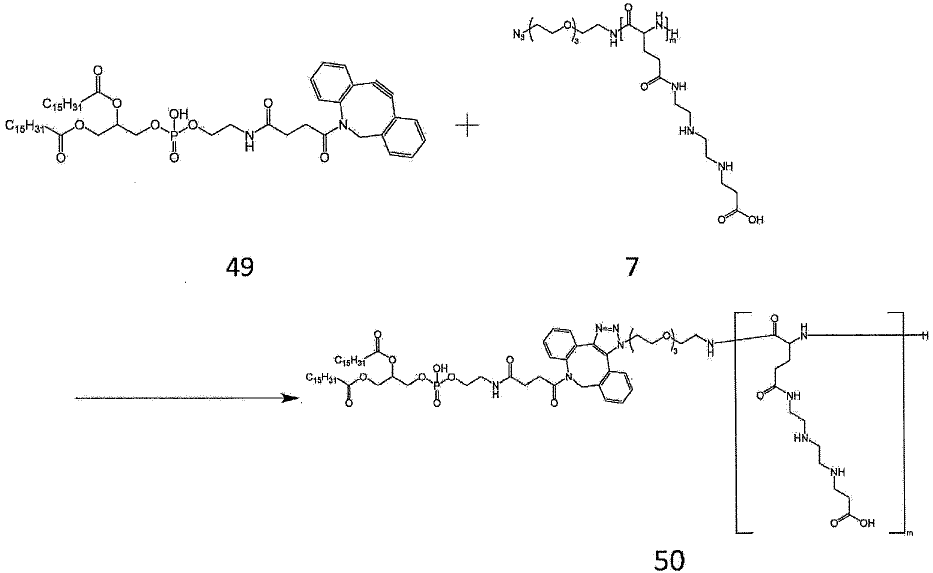

By an operation similar to that in Example 1-3 except that DPPE-DBCO (184 mg, 188 µmol, compound 49) was used instead of DSPE-EG₄-DBCO, DPPE-DBCO-AZ-pGlu(DET-C2-Car)20 (compound 50) was obtained as a white yellow powder (519 mg).

By a method similar to that in Example 1-3, the structure was confirmed by ¹H-NMR analysis, and it was confirmed to be DPPE-DBCO-AZ-pGlu(DET-C2-Car)20 from the shift value thereof.

¹H NMR(40% deuterated THF aqueous deuterium chloride 400MHz)5 7.20-7.80(m,8H),5.20(d,1H),4.30-4.50(m, degree of polymerization×1H),3.30-4.25(m, degree of polymerization×10H+34H),2.90(b, degree of polymerization×2H),2.00-2.60(m, degree of polymerization×4H),1.28(b,52H),0.85(t,6H).

### [Example 1-7] Synthesis of DPPE-DBCO-AZ-pGlu(DET-C2-Car)30 (compound 50, m=32)

By an operation similar to that in Example 1-4 except that DPPE-DBCO (111 mg, 113 µmol, compound 49) was used instead of DSPE-EG₄-DBCO, DPPE-DBCO-AZ-pGlu(DET-C2-Car)30 (compound 50) was obtained as a white yellow powder (699 mg).

By a method similar to that in Example 1-3, the structure was confirmed by ¹H-NMR analysis, and it was confirmed to be DPPE-DBCO-AZ-pGlu(DET-C2-Car)30 from the shift value thereof.

¹H NMR(40% deuterated THF aqueous deuterium chloride 400MHz)5 7.20-7.80(m,8H),5.20(d,1H),4.30-4.50(m, degree of polymerization×1H),3.30-4.25(m, degree of polymerization×10H+34H),2.90(b, degree of polymerization×2H),2.00-2.60(m, degree of polymerization×4H),1.28 (b,52H),0.85(t,6H).

### [Example 1-8] Synthesis of DSGE-DBCO-AZ-pGlu(DET-C2-Car)30 (compound 52, m=32)

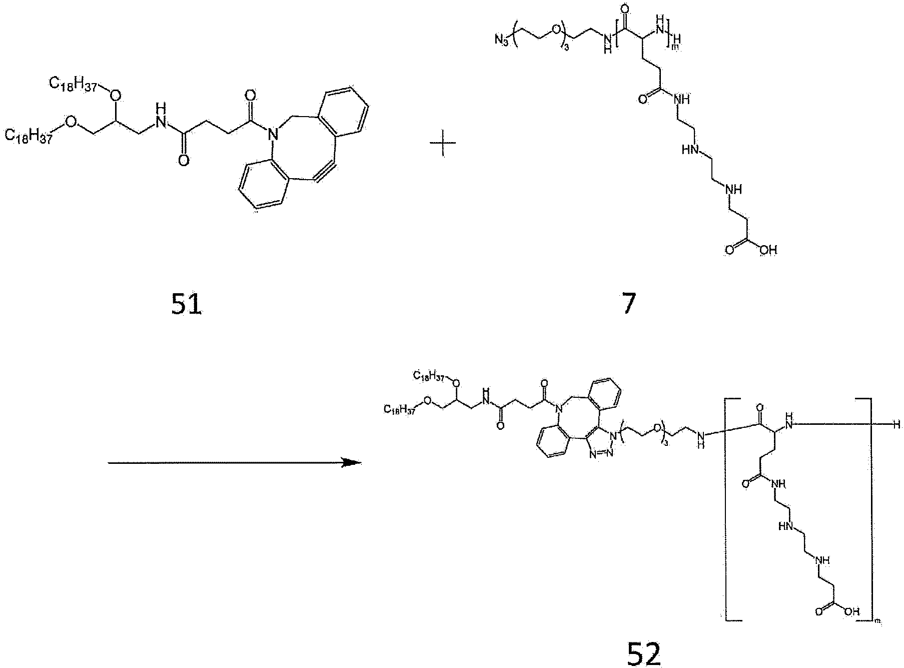

By an operation similar to that in Example 1-4 except that DSGE-DBCO (111 mg, 126 µmol, compound 51) was used instead of DSPE-EG₄-DBCO, DSGE-DBCO-AZ-pGlu(DET-C2-Car)30 (compound 52) was obtained as a white yellow powder (666 mg)

By a method similar to that in Example 1-3, the structure was confirmed by ¹H-NMR analysis, and it was confirmed to be DSGE-DBCO-AZ-pGlu(DET-C2-Car)30 from the shift value thereof.

¹H NMR(40% deuterated THF aqueous deuterium chloride 400MHz)5 7.20-7.80(m,8H),5.20(d,1H),4.30-4.50(b, degree of polymerization×1H),3.10-4.00(m, degree of polymerization×10H+30H),2.90(b, degree of polymerization×2H),2.45(b, degree of polymerization×2H),1.80-2.30(m, degree of polymerization×2H),1.25-1.60(b,64H)0.85(t,6H).

### [Example 1-9] Synthesis of DSPE-DBCO-AZ-pGlu(DET-C2-Car)20 (compound 46, m=22)

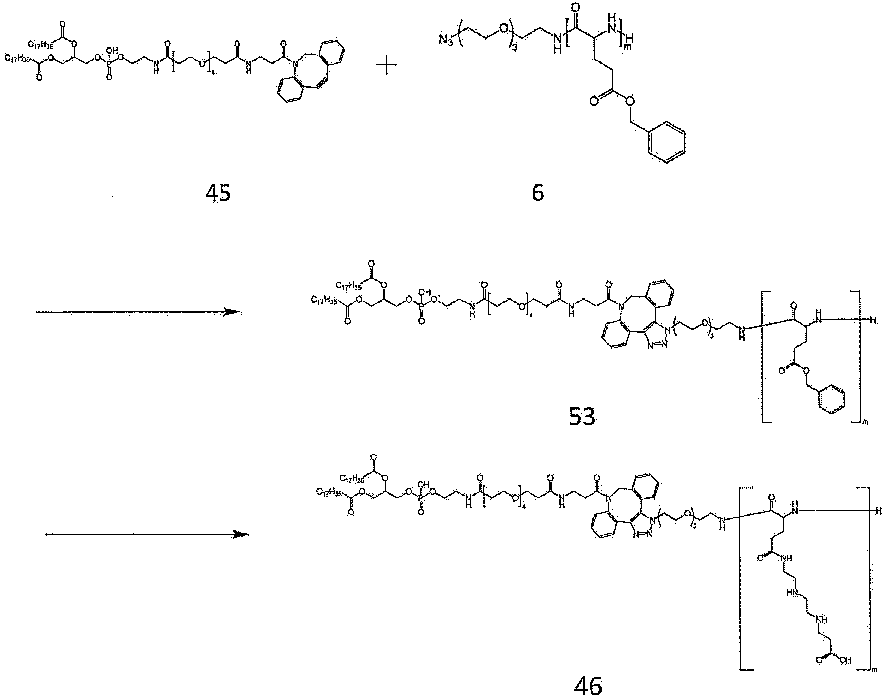

AZ-pBLG20 (305 mg, 63 µmol, compound 6, m=22) synthesized in Production Example 2 and DSPE-EG₄-DBCO (241 mg, 188 µmol, 3-fold amount relative to number of moles of polymer, compound 45) were uniformly dissolved in 30.5 mL of DCM (100-fold amount relative to relative to polymer weight), and the mixture was stirred at room temperature for 2 days.

After completion of the reaction, DCM was evaporated under reduced pressure, the residue was dissolved in 2.1 mL of DMF (7.0-fold amount relative to polymer weight), and 2.1 mL of methanol (7.0-fold amount relative to polymer weight) and 4.2 mL of ion exchange water (14.0-fold amount relative to polymer weight) to precipitate a white solid. The obtained solid was collected by filtration, washed with EtOH and dried under reduced pressure to obtain DSPE-DBCO-AZ-pBLG20 (compound 53) as a white yellow powder (362 mg).

Successively, by an operation similar to that in Production Example 2, DSPE-DBCO-AZ-pGlu(DET-C2-Car)20 (compound 46) was obtained as a white yellow powder (420 mg).

By a method similar to that in Example 1 -3, the structure was confirmed by ¹H-NMR analysis, and it was confirmed to be DSPE-DBCO-AZ-pGlu(DET-C2-Car)20.

¹H NMR(40% deuterated THF aqueous deuterium chloride 400MHz)5 7.20-7.80(m,8H),5.20(d,1H),4.30-4.50(m, degree of polymerization×1H),3.30-4.25(m, degree of polymerization×10H+54H),2.90(b, degree of polymerization×2H),2.00-2.60(m, degree of polymerization×4H),1.28(b,60H),0.85(t,6H).

### [Example 1-10] Synthesis of DSPE-DBCO-AZ-Ph-(pGlu(DET-C2-Car)30)2 (compound 54, m=29, n=2)

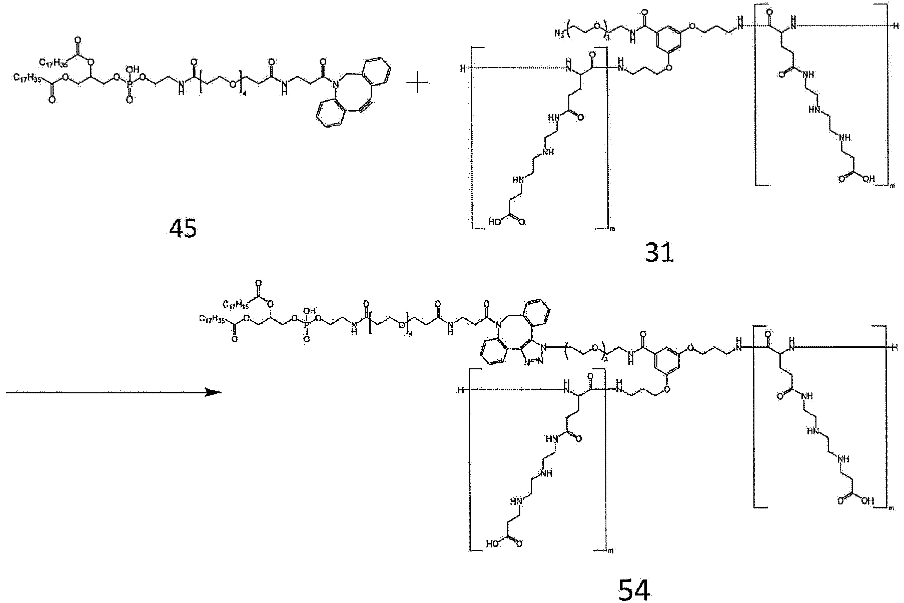

By an operation similar to that in Example 1-3 except that AZ-Ph-(pGlu(DET-C2-Car)30)2 (1.0 g, 63 µmol, compound 31, m=29) synthesized in Production Example 9 was used, DSPE-DBCO-AZ-Ph-(pGlu(DET-C2-Car)30)2 (compound 54) was obtained as a white yellow powder (0.99 g).

By a method similar to that in Example 1-3, the structure was confirmed by ¹H-NMR analysis, and it was confirmed to be DSPE-DBCO-AZ-Ph-(pGlu(DET-C2-Car)30)2 from the shift value thereof.

¹H NMR(40% deuterated THF aqueous deuterium chloride 400MHz)5 7.20-7.80(m,11H),5.20(d,1H),4.30-4.50(m, degree of polymerization×1H),3.30-4.25(m, degree of polymerization×10H+58H),2.90(b, degree of polymerization×2H+4H),2.00-2.60(m, degree of polymerization×4H+4H),1.28(b,60H),0.85(t,6H).

### [Example 1-11] Synthesis of terminal acetylated DSPE-DBCO-AZ-pGlu(DET-C2-Car)30 (compound 57, m=32)

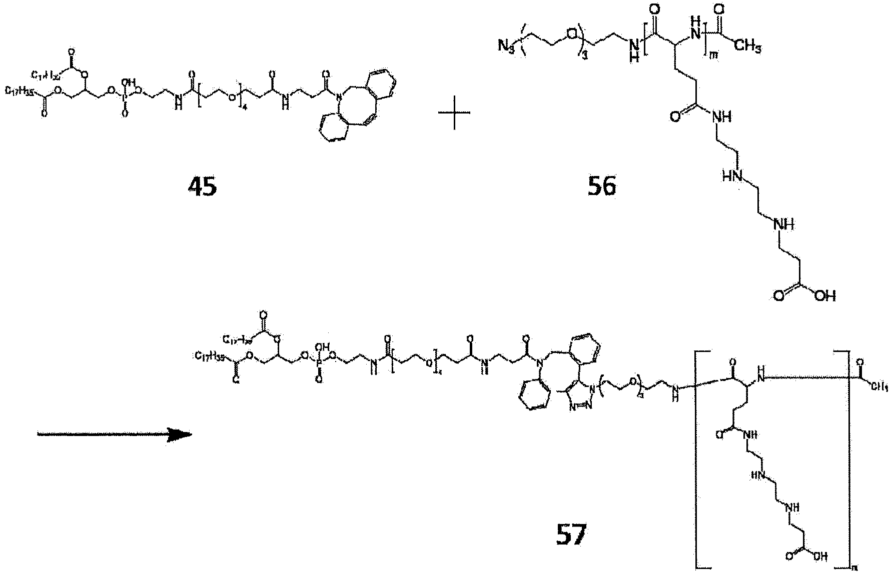

By an operation similar to that in Example 1-3 except that terminal acetylated AZ-pGlu (DET-C2-Car) 30 (30 mg, 2.8 µmol, compound 56, m=32) synthesized in Production Example 10 was used instead of AZ-pGlu(DET-C2-Car)20, terminal acetylated DSPE-DBCO-AZ-pGlu(DET-C2-Car)30 (compound 57) was obtained as a white yellow powder (27 mg).

By a method similar to that in Example 1-3, the structure was confirmed by ¹H-NMR analysis, and it was confirmed to be terminal acetylated DSPE-DBCO-AZ-pGlu(DET-C2-Car)30 from the shift value thereof.

¹H NMR(40% deuterated THF aqueous deuterium chloride 400MHz)δ 7.20-7.80(m,8H),5.20(d,1H),4.30-4.50(m, degree of polymerization×1H),3.30-4.25(m, degree of polymerization×10H+54H),2.90(b, degree of polymerization×2H),2.00-2.60(m, degree of polymerization×4H+3H),1.28(b,60H),0.85(t,6H).

### [Example 1-12] Synthesis of terminal acetylated DSGE-pGlu(DET-C2-Car)30 (compound 59, m=31)

### Synthesis of (terminal acetylated DSGE-pBLG30 (compound 58, m=31))

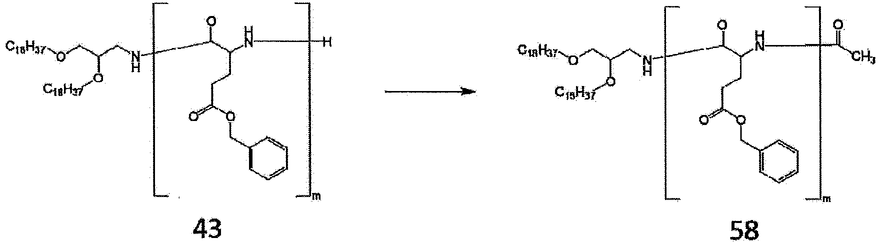

By an operation similar to that in Production Example 10 except that DSGE-pBLG30 (0.3 g, 39 µmol, compound 43, m=31) obtained in Example 1-1 was used instead of AZ-pBLG30 (compound 6), terminal acetylated DSGE-pBLG30 (compound 58) was obtained as a white powder (0.24 g).

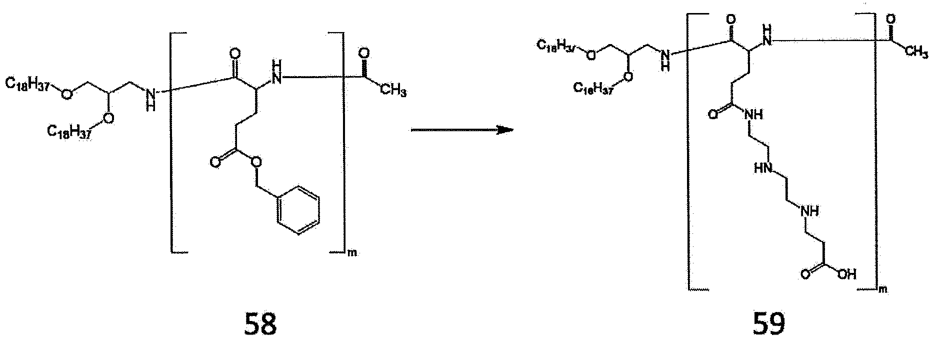

Successively, by an operation similar to that in Production Example 2 except that terminal acetylated DSGE-pBLG30 (0.21 g, 27 µmol, compound 58) was used instead of compound 6, and the deprotection reaction using 6N hydrochloric acid solution was carried out at 50°C, terminal acetylated DSGE-pGlu(DET-C2-Car)30 (compound 59) was obtained as a whiteyellow powder (0.25 g).

By a method similar to that in Production Example 2, the structure was confirmed by ¹H-NMR analysis, and it was confirmed to be terminal acetylated DSGE-pGlu(DET-C2-Car)30.

¹H NMR (aqueous deuterium chloride 400MHz)5 4.30-4.50(b, degree of polymerization×1H),3.20-3.90(m, degree of polymerization×10H+9H),2.90(b, degree of polymerization×2H),2.45(b, degree of polymerization×2H),1.80-2.25(m, degree of polymerization×2H+3H),1.20-1.40(b,64H),0.80-0.95(b,6H).

With a peak (0.80-0.95 ppm) derived from the methyl group at the lipid terminal as the standard value (6H), the degree of polymerization was calculated from the integration value of a peak (4.30-4.50 ppm) derived from the amide group α-position of polypeptide, and the degree of polymerization was confirmed to correspond to 31.

### [Example 2-1] Preparation of solid lipid nanoparticles using DSPE-DBCO-AZ-pGlu(DET-C2-Car)20

First, using 90% t-BuOH aqueous solution, 5 mM cholesterol solution, 5 mM DOTAP solution, and 5 mM DOPE solution were respectively prepared.

Then, the above-mentioned cholesterol solution (45 µL), DOTAP solution (52.5 µL), DOPE solution (52.5 µL), and 90%t-BuOH aqueous solution (50 µL) were added to 1.5 mL tubes. Furthermore, separately-prepared 1 mg/ml siRNA (siGL3, sense: 5'-CUUACGCUGAGUACUUCGAdTdT-3' (SEQ ID NO: 1), antisense: 5'-UCGAAGUACUCAGCGUAAGdTdT-3' (SEQ ID NO: 2), manufactured by Hokkaido System Science Co., Ltd.) aqueous solution (20 µL), 1.2 mM polymer aqueous solution (20 µL) of Example 1-3, and pure water (10 µL) were mixed.

Each prepared mixed solution was mixed by stirring, and the obtained solution was added dropwise to 20 mM HEPES buffer (pH 7.4, 2 mL) with stirring to form solid lipid nanoparticles.

The obtained solid lipid nanoparticles were purified by ultrafiltration using PBS buffer (manufactured by Merck Co., Ltd., Amicon Ultra-15 (MWCO 50kDa)).

### [Example 2-2] Preparation of solid lipid nanoparticles using DSPE-DBCO-AZ-pGlu(DET-C2-Car)20

By a method similar to that in Example 2-1 except that 2.0 mM polymer aqueous solution (20 µL) of Example 1-3 was used, solid lipid nanoparticles were prepared.

### [Example 2-3] Preparation of solid lipid nanoparticles using DSPE-DBCO-AZ-pGlu(DET-C2-Car)30

By a method similar to that in Example 2-1 except that 1.0 mM polymer aqueous solution (20 µL) of Example 1-4 was used instead of 1.0 mM polymer aqueous solution of Example 1-3, solid lipid nanoparticles were prepared.

### [Example 2-4] Preparation of solid lipid nanoparticles using DSPE-DBCO-AZ-pGlu(DET-C2-Car)30

By a method similar to that in Example 2-3 except that 2.0 mM polymer aqueous solution (20 µL) of Example 1-4 was used, solid lipid nanoparticles were prepared.

### [Example 2-5] Preparation of solid lipid nanoparticles using DSPE-DBCO-AZ-pGlu(DET-C2-Car)30

By a method similar to that in Example 2-3 except that 3.0 mM polymer aqueous solution (20 µL) of Example 1-4 was used, solid lipid nanoparticles were prepared.

### [Example 2-6] Preparation of solid lipid nanoparticles using DSPE-DBCO-AZ-pGlu(DET-C2-Car)30

By a method similar to that in Example 2-3 except that 4.0 mM polymer aqueous solution (20 µL) of Example 1-4 was used, solid lipid nanoparticles were prepared.

### [Example 2-7] Preparation of solid lipid nanoparticles using DSPE-DBCO-AZ-pGlu(DET-C2-Car)30

By a method similar to that in Example 2-3 except that siRNA to be used was changed to Alexa647-labeled siRNA (Alexa647-siGL3, sense: 5'-CUUACGCUGAGUACUUCGAdTdT-3' (SEQ ID NO: 3), antisense: 5'-Alexa647-UCGAAGUACUCAGCGUAAGdTdT-3' (SEQ ID NO: 4), manufactured by GeneDesign, solid lipid nanoparticles were prepared.

### [Example 2-8] Preparation of solid lipid nanoparticles using DSPE-DBCO-AZ-pGlu(DET-C2-Car)30

By a method similar to that in Example 2-3 except that siRNA to be used was changed to siPLK1 (sense: 5'-AGAuCACCCuCCUuAAAuAUU-3' (SEQ ID NO: 5), antisense: 5'-UAUUUAAgGAGGGUGAuCUUU-3' (SEQ ID NO: 6), manufactured by GeneDesign), solid lipid nanoparticles were prepared.

In the above-mentioned siPLK1 sequence, those written in lowercase letters indicate that the base portion is the same, but the sugar portion has been modified by 2'-O-methylation.

### [Comparative Example 2-1] Preparation of solid lipid nanoparticles using DSPE-PEG5k (SUNBRIGHT DSPE-050CN, manufactured by NOF CORPORATION)

By a method similar to that in Example 2-1 except that 2.0 mM DSPE-PEG5k aqueous solution (20 µL) was used instead of the polymer aqueous solution (20 µL) of Example 1-3, solid lipid nanoparticles were prepared.

### [Comparative Example 2-2] Preparation of solid lipid nanoparticles using DSPE-PEG5k

By a method similar to that in Example 2-1 except that 3.0 mM DSPE-PEG5k aqueous solution (20 µL) was used instead of the polymer aqueous solution (20 µL) of Example 1-3, solid lipid nanoparticles were prepared.

### [Comparative Example 2-3] Preparation of solid lipid nanoparticles using DSPE-PEG5k

By a method similar to that in Example 2-1 except that 4.0 mM DSPE-PEG5k aqueous solution (20 µL) was used instead of the polymer aqueous solution (20 µL) of Example 1-3, solid lipid nanoparticles were prepared.

### [Comparative Example 2-4] Preparation of solid lipid nanoparticles using DSPE-PEG5k

By a method similar to that in Example 2-1 except that 1.0 mM DSPE-PEG5k aqueous solution (20 µL) was used instead of the polymer aqueous solution (20 µL) of Example 1-3, and siRNA to be used was changed to Alexa647-labeled siRNA (Alexa647-siGL3, sense: 5'-CUUACGCUGAGUACUUCGAdTdT-3' (SEQ ID NO: 3), antisense: 5'-Alexa647-UCGAAGUACUCAGCGUAAGdTdT-3' (SEQ ID NO: 4), manufactured by GeneDesign), solid lipid nanoparticles were prepared.

### [Comparative Example 2-5] Preparation of solid lipid nanoparticles using DSPE-PEG5k

By a method similar to that in Example 2-1 except that 1.0 mM DSPE-PEG5k aqueous solution (20 µL) was used instead of the polymer aqueous solution (20 µL) of Examples 1 to 3, and siRNA to be used was changed to siPLK1 (sense: 5'-AGAuCACCCuCCUuAAAuAUU-3'(SEQ ID NO: 5), antisense: 5'-UAUUUAAgGAGGGUGAuCUUU-3'(SEQ ID NO: 6), manufactured by GeneDesign), solid lipid nanoparticles were prepared.

### [Experimental Example 1] pH-responsiveness test in the presence of heparin

Using the polymer of Production Example 4-7 and the polymers of Comparative Example 1-1, 1-2, fluorescence correlation spectroscopy (FCS) was measured in the presence of heparin.

Various polymers were labeled with Cy3 using click chemistry, and aqueous solutions with a final concentration of 200 nM were prepared at pH 6-8 (10 mM HEPES or MES, 150 mM NaCl). Heparin was added thereto at a concentration of 200 µg/mL, and the influence of the presence or absence of heparin on the polymer size was confirmed.

The results are shown in Fig. 1. The horizontal axis in Fig. 1 represents pH, and the vertical axis in Fig. 1 represents relative diffusion coefficient. The relative diffusion coefficient is a value calculated from (diffusion coefficient of the polymer in the presence of heparin)/(diffusion coefficient of the polymer in the absence of heparin).

That is, when the relative diffusion coefficient is less than 1, it indicates that anionic heparin and the polymer are interacting, suggesting that the polymer is cationic. For convenience, a relative diffusion coefficient of less than 0.95 is defined here as cationic.

All polymers exhibited cationic property with a relative diffusion coefficient of less than 0.95 at pH 6.0 to pH 6.5. Furthermore, it was confirmed that the polymers of Production Examples 4 to 7 exhibited a relative diffusion coefficient of not less than 0.95 at pH 7.4, and their cationic properties almost disappeared. This result shows that the polymer responds to minute pH changes.

On the other hand, the polymers of Comparative Examples 1-1, 1-2 had a relative diffusion coefficient of less than 0.95 even at pH 7.4, clarifying that they maintain cationic properties.

From the above results, it has become clear that each structure of DET-C2-Car, DET-C3-Car, DET-C2-Sul, and DET-C3-Sul, which are polymer side chain structures of Production Examples 4, 5, 6, and 7 of the present invention, exhibits pH responsiveness in a minute pH range.

### [Reference Experimental Example 1] Measurement of particle size

Respective particles prepared in Examples 2-1 to 2-7 and Comparative Examples 2-1 to 2-4 were analyzed by Zetasizer Nano ZS (manufactured by Malvern Instruments). The measurement results are shown in Table 1.

**[Table 1]**

| | particle size (nm) | | particle size distribution |
|---|---|---|---|
| Example 2-1 | 171 | | 0.24 |
| Example 2-2 | 165 | | 0.19 |
| Example 2-3 | 138 | | 0.19 |
| Example 2-4 | 108 | | 0.23 |
| Example 2-5 | 84 | | 0.24 |
| Example 2-6 | 89 | | 0.25 |
| Example 2-7 | 130 | | 0.27 |
| Comparative Example 2-1 | 81 | | 0.23 |
| Comparative Example 2-2 | 63 | | 0.23 |
| Comparative Example 2-3 | 79 | | 0.15 |
| Comparative Example 2-4 | 130 | | 0.19 |

### [Experimental Example 2] Measurement of zeta potential of particles

The results of zeta potential measurement of the particles obtained in Examples 2-2, 2-7 and Comparative Examples 2-2, 2-4 with 10 mM HEPES (pH 7.4) or 10 mM MES (pH 6.5) are shown in Table 2.

**[Table 2]**

| | pH7.4 | pH6.5 |
|---|---|---|
| Example 2-2 | 9.0 mV | 14.8 mV |
| Example 2-7 | 4.9 mV | 19.2 mV |
| Comparative Example 2-2 | 0.2 mV | -2.9 mV |
| Comparative Example 2-4 | 4.2 mV | 2.3 mV |

The particles of Examples 2-2 and 2-7 had 9.0 mV and 4.9 mV, respectively, at pH 7.4, but 14.8 mV and 19.2 mV, respectively, at pH 6.5. This result revealed that particles obtained from the pH-responsive lipid derivative of the present invention have cationic property in response to minute pH changes.

On the other hand, the particles of Comparative Examples 2-2 and 2-4 had 0.2 mV and 4.2 mV, respectively, at pH 7.4, but -2.9 mV and 2.3 mV at pH 6.5. From this result, it was found that particles obtained from conventional PEG lipids that do not include the pH-responsive lipid derivative of the present invention maintain neutrality regardless of pH.

### [Reference Experimental Example 2] Encapsulation efficiency of siRNA

The encapsulation efficiency of siRNA into the particles of Examples 2-2, 2-7 and Comparative Examples 2-2 and 2-4 was evaluated by fluorometry using RiboGreen reagent.

The amount of encapsulated siRNA was calculated by comparing the fluorescence intensity derived from the RiboGreen reagent in the particle solutions prepared in Examples 2-2, 2-7 and Comparative Examples 2-2 and 2-4, and the fluorescence intensity derived from the RiboGreen reagent in the system in which each particle was disintegrated by Triton X treatment.

The siRNA encapsulation efficiency is shown in Table 3.

**[Table 3]**

| | siRNA encapsulation efficiency |
|---|---|
| Example 2-2 | 93.0% |
| Example 2-7 | 95.0% |
| Comparative Example 2-2 | 98.4% |
| Comparative Example 2-4 | 90.0% |

The siRNA encapsulation efficiency was 93.0% in Example 2-2, 95.0% in Example 2-7, 98.4% in Comparative Example 2-2, and 90.0% in Comparative Example 2-4. From this result, it was found that both the particles obtained from the pH-responsive lipid derivative of the present invention and the particles obtained from the conventional PEG lipid had almost the same siRNA encapsulation efficiency.

### [Experimental Example 3] siRNA cell uptake test

The efficiency of siRNA uptake into cells was evaluated using the particles of Example 2-2 and Comparative Example 2-2. Each particle was separately prepared using Cy5-labeled siRNA according to the method described in each Example.

A549 cells were seeded at 50,000 cells/well (24-well plate) and cultured overnight. Each particle encapsulating Cy5-labeled siRNA was applied thereto (final siRNA concentration: 100 nM), incubated for 4 hr in a serum medium at pH 7.4 or pH 6.5, and the amount of siRNA uptake was evaluated using a flow cytometer. The results are shown in Fig. 2.

In the cells to which the particles of Example 2-2 were applied, a higher amount of Cy5-derived fluorescence was observed at pH 6.5 than at pH 7.4. Furthermore, compared to the particles of Comparative Example 2-2, the particles of Example 2-2 had the same amount of siRNA uptake at pH 7.4, but the amount of siRNA uptake at pH 6.5 increased by about 40%.

From the above results, the particles containing the pH-responsive lipid derivative of the present invention become cationic in response to minute pH changes, compared to particles using conventional PEG lipids, and as a result, the amount of uptake into the cells was improved.

### [Experimental Example 4] siRNA pharmacokinetics test

The tumor accumulation property of siRNA using particles of Example 2-7 and Comparative Example 2-4 were evaluated. A tumor-bearing model mouse was created by subcutaneously transplanting mouse colorectal cancer cell line CT26 into BALB/c mice (5×10⁵ cells/mouse). After the tumor size increased to about 200 mm³, each particle was injected into the tail vein (dose: 0.5 mg/kg, converted to siRNA).

Tumors were removed 1, 6, and 24 hr after administration. Tumors were homogenized using Lysis buffer and processed using a centrifuge. Next, the supernatant liquid was taken out and adjusted to have a concentration of 3% sodium lauryl sulfate and 60% t-BuOH. The concentration of siRNA in the obtained solution was determined using a microplate reader (SPARK TKS01, manufactured by TECAN) (excitation wavelength: 630 nm, fluorescence wavelength: 690 nm). The results are shown in Fig. 3. Statistically significant differences were confirmed using 2-way ANOVA with Tukey's multiple comparisons method, and *P<0.05, **P<0.01, ***P<0.001 were determined to be significant.

In the particles of Example 2-7, siRNA was detected at all times 1, 6, and 24 hr after administration, whereas in the particles of Comparative Example 2-4, siRNA was not detected after 6 hr from the administration.

From the above results, the particles containing the pH-responsive lipid derivative of the present invention become cationic in response to minute pH changes when they reach tumor tissues, compared to particles using conventional PEG lipids, as a result of which they remain around the tumor tissues, that is, they have accumulation property in the tumor tissues. Furthermore, siRNA was detected in the tumor tissue even after 6 and 24 hr, indicating that they have blood retention property.

### [Experimental Example 5] Antitumor effect using siPLK1

The antitumor effect of siPLK1 was evaluated using the particles of Example 2-8 and Comparative Example 2-5. A tumor-bearing model mouse was created by subcutaneously transplanting human ovarian cancer cell line SKOV3-luc cells into Balb/c nude mice (5×10⁶ cells/mouse). When the size of the tumor increased to about 25 mm³, the animals were randomized and divided into three groups of Example 2-8 particle administration group, Comparative Example 2-5 particle administration group, and the control group, and tail vein injection was performed. One dose was 2.5 mg/kg in terms of siPLK1, and administration was performed once every two days (9 times in total). The tumor volume was confirmed on days 0, 2, 6, 12, 14, and 19, including immediately after administration.

The results are shown in Fig. 4. Statistically significant differences were confirmed using 2-way ANOVA with Tukey's multiple comparisons method, and *P<0.05, **P<0.01, ***P<0.001 were determined to be significant.

It was found that the size of the tumor increased over time in the control group and the group administered with the particles of Comparative Example 2-5. On the other hand, in the group to which the particles of Example 2-8 were administered, the growth of tumor cells was suppressed and the increase in tumor size became gradual.

From the above results, it was confirmed that particles containing the pH-responsive lipid derivative of the present invention exhibit a significant antitumor effect compared to particles using conventional PEG lipids.

### [Industrial Applicability]

As described above, according to the present invention, a pH-responsive transport carrier which is effective as a transport carrier capable of achieving an introduction system that can safely and efficiently perform delivery of low-molecular-weight drugs, nucleic acid pharmaceutical products, and the like to around tumor tissues and transfection thereof to tumor cells, and further, a pH-responsive lipid derivative useful for forming such transport carriers can be provided.

The pH-responsive transport carrier of the present invention has the property of being electrically neutral under neutral environment (pH 7.4) and changing to cationic under weakly acidic conditions (pH 6.5) around tumor tissues. Therefore, it exhibits stealth property in blood components and normal tissues which are under neutral environment in vivo, and improves the accumulation efficiency and uptake efficiency into tumor tissues in response to minute pH changes around tumor tissues. As a result, the low-molecular-weight drugs or nucleic acid pharmaceutical products encapsulated in the transport carrier can be released around the tumor tissues or introduced efficiently into tumor tissues cells.

Furthermore, the pH-responsive lipid derivative of the present invention is composed of a polymer of amino acids contained in living organisms. Therefore, after delivery of a low-molecular-weight or nucleic acid pharmaceutical product, it is decomposed within the body and is not accumulated within the body.

This application is based on a patent application No. 2021-113189 filed in Japan (filing date: July 7, 2021), the contents of which are incorporated in full herein.

## Claims

1. A pH-responsive lipid derivative represented by a structure represented by the following formula (i): 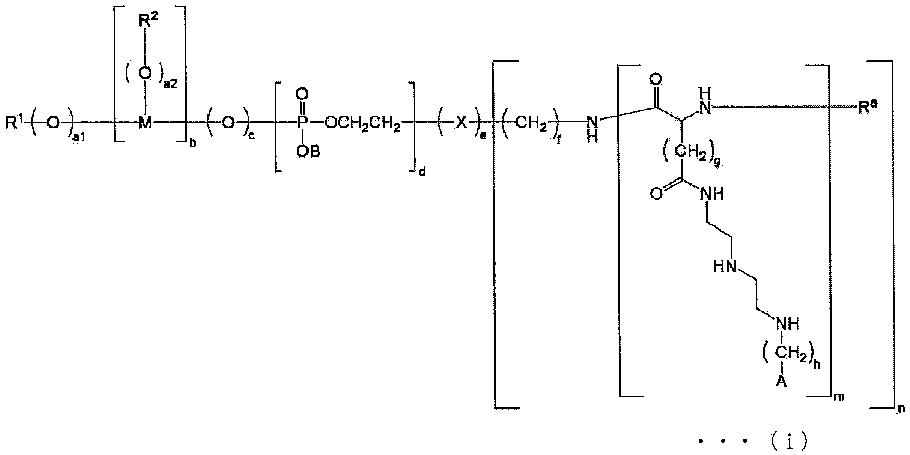 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{a} is H, -COCH₃, or -CO-(CH₂)_{b1}-COOH (wherein b1 is 2 or 3) .

2. The pH-responsive lipid derivative according to claim 1, which is represented by a structure represented by the following formula (1): 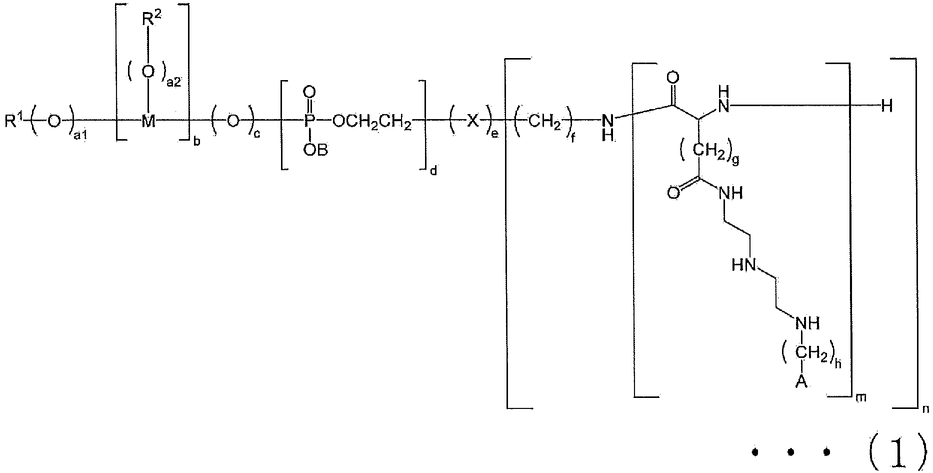 wherein
R¹ and R² are each independently an alkyl group having 8 to 24 carbon atoms, or an acyl group having 8 to 24 carbon atoms, or
a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction,
a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2, and
m is 5 to 300.

3. The pH-responsive lipid derivative according to claim 1, which is represented by a structure represented by the following formula (1-1): 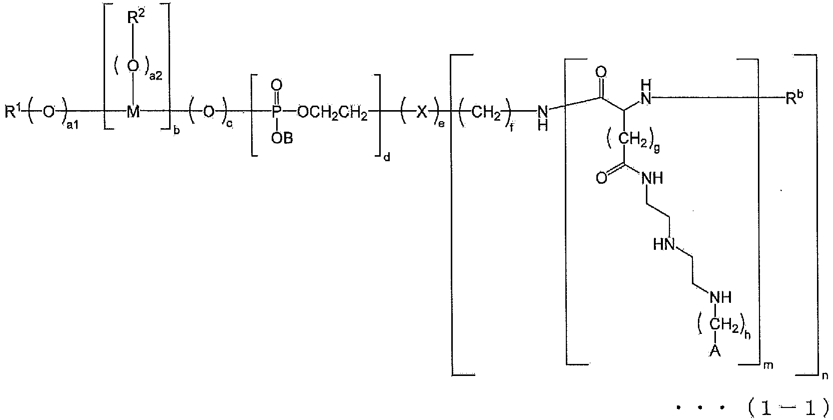 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{b} is -COCH₃ or -CO- (CH₂)_{b1}-COOH (wherein b1 is 2 or 3) .

4. The pH-responsive lipid derivative according to claim 1, wherein, in the aforementioned formula (i),
X is a linking group represented by the following formula (2): 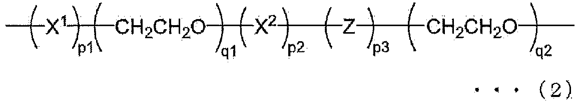 (in the formula (2), X¹ and X² are each independently a group having 1 to 5 carbon atoms and containing a bonding group resulting from a nucleophilic substitution reaction, Z is a group formed by a Michael addition reaction, a thiol-ene reaction, or a cyclization addition reaction, p1, p2, p3 are each independently 0 or 1, and q1 and q2 are each independently 0 to 50).

5. The pH-responsive lipid derivative according to claim 1 or 4, wherein, in the aforementioned formula (i), m is 5 to 150.

6. The pH-responsive lipid derivative according to claim 1 or 4, wherein, in the aforementioned formula (i), g and h are each 2, and A is a carboxy group.

7. The pH-responsive lipid derivative according to claim 1 or 4, wherein, in the aforementioned formula (i),
R¹ and R² are each independently an alkyl group having 8 to 24 carbon atoms,
b and n are each 1, and
d is 0.

8. The pH-responsive lipid derivative according to claim 1, wherein, in the aforementioned formula (i),
R¹ and R² are each independently an alkyl group having 8 to 24 carbon atoms,
M is an optionally branched hydrocarbon trivalent group having 3 to 7 carbon atoms,
A is a carboxy group,
a1, a2, b, and n are each 1,
c, d, e, and f are each 0, and
g and h are each 2.

9. The pH-responsive lipid derivative according to claim 1 or 4, wherein, in the aforementioned formula (i),
R¹ is a sterol residue, and a1, b, c, and d are each 0.

10. The pH-responsive.lipid derivative according to claim 1 or 4, wherein, in the aforementioned formula (i),
R¹ and R² are each independently an acyl group having 8 to 24 carbon atoms,
M is an optionally branched hydrocarbon trivalent group having 3 to 7 carbon atoms,
B is a hydrogen atom, an alkali metal, or an ammonium group, a1, a2, b, c, d, e, and n are each 1,
f is an integer of 2 to 5, and
for X in the aforementioned formula (2),
X¹ and X² are each a group having 1 to 5 carbon atoms and containing a bonding group resulting from a nucleophilic substitution reaction, and p1, p2, and p3 are each 1.

11. The pH-responsive lipid derivative according to claim 1 or 4, wherein, in the aforementioned formula (i),
R¹ and R² are each independently an acyl group having 8 to 24 carbon atoms,
M is an optionally branched hydrocarbon trivalent group having 3 to 7 carbon atoms,
B is a hydrogen atom, an alkali metal, or an ammonium group, a1, a2, b, c, d, e, and n are each 1,
f is an integer of 2 to 5, and
for X in the aforementioned formula (2),
X¹ and X² are each a group having 1 to 5 carbon atoms and containing a bonding group resulting from a nucleophilic substitution reaction,
Z is a group formed by a cyclization addition reaction,
p1, p2, and p3 are each 1, and
q2 is 0 to 5.

12. The pH-responsive lipid derivative according to claim 1 or 4, wherein, in the aforementioned formula (i),
R¹ and R² are each independently an acyl group having 8 to 24 carbon atoms,
M is an optionally branched hydrocarbon trivalent group having 3 to 7 carbon atoms,
B is a hydrogen atom, an alkali metal, or an ammonium group, a1, a2, b, c, d, e, and n are each 1,
f is an integer of 2 to 5, and
for X in the aforementioned formula (2),
X¹ and X² are each a group having 1 to 5 carbon atoms and containing a bonding group resulting from a nucleophilic substitution reaction,
Z is a group formed by a cyclization addition reaction, p1, p2, and p3 are each 1, and
q1 is 0 to 5.

13. The pH-responsive lipid derivative according to claim 1 or 4, wherein, in the aforementioned formula (i),
R¹ and R² are each independently an acyl group having 8 to 24 carbon atoms,
M is an optionally branched hydrocarbon trivalent group having 3 to 7 carbon atoms,
B is a hydrogen atom, an alkali metal, or an ammonium group, A is a carboxy group,
a1, a2, b, c, d, e, and n are each 1,
f is an integer of 2 to 5,
g and h are each 2, and
for X in the aforementioned formula (2),
X¹ and X² are each a group having 1 to 5 carbon atoms and containing a bonding group resulting from a nucleophilic substitution reaction,
Z is a group formed by a cyclization addition reaction, p1, p2, and p3 are each 1, and
q1 and q2 are each independently 0 to 5.

14. The pH-responsive lipid derivative according to claim 1 or 4, wherein, in the aforementioned formula (i),
R¹ and R² are each independently an alkyl group having 8 to 24 carbon atoms or an acyl group having 8 to 24 carbon atoms, b, e, and n are each 1,
d is 0,
f is an integer of 2 to 5, and
for X in the aforementioned formula (2),
X¹ and X² are each a group having 1 to 5 carbon atoms and containing a bonding group resulting from a nucleophilic substitution reaction, and
p1, p2, and p3 are each 1.

15. The pH-responsive lipid derivative according to claim 1 or 4, wherein, in the aforementioned formula (i),
R¹ and R² are each independently an alkyl group having 8 to 24 carbon atoms or an acyl group having 8 to 24 carbon atoms,
A is a carboxy group,
b, e, and n are each 1,
d is 0,
f is an integer of 2 to 5,
g and h are each 2, and
for X in the aforementioned formula (2),
X¹ and X² are each a group having 1 to 5 carbon atoms and containing a bonding group resulting from a nucleophilic substitution reaction,
Z is a group formed by a cyclization addition reaction, p1, p2, and p3 are each 1, and
q1 and q2 are each independently 0 to 5.

16. A method for producing a pH-responsive lipid derivative represented by a structure represented by the following formula (47-i): 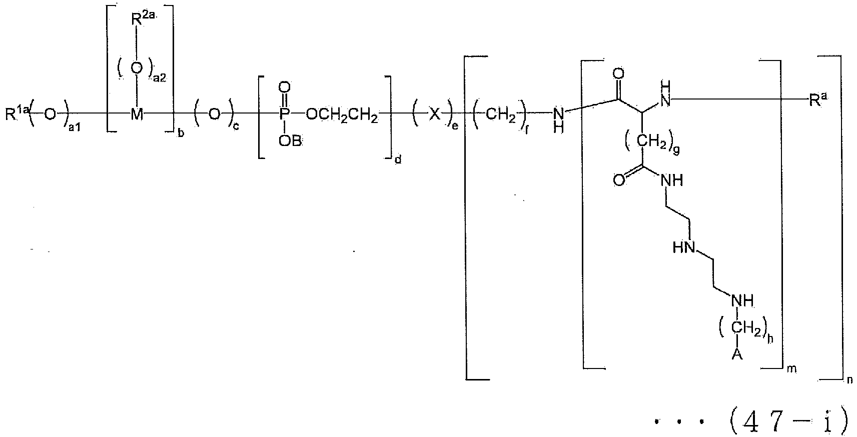 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
X is a linking group containing a bonding group formed by a nucleophilic substitution reaction, a Michael addition reaction, a thiol-ene reaction, or a cyclization addition reaction,
A is a carboxy group or a sulfo group,
a1, a2, b, c, d, and e are each independently 0 or 1,
f is an integer of 0 to 5,
h is an integer of 1 to 3,
g and n are each independently 1 or 2,
m is 5 to 300, and
R^{a} is H, -COCH₃, or -CO-(CH₂)_{b1}-COOH (wherein b1 is 2 or 3), the method comprising the following step (A), step (B), and step (C) performed in this order when R^{a} in the formula (47-i) is H, and the following step (A1), step (B), and step (C) performed in this order when R^{a} in the formula (47-i) is -COCH₃ or -CO-(CH₂)_{b1}-COOH (wherein b1 is 2 or 3) :
[step (A)]
a step of obtaining a polymer represented by the following formula (5) by a ring opening polymerization using a polymerization initiator represented by the following formula (3) and an α-amino acid-N-carboxylic acid anhydride represented by the following formula (4) 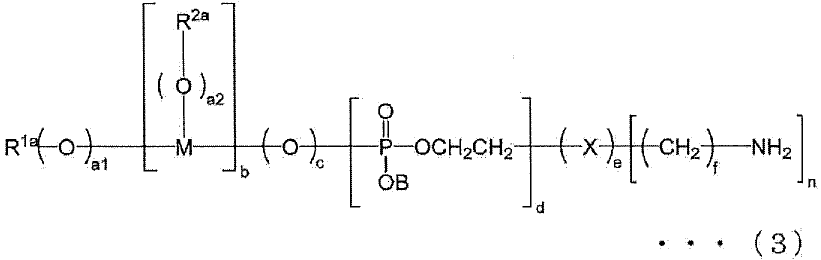 (in the formula (3), each symbol is as defined above) 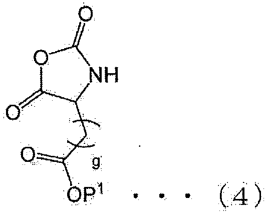 (in the formula (4), P¹ is a carboxy-protecting group, and g is 1 or 2) 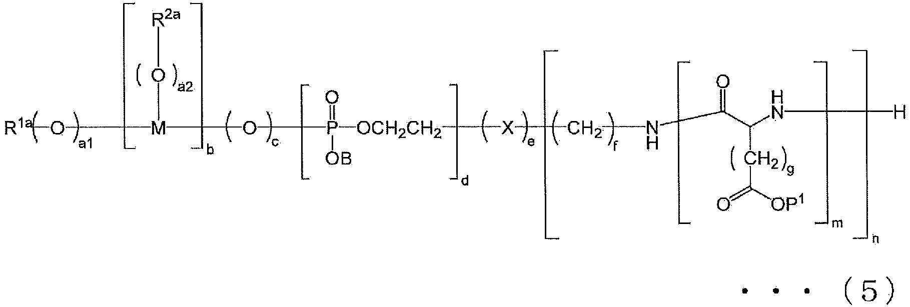 (in the formula (5), each symbol is as defined above)
[step (A1)]
a step of obtaining a polymer represented by the following formula (5) by a ring opening polymerization using a polymerization initiator represented by the following formula (3) and an α-amino acid-N-carboxylic acid anhydride represented by the following formula (4), and further introducing -COCH₃ or -CO-(CH₂)_{b1}-COOH (wherein b1 is 2 or 3) into a terminal amino group of the obtained polymer to obtain a polymer represented by the following formula (5-1) 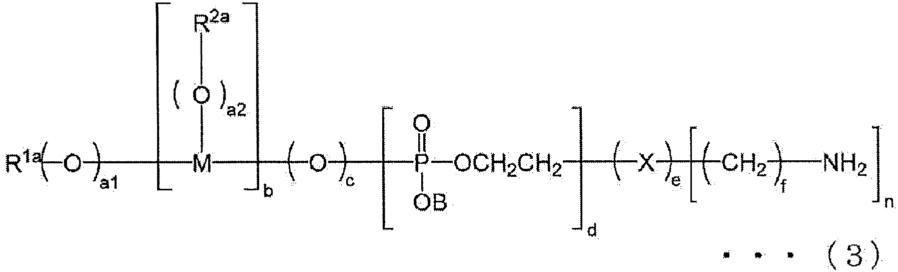 (in the formula (3), each symbol is as defined above) 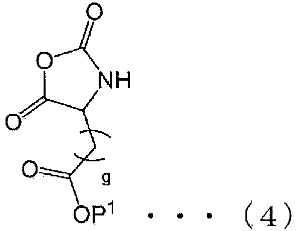 (in the formula (4), P¹ is a carboxy-protecting group, and g is 1 or 2) 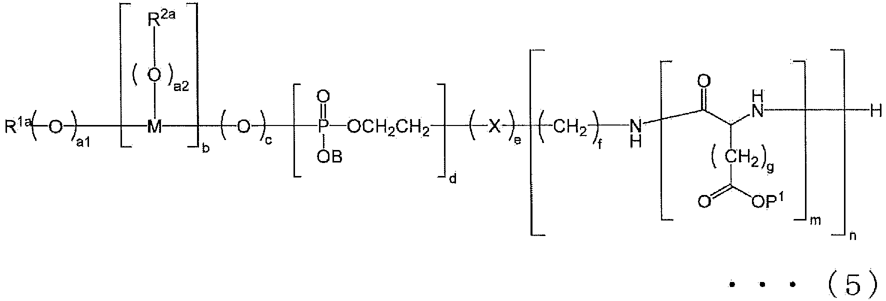 (in the formula (5), each symbol is as defined above) 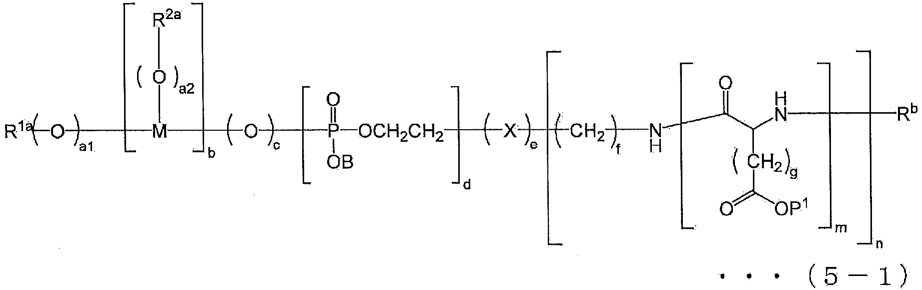 (in the formula (5-1), R^{b} is -COCH₃, or -CO- (CH₂)_{b1}-COOH (wherein b1 is 2 or 3), and other symbols are as defined above)
[step (B)]
a step of obtaining a polymer represented by the following formula (7-i) by reacting a polymer represented by the following formula (5-i) obtained in the aforementioned step (A) or the aforementioned step (A1) (the polymer represented by the aforementioned formula (5) and the polymer represented by the aforementioned formula (5-1) are comprehensively denoted) with a diethylenetriamine derivative represented by the following formula (6) 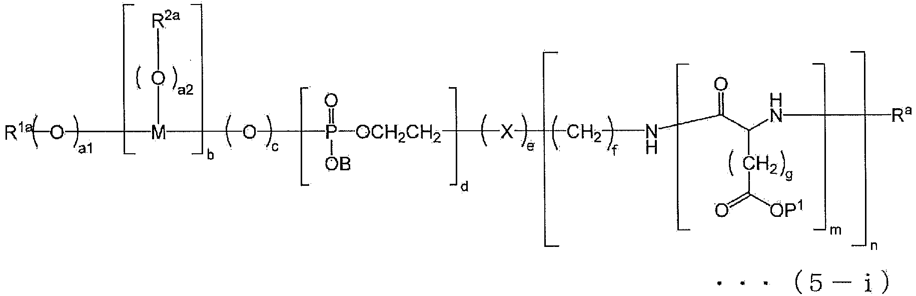 (in the formula (5-i), each symbol is as defined above) 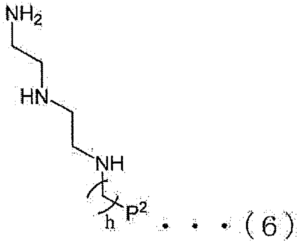 (in the formula (6), P² is a protected carboxy group or a protected sulfo group, and h is as defined above) 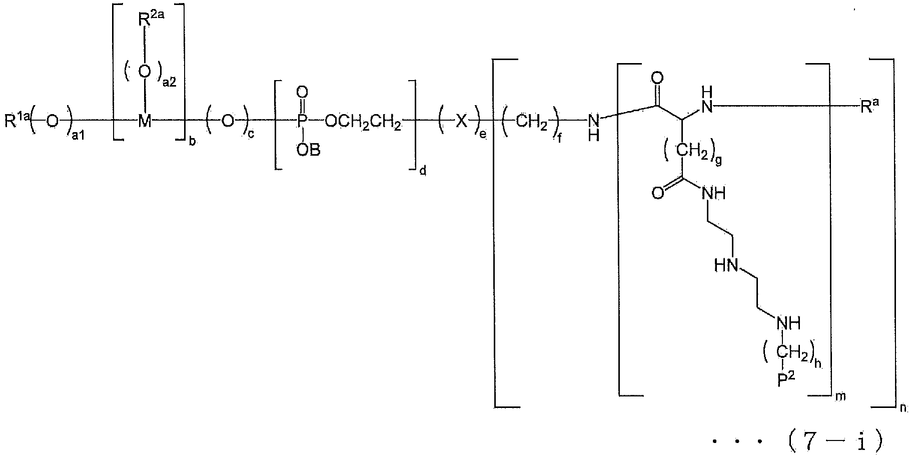 (in the formula (7-i), each symbol is as defined above)
[step (C)]
a step of obtaining a pH-responsive lipid derivative represented by the aforementioned formula (47-i) by deprotecting a polymer represented by the aforementioned formula (7-i) obtained in the aforementioned step (B).

17. A method for producing a pH-responsive lipid derivative represented by a structure represented by the following formula (36-i), comprising performing the following step (D): 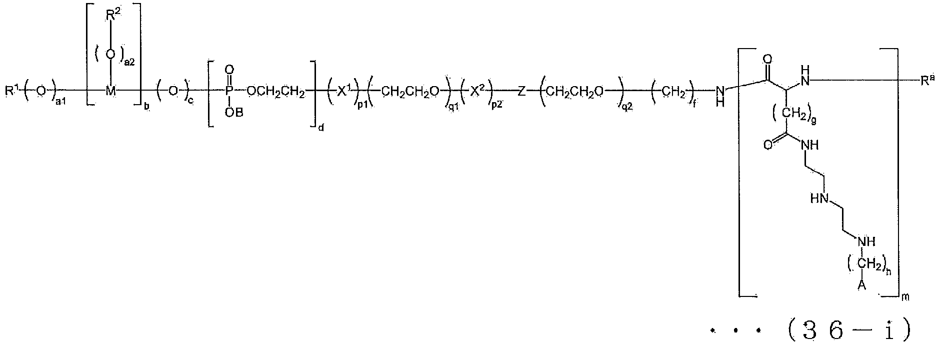 wherein
R¹ and R² are each independently an alkyl group having 8 to 24 carbon atoms, or an acyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1,
q1 and q2 are each independently 0 to 50, and
R^{a} is H, -COCH₃, or -CO-(CH₂)_{b1}-COOH (wherein b1 is 2 or 3)
[step (D)]
a step of obtaining a pH-responsive lipid derivative represented by the aforementioned formula (36-i) by reacting a functional group-containing lipid represented by the following formula (8) with a functional group-containing pH-responsive polymer represented by the following formula (9-i) 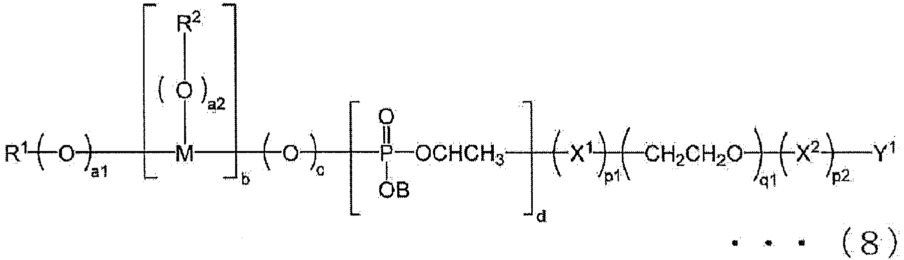 (in the formula (8), Y¹ is an atomic group containing a functional group capable of forming a covalent bond with a functional group contained in the following Y², and other symbols are as defined above) 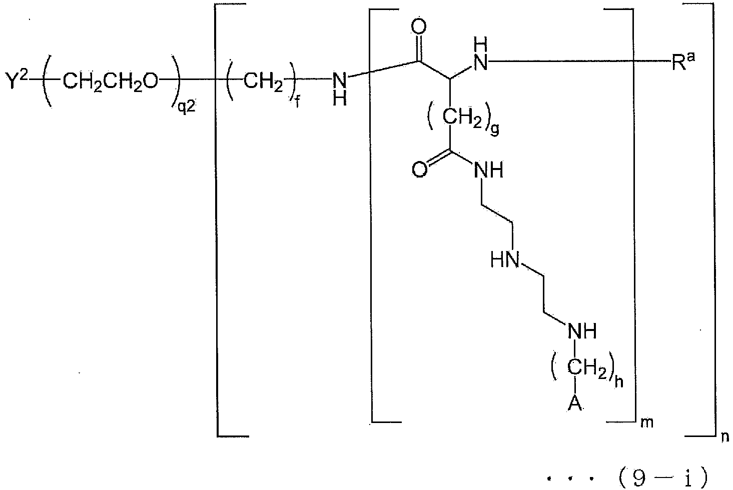 (in the formula (9-i), Y² is an atomic group containing a functional group capable of forming a covalent bond with a functional group contained in the aforementioned Y¹, and other symbols are as defined above).

18. A method for producing a pH-responsive lipid derivative represented by a structure represented by the following formula (46-i), comprising performing the following step (E), step (F), and step (G) in this order: 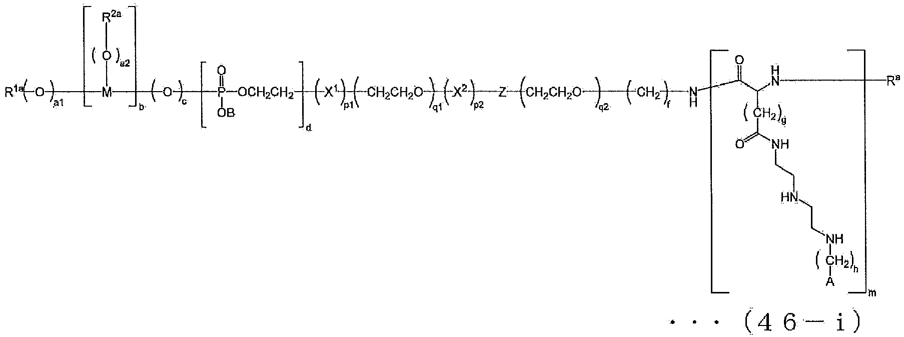 wherein
R^{1a} and R^{2a} are each independently an alkyl group having 8 to 24 carbon atoms, or a sterol residue,
M is a trivalent group represented by>N-CH₂-CH₂- or an optionally branched hydrocarbon trivalent group having 3 to 7 carbon atoms,
B is a hydrogen atom, an alkali metal, or an ammonium group,
A is a carboxy group or a sulfo group,
a1, a2, b, c, and d are each independently 0 or 1,
f is an integer of 0 to 5,
h is an integer of 1 to 3,
g is 1 or 2,
m is 5 to 300,
X¹ and X² are each independently a group having 1 to 5 carbon atoms and containing a bonding group resulting from a nucleophilic substitution reaction,
Z is a group formed by a Michael addition reaction, a thiol-ene reaction, or a cyclization addition reaction,
p1 and p2 are each independently 0 or 1,
q1 and q2 are each independently 0 to 50, and
R^{a} is H, -COCH₃, or -CO-(CH₂)_{b1}-COOH (wherein b1 is 2 or 3)
[step (E)]
a step of obtaining a polymer represented by the following formula (12-i) by reacting a functional group-containing lipid represented by the following formula (10) with a polymer represented by the following formula (11-i) 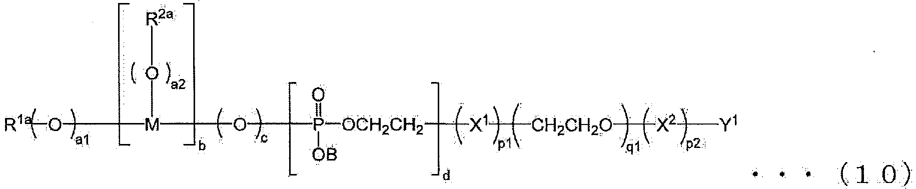 (in the formula (10), Y¹ is an atomic group containing a functional group capable of forming a covalent bond with a functional group contained in the following Y², and other symbols are as defined above) 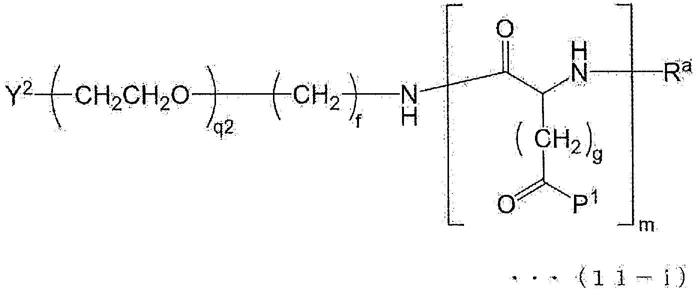 (in the formula (11-i), P¹ is a carboxy-protecting group, Y² is an atomic group containing a functional group capable of forming a covalent bond with a functional group contained in the aforementioned Y¹, and other symbols are as defined above) 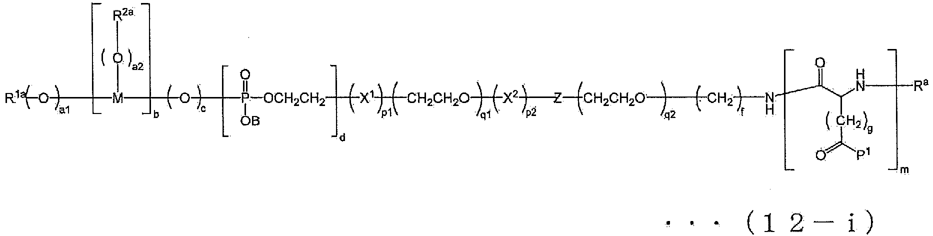 (in the formula (12-i), each symbol is as defined above)
[step (F)]
a step of obtaining a polymer represented by the following formula (13-i) by reacting a polymer represented by the aforementioned formula (12-i) obtained in the aforementioned step (E) with a diethylenetriamine derivative represented by the aforementioned formula (6) 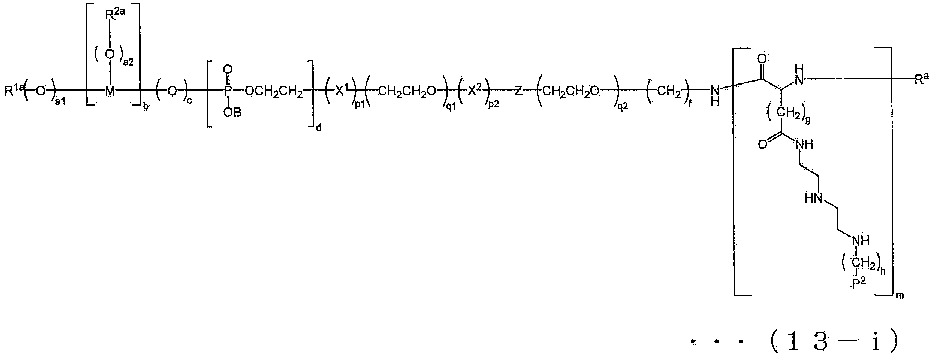 (in the formula (13-i), P² is a protected carboxy group or a protected sulfo group, and other symbols are as defined above)
[step (G)]
a step of obtaining a pH-responsive lipid derivative represented by the aforementioned formula (46-i) by deprotecting a polymer represented by the aforementioned formula (13-i) obtained in the aforementioned step (F).

19. A transport carrier for drug delivery comprising 0.1 to 30 mol% of a pH-responsive lipid derivative according to claim 1.

20. The transport carrier for drug delivery according to claim 19, wherein the carrier is a solid lipid nanoparticle.

21. The transport carrier for drug delivery according to claim 19, wherein a difference (Q) in the zeta potential represented by the following formula (F1) is not less than 2.5 mV:
formula (F1): Q=(zeta potential at pH 6.5)-(zeta potential at pH 7.4)

22. The transport carrier for drug delivery according to claim 19, wherein the difference (Q) in the zeta potential represented by the aforementioned formula (F1) is not less than 2.5 mV and the zeta potential at pH 6.5 is higher than 0.

23. The transport carrier for drug delivery according to claim 19, wherein the particle size is 10 to 200 nm.

24. The transport carrier for drug delivery according to claim 19, further comprising a phospholipid.

25. The transport carrier for drug delivery according to claim 19, further comprising a cationic lipid.

26. The transport carrier for drug delivery according to claim 19, wherein the aforementioned cationic lipid is DOTAP (1,2-dioleoyloxy-3-trimethylammonium propane) or DODAP (dioleoyl-3-dimethylammonium propane).

27. The transport carrier for drug delivery according to claim 19, further comprising sterol.

28. The transport carrier for drug delivery according to claim 19, comprising 0.1 to 30 mol% of the pH-responsive lipid derivative according to any one of claims 1 to 15, 0 to 60 mol% of the phospholipid, 1 to 60 mol% of the cationic lipid, and 0 to 40 mol% of sterol.

29. The transport carrier for drug delivery according to claim 19, wherein the transport drug is a nucleic acid derivative.

30. The transport carrier for drug delivery according to claim 19, wherein the transport drug is peptide.

31. The transport carrier for drug delivery according to claim 19, wherein the transport drug is a low-molecular-weight drug or an anticancer agent.

32. The transport carrier for drug delivery according to claim 19, wherein the pH-responsive lipid derivative is the pH-responsive lipid derivative according to claim 2.

33. The transport carrier for drug delivery according to claim 19, wherein the pH-responsive lipid derivative is the pH-responsive lipid derivative according to claim 3.
